# EUROPEAN PATENT SPECIFICATION

(11) **EP 2 873 739 B1**
(45) Date of publication and mention of the grant of the patent: **12.04.2017**
(21) Application number: 13306548.2
(22) Date of filing: 13.11.2013
(51) Int. Cl.: C12Q 1/68, G01N 33/53

(54) **Diagnosis of syndromic obesity**
Diagnose von syndromatischer Adipositas
Diagnostic de l'obésité syndromique

(43) Date of publication of application: 20.05.2015
(73) Proprietor: Centre National de la Recherche Scientifique (C.N.R.S.), 75016 Paris (FR); UNIVERSITE PARIS DESCARTES, 75006 Paris (FR); ASSISTANCE PUBLIQUE, HOPITAUX DE PARIS, 75004 Paris (FR); Godtech, 75011 Paris (FR)
(72) Inventor: Abitbol, Marc, 75016 Paris (FR); Burgain-Chain, Aurore, 45300 Thignonville (FR); Rametti-Lacroux, Armelle, 78230 Le Pecq (FR); Vieira, Véronique, 02540 La Celle Sous Montmir (FR)
(74) Representative: Regimbeau

(56) References cited:
- WO-A2-2011/138671
- OHLEMILLER K K ET AL: "Progression of cochlear and retinal degeneration in the tubby (rd5) mouse", AUDIOLOGY AND NEUROTOLOGY, KARGER, BASEL, CH, vol. 2, no. 4, 1 July 1997 (1997-07-01), pages 175-185, XP009177593, ISSN: 1420-3030
- SNIEDER H ET AL: "TUB is a candidate gene for late-onset obesity in women", DIABETOLOGIA ; CLINICAL AND EXPERIMENTAL DIABETES AND METABOLISM, SPRINGER, BERLIN, DE, vol. 51, no. 1, 23 October 2007 (2007-10-23), pages 54-61, XP019558691, ISSN: 1432-0428, DOI: 10.1007/S00125-007-0851-5
- KONG L ET AL: "Molecular mechanisms underlying cochlear degeneration in the tubby mouse and the therapeutic effect of sulforaphane", NEUROCHEMISTRY INTERNATIONAL, PERGAMON PRESS, OXFORD, GB, vol. 54, no. 3-4, 1 March 2009 (2009-03-01), pages 172-179, XP025999857, ISSN: 0197-0186, DOI: 10.1016/J.NEUINT.2008.08.013 [retrieved on 2008-12-09]
- IKEDA AKIHIRO ET AL: "Genetic modification of retinal degeneration in tubby mice", EXPERIMENTAL EYE RESEARCH, vol. 74, no. 4, April 2002 (2002-04), pages 455-461, XP55114495, ISSN: 0014-4835

### SEQUENCE LISTING

<110> CENTRE NATIONAL DE LA RECHERCHE SCIENTIFIQUE (CNRS) UNIVERSITE PARIS DESCARTES ASSISTANCE PUBLIQUE - HOPITAUX DE PARIS GODTECH
<120> DIAGNOSIS OF SYNDROMIC OBESITY
<130> 70744D32646
<160> 10
<170> PatentIn version 3.5
<210> 1
   <211> 74475
   <212> DNA
   <213> homo sapiens
<220>
   <221> misc_feature
   <223> TUB gene
<400> 1
   cttattctcc tcggggccac ctgccccaac ccagggcctg gcacggcaga gatggcagag      60
   gtgtttggtg gggtgtaatg tgtaaacagc agagtgctgc tgtcgtcatt catcccacca     120
   tagtttgtct ggtgaatgca tttttagtgt caagctgcct gaaggagaag ccagggataa     180
   agacccaagc tcagaatgta tcctggggag agggattggt tcacagagag aagctgtctt     240
   gcagcctttc cccctctggc ctggttctgg ctgttgccag cattctagga gttctctaga     300
   cgggctgaaa cgcaccgcag ggatacaggg agggccggag aataggcgtt tgtttccagg     360
   tagaattttg gggcataccc ggccttgtct gggagcaatc agggaccaga ggcaagggct     420
   gcgatgggct ctggggccta ctgtggcctc atccctctca cctggcccca gctcaggcca     480
   ttccaagagc ctcccagcct aacagcaacg tgtggctatc caagggtccc agacagagga     540
   ttggagggct gcacctgtgt ttaggggaca gccacccctc cccctaagca cctgctctga     600
   cagcatggga tgatgtcaac aagggacttc catgaagccc aagggggaag gacagtggga     660
   gtggggtctg aggtctggac tctgcttgaa gattgacaat gatgggtggg agtccctcac     720
   ccactgtaag ctctaggaag agggtgagca ttcctgttga tactgtggcc cattgtgttg     780
   gcagagtcca ggccagtttg tgctcttggt gtgaccccag gagggagtcc tttgctggat     840
   catctacctc atgggctggt actgacatgc aggtgcgatt tccctgccta aaacaggctc     900
   cagagtaaga ctggcatcgc tcaccagggt aattattggt ttgggttcaa tttccattca     960
   aaacagtaat cccagcctga gctgggtgtc agatctgaag gttgattatt agtaacattt    1020
   atcaacagcc tctctcagct tcaggcaatt acagctcatc tgccattcct gctcccagtc    1080
   atgcaaactt gccagcttct tccctgccca ccccctccat tcccctctcc ccttcttctc    1140
   ccatctcctc cccttagcag acaacctgac ggagggcagg aggtgggtgc caccttatga 1200
   ctcactatca ccctgtatgg agggggtcca tgtgcatgct aggcacctgt gctccccagc 1260
   agcaatattc atgttgcagt cttgtgaaat ctgaatctga ttctatcaga actgaggaga 1320
   atctgtgaag gggacagatg ggaacccatg tctcccggct ccttgtcgca tgatgcgttt 1380
   tcaatggcac tgtgctcctt cctgctcctg actcagtctg tccctcccct ccagggctga 1440
   gactagggtg aggctagaaa gacattgacc tcaagtgcaa cattcaaagg acctcgaaca 1500
   agctcagtaa ttaagataaa tgatatttca atatagtatt tttttaaatc aaaatgaatg 1560
   caaagcacat acaacaaaat caaaccttta ataaagacaa gatacaacac tggatttgcg 1620
   ggccttgtat tggcctcact tgccttaccc taactccagt cttgtttatc atggacagtt 1680
   ttgctttgat ttgctggaag tattaaattt cttggctgct gagttttttg gcaaatcttt 1740
   aaattctgcg cctcaggcga gagctttatt cagcttaccc tggtgctggc cccactgctc 1800
   tcacttcccg ctgggcccta acctcctgct cccttcagct cttactgcct actgcctcag 1860
   gcagggtggc tcagcttctc tcttcgcagg tgcccagggc agcaggggac ccaaagggcc 1920
   cctccatggg ctgtcttcca gggtgcccat ccttcattcc atcccactgg accctgcttc 1980
   agccgtcaga cacctcaggg agggcctgca ggttgccaga gtaactgctg tgataactgg 2040
   aggacagaac atgctggtct tgctctgctc gtagaatcac atccagccag ggctggatga 2100
   gtgcaagcag gcacgcctga cagcgtccct gacacgctga tccaaaacgt cactggacat 2160
   gcatggaggt ggagaacatt ccatgtaccc acattcctct agggggagga cagcatgagg 2220
   ctggaggaaa actgtggtga tctgtttgtg acagggaggt gagacgctga agtagacatg 2280
   gatgcttcct aaccagcctt ccgcagaggg taggtctcat tcgctgaagg gcttctgttc 2340
   tgctgagcag ggtctgtcag taggggggca cacctgtctc cagagaatac cctcctcctg 2400
   tcctcccctg gctgtgctcc actagcctaa aaggtaaaca gacattttag aaagatcagt 2460
   gttgaagggg tacccaagat gccaaattat atctgggact tgagacactg ttatgtcgag 2520
   gtccaggcct aggccagctg gtcacagtgt ccagatgcct gtcacggtgg gaggcctgag 2580
   ggtctcaggg gacatgtatc agaagcacct ctgccctgcc tgttccactc tgtaatctcc 2640
   cttctgagcc cctactgcag cacagagcca gctggtcatc tagcctggca gtagtaagtc 2700
   attctatttt cctgcagata ggaatacatg gttcctgttc ctcctatgca ctctgccact 2760
   taaattcccc tctctgagtc ctaggttccc cttttgtgaa atatcaatga tagcatcctt 2820
   cttagaaggc tgtgctcacc attcagtgca ctaatgcaaa gcactgtgac cgactgaaga 2880
   gtcatgttct gtggggccat ggaggacaga actaggactg aagggaggtg tgtttgagct 2940
   ttaggtgaag cagcaatggc caactacaaa gatggaggga ccgctctggg aagagcaaac 3000
   accctgatgc tcagagtgtg catgaggagg tttcataatc accatccagc agcttagcct 3060
   caaaagggct gcctgcccca gggagctatg acccttgaga gatgcagttt atccagccct 3120
   gaggttctgt ttgaccatct ttccccggtt gtcctccagg gggtcatggc acaagtctca 3180
   gtagcacggg ccccatggtc cagccttaag gtaagaatgg acctccctgg aggaagctgg 3240
   cttcatctac agttgataag ttcacctttt ttcctggtcc actttccttg gtttaaccct 3300
   gtgaccaaac ctgagagctt tggcaggaag gaaaccaggg aggatgttgt gcttgagaaa 3360
   gtgctggcct gagcattggc tttgagatgt ccttttactc tgactggagg gtctcattcc 3420
   acctgtagca agactaaaga cacctgaaag agagtttctg ggagatggag gatgaggtct 3480
   ccagttgcag gtgcatgcac acgtccactt ccccacctgg caggtgccgg catgcaggat 3540
   gtctgtgcgt gtgccccttg cactgactcc cttgaggctg gctgtgcagc tttggggcat 3600
   gtgtccaagc agagagaatg gaagactcca tattgggagc cttggctttg acctttcctt 3660
   tctctgagcc tgattttccc aacagtgtta tgggagggga aggatgagat gcgcttctca 3720
   gctgatgtcc gtgattcttc tgttttctgg aggccatgag tgttaacaga atgtgttcac 3780
   ttttgcaccc ttcttccatg accacttaca gtctgtctgc ttagcagatg aggggtctgg 3840
   gtctccagcg tccatttggg gtggggtcag caatgtccag ctttgcatct gggtatcact 3900
   tttccttctg atacttgaaa ttggattctg aagattccta attattgttc caagttctca 3960
   ttgaaaatct gggtgtaatt tttacaagag catggctgag gatggacatg gaggggaagt 4020
   agtggggctg gagggaggga agggacagac agaaggtgat gttgtcatta ggagttaaag 4080
   ccagggcctg gtagtagata aggctggaca gttggcagga tcatcgggcg gactaaagta 4140
   gcttagattc tgtccagagg aagtgggggt cttctgaagg gtttaagtag gctgggagga 4200
   catgatctta ggaagctcac tctggtgtca gttgcaggat ggatttgaga ggagcaagtt 4260
   aggtgtagat gcccatgatg atgccaagat ctgggcaaca gacaggaagg ccctagctca 4320
   gaagtggctc tagggaaggt gaggtgcata gaattgagag atgctcagta gatggcatga 4380
   gcagtgcttg atgattgtct gggttggtgg aggaaggtgg acagggaaaa gcagaaagct 4440
   acgatggtgc ctcaaggggc tgagtgatgt cactcacaga gacagaatgt atagagtgaa 4500
   tgttcagact cacaggaagt ccaaaactac ataccccaac gtgaggtgct gtgggacatc 4560
   cggggtgcag ggtccagaga gcaggtaggt agagtttaga agagggctgg gtccacaatg 4620
   cagccttgga tgttctcaat gtaagagttg tgggagatga agccttgtga gtggatggga 4680
   acacccaggt gcatttcagg tgaagcaagg ggacaagagg ctgaggacac agacaagcaa 4740
   atcctagatc ttccatcagt ccctagaagg cacgatgtgt gcccctccca gcacacagcc 4800
   tgagccctag cacagagctg gccgcagaga gggcagcagt gaatgtgtcc tcggtggttc 4860
   ctccagatgg ggcctttgtc cgcagtgcac ttgtctctgc ctgggttgct atagtaaccc 4920
   acagatgcag agagacttgg cctccgtgtt gccatggaaa ccagcaattg ggtgtccctg 4980
   tgtggcatgg ccactgagac cttgaggatt cagtctggtc ctgaagggtt tggggggaga 5040
   ctgcgaccag aagatgtttc catgtcctaa ttaatgggtg atggtggttg ttagtctgac 5100
   tgttgccacg gtgatgaagg gagacatcca agtgctggtt tcagtactga ggcgaataca 5160
   gggaatttca acaggctcca ggtcttacta tgcagcctga agtgggacca tcccttaaac 5220
   ccactccatc ctgtggccac gatgggggcc aggacacctt tgccttcttt ctgggtttct 5280
   ttctttgccg agacagggat tttgttccca ggaggcactc cctggcccat gggatctcag 5340
   cattcaaagc agcacaggaa acctgggccc ctgaaacggg gccaccgaag agatcggtaa 5400
   gctttcaaca tcctgccttt agcccatggg cccaaccatt gcgtcagctc cacccaccca 5460
   ccctcactga cctcaaccct ttacagtccc aaggcctccc cttcctgatg gtgctgctcc 5520
   cctatcacca cgtggagccc cacaggtata tccccgcaaa cccctcttgc tccagacagg 5580
   tgatcaggga ggcttcatta ctccaggagc tgcccaggac ctcagggagg tcccccacca 5640
   tgggagtaag ggcctctccc ccagcctcca gggctccggc agctccacac tccggccctc 5700
   tgcctcttct ggagggctcc tccttccagc ttctgcctgt ctccccaccc cccagtgctt 5760
   cattcccatg gctcaggtca tcactgccct catctggact gcctgcctgc ctgcctgcca 5820
   tccggggccc acagagtgag cactctatgg aagtccctct accctgatcc atcacgtaac 5880
   tcacctactg aggcagctga gaccagggca ctgaaggagg ccttgagtga ccaggtgtgg 5940
   ccaggcagca ggcagggatg atgaggtgag gctgcaagga tgtggagagt caccccttct 6000
   tttcctcctc aggagaacaa ccaggaggaa gtactggaag gaaggaaggg agatcgctcg 6060
   gtgagctgga ggggaggagg gcgtgccggc cctgggaaag ggccaaccac aggagactgt 6120
   gagggaggtg ggcggaagac gtgggtggct caggggccat gaaccccata tgcgcctggg 6180
   agtgtgtgtg tgaaatgggg tttaggggag ggtgtgacca ctggacaaag cctgctctta 6240
   ttgtgtgtga ctattgtggc cttgggtttg tccctcttgg atcctcctgt ggtctttttg 6300
   ccttgactct aaccgggtga tgctgatgag caggtccaga ggcctgggct ctgttcacta 6360
   gcctgtgctg ggactctcag ccaagccttc tcagaaagga ggtgtcctct acatgtggtc 6420
   acaccatagc ctaggaccac agagggaatc atggtggcag agcctccagc ccccctgccc 6480
   tcccattgcc caaacaccca gatgtggaga tgccctgccc cggggtgccg tcccaagcct 6540
   gctttctaac atcaggaggt gggtcttgcc taaggctcct aaccaggacc tcatttgttg 6600
   tttacacatg ttggggtccc aaggatatgt ttcttggccc acagaatggt cataaacaac 6660
   tgccctgtga aggagggggt gctgtcttgt gtacagatga ggcaaaatga caggatcctg 6720
   tccccagctg cagagtgtct gctgggcgtc cccaacacct gggaagtctg tctgtccttc 6780
   caagggacca ggccagagaa atggggggca cctggggctc tgctttgcct cctcatggat 6840
   gtcagtcccg ggtttctgat ggggtttgtg atgatggcgc cctaaaggga agctctgtgg 6900
   agagccaggg gtctcgagta tggccttcat ggcagggacc ccattgctct ccttttttac 6960
   acatggcctc aaaccaggtt gactttttag cctaaagaaa atctcatgaa gagaaagaca 7020
   gtggtgtggg ggagggtgta tcacttgagc ctacttcaca ggctcttggg aaggagcagt 7080
   aggaggagga ggggtgcaag tagtgaaccc cagagggatg actctgctgt gatgactgtt 7140
   aatgaggctt gagacttcag ctcatttgtc atgtgcagct ggtgtatttt gagtgatata 7200
   agttgatgac agcctccttc ctgaacccac tgtcaaggct gcaagaaatt tttgagatcc 7260
   cacatccaac cccttgtgta acaggcaaaa caggcctcag gttctgaacc aaggtcacac 7320
   tgcaagtttg tggtagactc agaattagaa cgcaggccac ctgctcaggg ttctttctct 7380
   gtactgcagg agagggaacc agcatcactg tgtgggagtc aaggggcttc tcaggagtga 7440
   gtagtccttt ggccatgacc ttgccttatg tgaatggaag ttctgagcat agtgtgaact 7500
   gtatgtctac agctgatggc tgcctggcag ggactcttgg ccatactggg tttctgggct 7560
   ggctcatttc tggtgtttgg tatctctcta cttcacgttg aatggctctt ccctgttgaa 7620
   aggctctgcc agtatccctt aatatcaggt aaaactgcac aattcaagat ggtgccagca 7680
   agcatgatgt ttcccccatg gtgacacagg gctgtaagta gggagatgag gagccaggca 7740
   tctttggaat tgagggctga cttatagggc tgacttatct ccgtaggttt ggaatagtca 7800
   taattaatgg caaagttaat ggtccagcca cttagggata tagcaaagca agagaatctt 7860
   ggcttgttgt cttcaacttg ttgaagaata tcatctccat ttcatccacc cattacttgg 7920
   catcttggga tcatttgaaa ctgttccacc tgtgaaacct taatctttgt gtacctcgta 7980
   tgcattctgc ctcccatatc tccaccatgt ctgttcatag agacctcagg cccctgagcc 8040
   ctagtttttc tcctcactct gaatcaactc ttgttcctat tctatctgag ctctgttgct 8100
   tgtcacttca actgcccacc attcaccacc tggcctctta gtctcctacc tcaaccttag 8160
   agcagggtca ctgcctgctt tctctagtcc tggtttaaag gtgtgggggg aattccttcg 8220
   tgaattgaca ttggtccaaa ttcatggccc gcagcctcca gcgagccccc agcaatgccc 8280
   agcagtcctg tgacctgggc ctgctccaat tcctccacaa ctatttcaaa gctctcacat 8340
   tacccactta ttgggatata attcatatac catacaagtt gcctgtttat agtgtacagt 8400
   tcaatggttt taagttgtat gaccatcact acaatttatt ttagaacatt ttcattactc 8460
   cccccaatga aaacccatac ccatttgcag tctccccctt ttcctacacc ccccagcctt 8520
   aggcagccac taatctattt tctatcttta tagatttgcc tattctggac atttcatgta 8580
   aatggaatca cataatatgt ggtcttttgt aactggcttc tttccttagt ataatatttt 8640
   ctaggttcat ctgtgtcata gcatttatca atacttcatt tctttttatt gctgaataat 8700
   attcaattgt atggatatac cacattttct ttatctattc atcaattgat gaacatttgg 8760
   gtgtttccac attttagcta ttataaataa tgctgatata aactcttgtg tccaggtttt 8820
   tatagggaca tatgttttca ctccttgggt atatgcctag gatctatgtt caaccttttg 8880
   aggaactgtc agagtgtttt cccaagtggt tgctgcattt tacatcccca ccagtagtat 8940
   atgagggtct cagttttttc atattcttgc caatacatgt tattatctgc aattttaagt 9000
   atagtcatcc tagtaagtgt gaagtagtat ctcattgtgg tttggcttac atttccctga 9060
   tcactaatga tgactaacaa tgtgcttatg gaccatttgt atatcttcct tggaaaaatg 9120
   tctattcata tctcttatcc attttttaaa ttgggttgcc tttttattat tgagttgaag 9180
   cattctttat atattctagg taaaaacaag acccttatca gatatatggt ttgcagatat 9240
   tttattctat tctgtggttg tcttcacttt cttgatggtg tcttttgcaa caccaatatt 9300
   ttacattttt atgaagtcta atttatctag tttccttttg ttgcttgtgc ttttggtgtc 9360
   acatgtaaga aactgtagga ttttttacaa agatttttgc ctatgttttc ttctaagagc 9420
   tttatagttt tggctctttc atttaggtct ttgaatcatt ttgagttaat tttgttattg 9480
   gtgtgaggta ggggtcccaa ctcattcatt tgaatatgga tattcagttg tcccagcacc 9540
   atttggtgaa aaaaccattt tacctccatt gaatggtctc gctatcctgt tgtaaatcag 9600
   ttgactgtaa atgtaagttt atttctgggc tctcaattcc actgctttga cctatatgtc 9660
   tatctttata ccagtaccac attaccttga ttactggagc tttgtggtaa gttttgagat 9720
   caggatgagt tctccacctt tgttcttttt caaggttgtt tggctattct gtatccctta 9780
   attttttctt acaaatttta gaatctgctt gtcaatttcc acaaagaagc cagccagtat 9840
   tctaatggag attggaccga atctgtagat cagtttggag agtattgcca tcttaatagt 9900
   atatagtctt ttgatccata aatacaggat gtcttcttgt ttattattta ggtcttcttt 9960
   aatttctttc aaagaagttt atagttttca atgtataata ttgcacttat ttttagcagt 10020
   ttattcctca gtgttttatt cttcttgatg cttttgtaaa ttgaattgct ttcttaatgc 10080
   aagtgtatag aactatgtat aactgatttt gtgtaatgat cttgtatcct gcaaacttgc 10140
   tgaacttgtt tattagttac aatcattttt tagtggataa cttagatttt tattacacaa 10200
   gattatgtct tgtatgagta gagatagttt tatttgttcc tttctaatct ggatgccttt 10260
   tgtttcattt tcttgcctaa tcatcttggc taaaacctcc agtacaatgt tgaatacatt 10320
   tgtctagaat gagcatctgc cccaccccac cccccttctt ctggctgctc ttaagatttt 10380
   tctctttatc gttggctttc aacaattgat tatgtgatgt cttggtatag ttttcttcac 10440
   gtttcttcca ctgaatgtct tttgagtttc ttggatatat gggtatatgg tgttcattaa 10500
   atttgaagaa acattgggca ttatttcttc taatattttt tctgactttt cccctgaaac 10560
   tccaactatg tgtatgttag cttgcttaat attgttccag agtttttcat ttttcagtat 10620
   ttttctgtgt tttattttga aatatttcta cctatgcctt tgaatttacg aatctttgct 10680
   ttgcagtgtc taattccatg aagtatatat tttattttag atactatgtt tttcatctct 10740
   tgaagcttga cttgcatctt aattatgtat ttcatttctc tcctcatcat agtcatattt 10800
   ttctctatct tttttacgtg gagtgagttt atcatagctc ttttaacatt tatgtctgct 10860
   aattctgtca tctgtgtctt cctacttgta ctgtttttac ccacaacact tctaacacta 10920
   aatgtgtggt ttctttccac accaacaacc aatttggcaa ctctctagat gccaactgag 10980
   ggtcctgcaa tttaatttga ttctggtact aattattata actgaagtta atgcagaccc 11040
   cacagtttaa gggctcagtc ccgcaagact tcccccattt cataagtccc aggttgtcac 11100
   ctatacttct ggccaaccag ctataaattg ggggttccta ggacccctgc tcaggtatga 11160
   acatttgcta gaatggctaa cagaactcag gaaagcactt tatttactct ttactgattt 11220
   attataaagg atacaactca ggaacagcca aatggaagag acacctaggg caaggtatgc 11280
   aatggcgtgg agcttccatg acttctccag gtgtgccacc tccccagcac cccgatatgt 11340
   ttacctgcca ggaactctcc aaactctgtc atttagggtt ttatggaggt cctgttacat 11400
   aggtacaact gattaaatca ttgaccattg gtgagtgaac tcaatctcta gcccttctcc 11460
   tttccccagt ggcttagggg tgagctgcaa gttctaatcc tctaatcaca tggttggttc 11520
   ctttggcagc cagcctccat cattaagcta tctaggagcc caccaagagt cacctcatta 11580
   gaataaactc aggtatggtt gaaagtggct tattgagaat aaaaaagatg ttcctatcat 11640
   gcaagaattt ccaagggttt tagaagctct gtgtcaagaa ctggggacaa agaccaaata 11700
   tatatttctt cttgtgtgca ctggtgattg atttttctcc tagttatgca tcttctcctc 11760
   ctcctttttt gaatgcctgg gtaatttttg attagatgat aaatgttttg gattttacaa 11820
   tgttgggctt tgttttatcc tttaatagtt aatggtgggc tttgttctga caaacaatta 11880
   agttacaggt tgagtatccg gaatatccga aacctgaaac gctccaaaat ctgaaacttt 11940
   ttgagagcca aatgacactc aaaggaaggg ctcattggag catttcagat tagggatgct 12000
   gaaccagtaa gtataataca aatattccaa aacttgaaaa aatccaaaat ctgaaacact 12060
   tctggtctca ggcattttgg ataagggata ttcatcctat acttggaatc agttggatcc 12120
   ttctggggtt ttcttttagg ctttgttagg gcaggtccag agcagccctt ggccgagggc 12180
   taagttggcc tcactactaa agagtgctct tctgaggacc ctccctgatc tgatgttctg 12240
   tgtattagga ggtctttctg ctcggactgg tgggaacaca gagtatttcc acccttctgt 12300
   gaatttctga gattgctctg cttacttctc tctgtggttc attccccagc cttgggtcgt 12360
   tttctcacaa gcatgtgcag atcagtactc agccagagat aagggaagcc ctctacaggc 12420
   ctccgaagca catttgtgcg ttatctccct cccctccctc catccctcct tctctttctc 12480
   agtctcttgc tctctcactc tctagctctt ccttctggaa accctttcca gggaataatc 12540
   agggcacttg cagggttcac tttgtttgtt ctctttcctc aggatcattg ccttgcacca 12600
   ccttttgtcc aatgtctgaa ctgtcgtttc atctatttca ccggttttta aatgttaagg 12660
   cagaaatgta aatctaggcc ctgttactcc attgtgactg aaggcagaag tctctcctta 12720
   gttttaggct caccatttta agtgcttact ctacattccc aactggatat cacacagacc 12780
   cctcaaactc aatgcatcca aactaacatc ttctctcacc aacctgtccc tcgtcttcta 12840
   tttcctatct cctgggggaa acccagcccc aaatcttaaa ggccactgtg acctcttttt 12900
   caccattccc tgatatttaa ttaaataaaa tcctttcaat ttcatattct ttgttaggcc 12960
   caaatctgtg catctctttc ttttctctct gaccaccaga ttacctaggt cctcacggta 13020
   tttagtctaa gtgactatat agcagcctgc aaactgcctc tttgcctcca gcctatctta 13080
   ctccaagcca tgggccacac tgaagccaga gtcagttttc caaagcatag ataggatcct 13140
   atcatccccc acttaatgta tttcagtgga ttctctgaag cagggctgtc caattttttg 13200
   gcttccttgg gccacattgg aagaagaaaa attgtcttgg gccacacata aaatacacta 13260
   gcactaacga cagctgcttc acttttttaa aattcgcaaa aaattttcat aatattttaa 13320
   gaaagtttac aaatttatgt tgggccacac tcaaagccat cctgggctac gtgcagcctg 13380
   caggctgcag ggtggacaag cttgctctaa agctttagca cagagcactg cattcttagt 13440
   ggagacaaaa ggctgtttac tatctggttc ctgttatatt ctccagcatc gtctcctctc 13500
   ctgcctctcc ccactgccag ctgcaccagc cccccaaact catgtactct tttccatttc 13560
   tgggcttcac ctgtgactcc ttccatatgt agtgctcttc cctgtcttca cctggtagac 13620
   ttctcaccag ccttcaagac ttggtatcac ctcctccagg aagcctactc agatgtcctc 13680
   aatcacatgc agtgctaaac tctatcacaa gtcttagtaa atttatatca atcatgattc 13740
   catccaaatc tactactggg ctgtgatctc tctgagggta agaacgtatc ttgcttagct 13800
   cagtaccctc agtggaaagc catgcacaga ataggtactc attataagtg cactggatga 13860
   aggaatggag gtgtggagcc atggctggtg gaaatgagat ccaaggccag atgccctcga 13920
   gtggcctgga gtcccaatag cagaagggct ctgtgcagca gggccttcct cctagattca 13980
   ggcaatattg agcttgaggg tttgtgctgc ctccctgctc tactgcacag gggctagact 14040
   taaggaactt gccattgctg catttatacc ttttgtttca aagggatccc tgatgaatca 14100
   tccaaacctg cacggagcct gagccccagg gagggtcctt ggggtggctg ggccatctcc 14160
   tgcatgtgac ttagaaactg tgccacaaag agcctgatgc atcacaggac atttttcttg 14220
   aaaagagatt ttagaatagg atgtgatgaa ggtctttgat gactgtgatg tgccaccatg 14280
   caatataact ctgctagaaa gggtcccatt tgctcatcca ttttaataat tatttatatc 14340
   cttcactgtc atttgattac aagcaccatg aaggcagggc tgtatctttt ttttttcatt 14400
   gttgtgtcct cagcctacag tgcatgtaca cagtgagcac tcagtaaata attttcaaat 14460
   gaataataat agctaacatt tgttgaggac ttataccaca catgagtcta ggccatttac 14520
   acacattaac tcatttcata gtcacagtaa ccctatgagg tgagtactgt tatcattctc 14580
   attttgtgga agaaaaatta gacacatagt ggttaagtaa attgcctgag gtcacatagt 14640
   taataactgg tgaggaaagc tttcaaagac ttcatgaatg aatgaataag tgaatgaatg 14700
   ttttaattta gagtgtccac aaaaagggac tacaatagat gaaggtgctt tttgttttgt 14760
   ttgttttctt ttttagattt ttttaatgag atgaggtctt gttctgtcac tcaggctgga 14820
   acatggtgat gcgatcatgg ctcactgcag cccagaagtc ctgggctcaa gtgatcctcc 14880
   cacctcaatc tactgaggac ctggaactgt aggaatatgc tgccagaccc agctaaatct 14940
   ttgttttctt ttttgtggag acagggcctc attatgttgc ccaggccagt ctggaactcc 15000
   tggtctcaag caatcctccc aacttggcct cctaaagtgc tgggattaca ggcatgagcc 15060
   actatgcctg gctgaaagct gtatttttta aaacaatcat cagagcatca gactttccat 15120
   aatttggact attcatactc aatatcaatt aatatatttc tctatataat atgtaatgtt 15180
   gtaatgttat gtaattatat gtaagattaa atatatgcaa ttaagtataa tattacatat 15240
   agcatatatc atttgacatt actgcatgcc agactctgta ttgttttaca taaattatca 15300
   tatttaatat tcgtaattct gtcaaagaga tatgagaatc cctatgaatt cccatggtac 15360
   agctaaagct caaagtggtt aacctcaaac atagagagct tactgcaagg caggatttga 15420
   acccaagtct atttgactcc atattcgtgc tcttacctgc tttgccatcc tctctcttct 15480
   gtagtcggcc caaagcatgg gctaccttgt gctttccaaa cccttgccct tctaggaagc 15540
   taccaaaccc tatggcatag tcttcatttc acagcctaac ccaaggggac tttcagtgcc 15600
   ccaggcaggt tttcccaacc cccatccagt aggaagagct gaggcttcca cagctcagac 15660
   cccatggtgt gtcatcctgc tattttagaa cttgaagggt gccgtattgg aggccatcct 15720
   agaccaacta aatcagagtc attgagagta gggcccaaga atctgaattt taaatagatc 15780
   tccaggtgat tctgatgtcc aggcagattt gagaaccact ggccgaggcc cagcaaagag 15840
   acaatgcata cccctcagga aaagaagtag tggcttattg cccaggccac cttcacagca 15900
   ccttttagaa ccatggtggt attatgtggt atatatatat atatatatag atagatagat 15960
   agatagatag atacacacac acatacatat aaaataaaat cacattactt ttattgtgta 16020
   attttattat gtaattttat tatataacca taataacata tgtatgtaat cactacatat 16080
   tcattgtagt aaatttggaa tacataaaac aatataagga agaaaaataa gatgacctat 16140
   agttccaaca cccagaggta attcctactg acattttgat taattttcat ctttaaattt 16200
   ttaattgaag tatatatagt aacatggaaa tacacaaatt ataagtggac agcttatgat 16260
   gaatttgcac agctgaacac agacagtcat gttaacacta cccagaccaa gaaacacaac 16320
   atttgccagc cccctagaag cccacgttga gcccttttct gataaatgca ccctcccaaa 16380
   gtagtgatcc cgacttttaa catcatagat taattttgcc tgtgtttgac atttatataa 16440
   gtgggataat ttattatcta ctctttgagg ctggtttatt ttgttcaata ttaatttgca 16500
   agatgcatgt atgtcaggta tgtagttata cggtagttca tttattattg ctgtatagta 16560
   ttcaattgtg tgactgttgt agtttgtata tgcattctac cgttcatgga catttggaaa 16620
   gtttaaagta tttggcagtt acgaatagtg ctgctataag cattcttgta catatctttt 16680
   ggtgcatgcg tctgtgtttc catgaggtgc gtacctagga gtggaactgc tgaatcttat 16740
   gttccattct agtggatgct gccaaagttt tccagaattg ttgtaccagt acactttcac 16800
   cagcaatata tgaatcccag ctgtcccata tctccaccaa cacttggtct tttctgtctt 16860
   tttattttag ctgttctggt gagtgtgttg catgtggtct tgtggtttta ctttacattt 16920
   tcctggtgaa taatgaagtt gagcatcttt tcattcatgt atttcttttt aatcttttac 16980
   ttattcaccc actcctggta atccttaatt catttttttt aaaaaagtga ataaggccaa 17040
   aaagctttcc ataaataaaa cggcctggtt aaagtactat tttatttttc ccttcagcga 17100
   agtctgtcat cttaaaaata agtgccatct aactagaaga tattaaagaa aatgtgtttt 17160
   ctgctcttct gtctcttagc tcttttttcc tatgctttca ataatctttt aacttttact 17220
   ggttccacaa taaaattgca tccttttgag ggatgtaatc ttatgagaca attgcacaca 17280
   tattctccaa agaaagacta gtaggaccat taaaacagag gaaaagctta aagtcaaggt 17340
   gagacaggaa gctttcaagt agctaacggc caggaccagt tgttccactg ggaaccgtca 17400
   ggaggggagc ccggggagtg gagtagtgag catctcagag tggatgctga aaatgaagtt 17460
   gtttctttta tttgctttgt gttttgctct ttgttactga aaaaaaaatg ggcttaaatt 17520
   cttaggtgct actttgaaaa tgatctcggg tgttacctga aaaaaatgtg cagtatgagt 17580
   ttgttcacaa gtggcaatta catatgtttg tgtgtgccta tatcctaatg acattgtgta 17640
   aactgttttg tcatctaaat ttttcatgta acatactttc ccatattgtt aaatattatc 17700
   gggaaaatac tgtttttaat ggctatataa tagctctttt atggatttgt tgaaatttaa 17760
   tcattccttt attttggtta tttccataat ttctaaattc tcaatgatga atatatttgt 17820
   acataaatct ctgtactctt gtgatcactt tcttagaata gcttcagaga aatgaaatta 17880
   atggctcaaa aagaatgaat ctctttaaag ttctttgata cattcatatt atcacatttc 17940
   tttccagaaa ggctatcaat ccactctctc cgcacagtat ctgaagggcc accctcactg 18000
   catattcgcc agctttgcac tcacttcaat tacagtgtct cttgccatat agaagacggt 18060
   gatttttacc taataaactt ttatggcttc tggatttagg aacatgctta gaaaagctcc 18120
   cccactccct gcacatgact agaagaatac tctctaacat gttctactta acagttttct 18180
   tttttgtaat aaacctttaa tcctctgtta tttgtttttg tatgtgatgt gaaataggga 18240
   tatagcttta tttgccccaa atggatagca agttgatctc taagccactt actaaataat 18300
   ccaatgtttc ttcagtattt tgaaaggcca tctttattgt aaaccaattt cctctatata 18360
   catggtacta tttctgtact gcattggtcc attggtcatt ctcacgtcga taccactgtt 18420
   tttggtacag tttcctagta cagatgattt attggagatt catagcacag tttgataact 18480
   ggcatggcaa atgcctcctc attcttttac ggttacaaaa ttgtcttggc taacttggaa 18540
   aactggctta tttctccaga tgaattttgg aataaatttg tccagttgaa aaaaaattct 18600
   gctgcattta tgattagaat gggatcacac ctgtcaattt atttgacaaa attgcagttc 18660
   catacaggaa caggggatgc atctcctttt atcaaggatg ttaatataag taaaatttta 18720
   tagtttgttt tattattaat agaattgtag tttgttttca tgtatgtcga aggcatttct 18780
   tgtgaagctt ccctagatag gttatttttt ctattgcttg tgtgaatagg accttttttt 18840
   tttttttttt tttttgagac ggagtctcgc tctgtctccc aggctggagt gcagtggtgc 18900
   aatctcggct cactgcaagc tctgcctcct gggttcatgc cattctcctg cctcagcctc 18960
   tcatgtagcc gggactacag gtgcccacca ccacgccctg ctaatttttt gtttttgtat 19020
   ttttagtaga gatgaggttt caccgtgtta gccaggatga tctcgatctc ctgacctcgt 19080
   gatccggcca ccttggcctc acaaagtgct gggattacag gcgtgagcca ctgtgcccgg 19140
   ccggaccttt ttaattatat ttttaaattg gatgtttctg atatgtagaa aagccattta 19200
   tttacaggta gttattctgt attaatcaat gccactgact tatttattaa ttctaatagt 19260
   ttttcagtta attttcttag attttgctag gtagacagtt ggatatgcaa ataatgatca 19320
   gtttatcttc ttttctaata attatgtgaa aaaataaatt attgcattgg ctagaactta 19380
   taaaacaatg cttagttata ttctttatcc tgtcaactat acttctgttc ctagtttact 19440
   aataaatttc ttttaaccaa aaatgagtgc tgagttttat ccaagacctc tttggtatct 19500
   gttaaaactt acgacattta tcattaggct tattaatgtg atgtattaga ttaatagggt 19560
   tactcatata taaaccatct ttccatttcc ggaaatggaa tgagctctat atggtgcatg 19620
   ttacattttt cattcaactt acttctaggt ttgatttgtt aatcttttat tgagaatgct 19680
   aaatatattt ttaataagta aggctattaa gtattggagt tttaggtttc tggggagtta 19740
   gatttttata tcagggtttg attaaccttg cagaattagt tggttgcatc tcatcttttt 19800
   ttatgtattg ctgagtgatt cctctaactc aataattgtc tgtttttatt tatttatctt 19860
   tatttattaa tttatctatt tattttgaga cagagtctcg ctctgtcgcc caggctggag 19920
   tgcagttgca cgatctcggc tcactgcaag ctccgcctcc tgagttcacg ccattctcct 19980
   gcctcagtct ccggagtagc tgggactacc ggcgcccgcc acgacgcccg gcgaattttt 20040
   ttgtattttt aatagagacg gggtttcacc gtgttagcca ggatggtctc gatctcctga 20100
   cctcgcgatc cgcctgcctt ggcctcccaa agtgctggga ttacaggcgt gagccaccgt 20160
   gcccggccaa taattgtcgt ttttaaaagt ttgaaatact tggccgggcg cagtggctca 20220
   cgcctgtaat cccagcactt tgggaggcca aggcaggcag atcaagaggt caggagatcg 20280
   agaccatcct ggctaacacg gtgaaacccc gtctctacta aaaatacaaa aatattagcc 20340
   agacgtggtg gcgggccctg tagtcccagc tactcgggag gctgaggcag gagaatggtg 20400
   tgaacccggg aggcggagct tgcagtgagc cgagatctca ccactgtact ccagccgggg 20460
   cgacagagcg agactctgtc tcaaaaagaa aaaaaagttt gaaatacttg tcccaaaatt 20520
   catcttggtc tggcaccttt cttgtctgga gctgtttagc aacatttcca gtttcttcta 20580
   tggttattaa tctaatcaac ttttctactt ctttttaagt ttagagattt atattttcta 20640
   gaaagttcat tgtgttgaga tttttaattt attagtctag aaatatagta agattcagat 20700
   gtgtacagtc tcatctgtat ctttgattac agccctttct taagggtttg attcttttat 20760
   ggtcctctga ctttccacat gcccctcctt gggcctggca ggggcaaggg ctggagagag 20820
   agagagagag agagacagag agagagcaca agtgggagta gggaacagtt gtgggcccat 20880
   gtccatttcc atcaattgga atggatatgt gtgagcctgt agacatgtcc tcacctgcat 20940
   gccatgtcat ctcccagccc gaatgtctgt tgtggcatat ctatgcatac accttgatgt 21000
   gtgtcctggc atatgtatgt gtgtatgtgt tcctccatat atgtgtcagc atgcatgcac 21060
   acatggtctc tgcagccatg gagatttcca ctttgtgtga atattgtgta catgttcctg 21120
   tgtgtactgt acctgtgttc atacatgtgc atgggtgtat gttcctgtgt tactgtcact 21180
   gggaggcaca tggcacatct ggctaaaggg atggtagagt agctcagcac agccttggtg 21240
   cttacatggc ttattctctg ccattttaaa caatctctca aagggccaga aggtgaagag 21300
   gaggtgggct tgggcaagat ggggtgtgga ggctggcccg tcctagagtc actctgtcat 21360
   gactaaccca agtctggagc ccaggcctag atgctcagcc tgtgtcccct ggaaaccccc 21420
   tcagctagac agagaggaga gggtttactg taactcatca gtcagggcag ctgtctgctg 21480
   ttgccatgga aatctgaagg cagcatcttc tgcaaggacc aggatgtaga gtctagagag 21540
   gacaggctgc atcagcctgg tgggatcagt ctggagtggc ctgtgtgtgt gcgtgtgtga 21600
   gagagagaga ggtgggagga gaatgcaaga tagtgagtga gctttctgaa gtgggtggcg 21660
   catacctcac cattgctctc taaggcctca gccattactc tggattatcg ctgggtggcg 21720
   ctgtaagaag gggtgtaccc ctgagatggc ttccctgtgc ccatcaggac ccggtctggc 21780
   ctgaggctgc tcttagagtg tcctgggacc acctagacag atgtgctggc atctgcccat 21840
   catgggcccc agagcccacc cagcatctgc tgcacactgg ctcctccttg ctcagatgat 21900
   ggggtggtcc aggaggcagg cccttaccct ggccgcacag atgctgggtg atgggagcag 21960
   acagacagag gcccctcacc ccaggactca gctctgctgc tgatgggctg ggggtggggg 22020
   caaggacagg gcaagagtac aggatgatga ctgggggaca ggatagagtc caaccttcag 22080
   gtggagctga gggctgggac agccaatgac agtgtgtgcc tctgccaccg cctcagtctg 22140
   gcaaggcata ggaaaggagc acccccttgg agtcctgaca gcacctgtgg agttggaagc 22200
   aggagccctg gcctccctgc tcaccttccc actagcccct tcgctcaggg ccctgtgact 22260
   tctgatccct ccctgtgaca ggggagcctg ggctcagcgt tcactgcagc ctgggcaagg 22320
   cccttaagcc cccaagagtc ccgctctcac agggcatggg aggagcttcc agaagcagat 22380
   gggtgtcctt gtttgggttg ggggtgctgg ggagagcagg aaggagctgg ctgctggagc 22440
   cgcctgaggc tggatctgga ggacacagag gtgtttagcc aacagttctg tcctatccct 22500
   tctcagctct ctgaagtcct ccctgcacct caagccaggg cctgacctcc agggaagcaa 22560
   gctgtcacca cgaggaagtg caccctagct ttcccacctc gtgctaccag gatgcccctc 22620
   actctcctta atccagggca tgggctcaga gggctggctg gggtctcctg tcctggaatg 22680
   tgcggccata tgggaatgcc ttgagtgtgt gagggccata tgggaatgcc tgtgcgtggg 22740
   ggtggcatga gtgtgtgctg tgaggtgtga gtttggagag aagtataaat aaatgatggg 22800
   cacatgtgtg tggtgcgcat gcttgtgctg acaggggaag gcagggggac gtctggagtg 22860
   tcagtgggtt gtggtgggca gggtgaggca tccatttctg tgaaatgcct ggggggaggg 22920
   aaaaggtatt tggcacagta catgtgggta caaagcacac gaaggaattt tgggtacata 22980
   tgcgcatggg agcctttgtg gggtgtggtt tgggtatgca ggtaggctgt gtgtgaggta 23040
   gagctggagg aaaatctatc tgtggagtgt ctgaggtgat gtgtgggagg cagctgtcta 23100
   caggtgagct tgtctctgag tggtatatgg ggtgacatgt tgttgtgtgt gatgtggttt 23160
   gggagggtga ggggtgtgca ggcatgtccc caaaacaagc acatggtgtg tctcaagtaa 23220
   ggtgtggggg cagtgatggg aacgaactac agtatgtagg ccaagtgtgt gggggaaggg 23280
   caagtgggcg tttcattctc tccacacgtc acgagttagg aggcaccatt ttccccctca 23340
   aagttgagga aactgaggcc cacagagtgg aaacaagctg gctggatcac acagcagggt 23400
   ctgttagact ctcaggatcc cagctctggc agctcacaag ctaggctctg gagggctggg 23460
   gaggggactg gccctctggg taatggcagt aatggagggt cccattcagt tctttagggt 23520
   gactctcctg gaactggccc acagacagct catgagaatc gattgcgcaa taaaggaaaa 23580
   tgtttgtaag ccctttgctc aacatagctg ttattaagca tcaaactggc cgggcacagt 23640
   tactcgcgcc tgtaatccca gtgttcctgg accaaaccga gggtcagctg cttattctca 23700
   aggcccagta acgagatgca gatgaactgg gagagagggg agtttttatt tctgtaaccg 23760
   gtcacaggga gaaggcctgg aaattattgc cagaccaact caaaattaca aagttttcca 23820
   gagcttatat atcttctaaa ctatatgtct atgtgtaagt gtgcattcgt ctaaagacat 23880
   aagtgattaa cttcttttaa tctataacta agattcgagt cctgaagacc ttcctctggg 23940
   gcctcagtaa atttacttaa tctagatggg tccaggtgcc aggggtaatt accctcctta 24000
   tcttgtcccc tgcgaagtca cggaggtttg gggagttcct tcagccctcc aataaaactt 24060
   atttgtggag gcctggggag tttcttcaga cccccaataa aacttgttta accttaaacg 24120
   aggcctgtta agaattcctt cattattttg tcatgcttta aggcccagga aaggcctagg 24180
   caaaactctt ggtgggcttt tgttacatcc cagcctttgt ataagggcac tggttctgtc 24240
   agcttttaat atttgactta accactcagt cagtactgaa acagttgtta tggaggcctg 24300
   tgttagtgat gcctggcctg ccgcaccagc actttgggag gccgaggcag gaggattgct 24360
   tgaggccagg agttcgagac cagcctgggc aacatggtga gacccccatc tctacaaaaa 24420
   aatacaaaaa ttagccaggc atggtggtac atgcctgtag tcccagctac tcaggaggct 24480
   gaggtgggag gatcacttga gcctaggagg tcaaggccac agtgagccga gatcacacca 24540
   ctgcactcca gcctgggcta cagagcaaga tcctctctct caaaaattaa aaaaaaaaaa 24600
   aaaaagaagc ggtataaatt taacattaaa caagttatgt ttacaaaggt aatatactga 24660
   aaactcacca cttcgtattc attttactgc ctttccctgc gcttgtctgt tgtgtctgcg 24720
   gggtggaact gccacgtgac cgtgcactgc tgagcatctc ctcgcacttc gatgctcagt 24780
   cacatcacag attggccatg gcgagggtgg ctagaccagg ggagtcaaca ggtgctgcac 24840
   attaagacct ggtttattgt tttgttgatt gtctagacct gagaaagtga tgggaaaaaa 24900
   tactactaat gcagattaca ttttaaaggg tgtcatgttt atagttgctc catggtgaat 24960
   agcagaaaac aaagattttt ccagtattca gaaactatta acctattcag caaagaagtc 25020
   actccgacca ccagtgaagg agtgaagttc tgacatgtct ttgttgtttc actttcaact 25080
   tacttgttaa cataaatgaa aatatcaagc agcattaagc tggaactaca gtaatttgtc 25140
   attgcaaccg taggttggct acagataaag agttaggcga aactcattga atgggttgat 25200
   ttctgggaca gtcatctggc tatatggaat ttacaataaa gaatattgga tatttcatta 25260
   gtatttgtaa attatgtgct atacatcctt tgtgtcagta aaatttataa taaacttatt 25320
   gagtctacgc atgcagacat tttctggaga gcttgttgtt aaatgtcact gttaacaccc 25380
   cgtaggccct ccattacaca tagtcccaac atgtctcctt actgtgcaga cggtgaggta 25440
   gggtggggtg gagggacaga tgtaacttgc agagaccctg ggagcagtgt cttaggtttc 25500
   cagacaagct gtggcttgtc actagatcag cctgacctcc tcctggccgg gggcactggg 25560
   gctcaggtgg gggtcccgtt cattggtccc ttctctgtag caggctcagg gatttacagg 25620
   tcagaggaag aagacgtggt cactgcctgt atggagctca cagcccagca gaggagacat 25680
   ccagtcgcag ttagttacac cctggagagt acaggggcaa aaaaagaaga aattgcccag 25740
   ctgaggcagg agcgaggctg ggacaagagg agctgctcag cggaacagtg cctctcgaca 25800
   tttagtgtgc agcatccaca gatttgtgtt aaaaatgcgg agattctctt ttggcaggtc 25860
   tgggcagggt ggtgcccggg agtatgctag gggatggtga agcaggtacc caagggcaca 25920
   ctgggaaaag ccttgatctg aggagacgag agggtgtgtg ctgaggcagg agatgagaaa 25980
   gaagttctgc ggggagatct gccgggagtt taggagtggc tggagctaag ccagcctgga 26040
   ggggagacag cagagcaagg caggagaggg gtcagggggc aggtcctgca gcctcgtaag 26100
   cctcatggaa gagcttggtt tgtatccttt ggaacgagga gcagtggaaa gagccttgtc 26160
   agcagacaag tgacatgctc gggttggctt ttaaaagtgt aatgttcctg gagcaggcag 26220
   aactgcagtc aaggagacca gaaggaggct ggggacatgt gaggcctcga tccgggcaga 26280
   ggcactgggg acaggggagg ggaccgatag gagggcttca tgggtggagc cacaggcgag 26340
   atgaggaggg ttagggtggg gcctgggttt ctggggtgat gactgagcga atggcaggac 26400
   tatgcacatt gccagaaaac aggaggaaga gcaggtctgg agtaaggtga gttgctttga 26460
   acctggagac ttagaggtat ccctggaggg gcccagaggc atttggggct caggggagcc 26520
   atcaggaagt agatttggga gccccatgga taggtgggaa agccaagaga gtgaagaggt 26580
   cacctgcgga gaacacagtc acaagggtcc agaactagtg ctgaagggcc aagaactcag 26640
   gcctgcctgg accttgtttc tggaaggggt gccagatgcc aggtccctcc gtacaggaaa 26700
   accagagagt gggcaggaga aggttggagg aggtcagaac catgggtcgg gttgctggcc 26760
   acctctgact taggagttgg aggccacagt ggttaggagt catggggcct cagttctagc 26820
   tccagccctg gctctgctac caacttgctg tgtgaccttt ggcagatctc tacccactct 26880
   gggcctcagc ttcttcatct gaaaataaga aagctggacc ataccagtga ttttcaaatg 26940
   acatttggag ccccagggtc acccagaagc atctccagga agcaggagtt gagggtgatg 27000
   gggagggaga gactgagagt gtggaaatct agtcccaccc tgtccccagc ttcatccaga 27060
   gccacttgca tttatgtttt ctgttagcct cttcatgaaa ggatgttttc caactgataa 27120
   ctactgggcc agaggatccc ttatactgtt gtacgtattt ggggtctgtg aatccttgcc 27180
   aacatagaag ctgatggagt ctgaggaatg acacacactg agaaaaccaa gcatcatcat 27240
   tacagacact gtgtgaactc cctgcctgcc acccacacag cacagtcatg gttatgtcca 27300
   tgcttatgta ggcctgggct ctgcctagct gcgggtgatt tcatcagtgg atgccgctca 27360
   gtttgcccct gcctgctggc tggctcgtcc ccagccccag gcatcagggt gccccatggt 27420
   ggggcctgtg tggccctggc ccttctctgc tgccccaggg ctgtgaagcc acaggtcggc 27480
   cccctcgttc cttgatgctc tctgtccatt gtctctacaa atacaaagag agtctctgaa 27540
   caggacaaag cacatttcct ccttccttgg gcctggcatc agcgacgatg ccacctgtgt 27600
   gggggaggac tcagtgggta gtgtcctggc ttcccttcag ccccacagag cttccctcaa 27660
   accttgctga cagctgggcg ctctcagtaa actgaggcaa gcctgggcct agaccagtgt 27720
   gtgggggcgt gtgctgtggg cagggaggat gagagagctg gcctcaggat gggggtggaa 27780
   acaccctgcg gtgcctctac agggaaaacc aggtccttca agtctgtgtg acctcagatc 27840
   ccaaataagt caatgccggc ctcccctccc cgtccctcag agcagggcct aggagttggg 27900
   gaggggcagc tttgcacacc ccttgccctg ggatcctggg gtgcaagagt aggacgagga 27960
   cagtcggctc agaggggaca gagatcttgc tagtttgctc tgtgcccctt tagcccacac 28020
   tcatgtccct gcatacactg gaccctccta ttgcctggaa gttgtcctgc ctcctgccgc 28080
   tgcccctacc cgctaagaga tccagatccc caaataaagc cacactggac cccaggatcc 28140
   caggcttgga gacagtgctt ttggggaact ccaaggtccc ccagctctct tgctacttca 28200
   ggaacagttt ggaggggggc ccagtagaag tgaccccctc cctgtcacct actcaacatc 28260
   ccagataggg ctcagaaagt tgctccaaaa ctcctgggaa gagaccccac ccctggagtt 28320
   ccttcctgct cctcctcctc tgttctcgct cccccaggac tatggaggct tcagcccgtt 28380
   ctcactcggc agggtggttg gtggggcagg gtgacgactc tcggtgtttc ctgacaacag 28440
   gctgggggac tcaagcgcca atgtgaagct gaagagcgtc ctggagacag ggggcggtga 28500
   gacggagtct gcccaggcca caccagtggc cggggtttgg acctgggcct ggggagggtc 28560
   gctgtttgag aggaggacgc agctttataa aatgactccg taagtatggc ctgggcaggt 28620
   gggggccgcg ggcccccact gccccacctg ggaccacggg catggaccaa gagctcctgc 28680
   agcaggagac cctgccacag gacgtgggga accagaaagc ttgggaggtt gcagtcctgg 28740
   ggagatacac atgcttgggc tgaggcggag gctggctctt ggcctctagg gatctaggga 28800
   gccaggcgct gtcaggggcc tgtggagggc tctcccagat gctctaggct actgggcagg 28860
   gccttcctct gaaggtttgt gtgcgtgtgt gtgtgtgtgt gtgtgtgtac acatgagggg 28920
   ttgcagtttc taccatggct gctggctcat gataagagcc ttttgaaccc aagaccccag 28980
   gaaggcaggc tcacaacctt ctttcttagg catcaaaggc tctagaacag tggtttgcat 29040
   catgagaagc cctaggaata tttttaaaat gcacattccc aggctctgcc cacagggact 29100
   ctagggcagg gcccaggaat ctttatttta gaaagctccc tgggtgattc tgacaggctg 29160
   cagctggggg aggaaaccac tgctcttggc ggacacctac ccggggccag gaagcccagg 29220
   ctctgcctac agggccacct tagacactca gacgttgtct ctggaaggtg ttcaggtcat 29280
   ctgcactctc gtgggaagga gaggccgaca tactcttcgg taaccttttc ctgaggcaca 29340
   ggagaaagca tggagcacag agtgggcccc agaggaagga aggctcagga aggggtgcga 29400
   cagagcatca gggccaggga ctgccccatc acagtgtctg ggcctgctga ggccggggac 29460
   cctcacatct gtctcagggc ccagcgcctc cggtccacag cagcccctgt ccagccacgt 29520
   gtgtgagccc aggtcctcct gcatagacac gaggaaagag agatgagatg tcagaaagga 29580
   ctccctggag atgagcgcac acaaggacag ttatgaaatc tcctccacgg agttccttgt 29640
   gagcatttta aatgtcttct taaaaaagaa ttcagttaat tcaaaatgac ctttagaaaa 29700
   agaaagtaaa ataaatgacc tttaaaaccc tcataattga catgaagttt actgacttaa 29760
   ctgaattaac atgaaggcag catgaagtag ccgactgcac gtttgcttcc atggttgaag 29820
   ctggagtcag gccatctgat tcggtttatt tcccgtcttg ccggcccaag gatgcacatt 29880
   cattttgccc cagactgcac atgtccacac ctcctggctg taaagcatcc gggagtgttc 29940
   actcgaccca gtttgcttta gtcaatcccc aagtgctgaa cctttgggct ccttccaatt 30000
   tcttgctatt ataaatatca tggctatgaa tatctctgta caaattactt tcttagtatt 30060
   ttactgggag gcattcccca agtggaacta ctaggttaaa gggcatgaac aattttatag 30120
   ttcttgtcac atattactag atggttctcc agaaatattg agcccgttta ctgtgacacc 30180
   aattatgtat agctgtcccc acagccctcc agtataaagg ttcatcattt ttatttattt 30240
   atagctgttt tatttacttt cttttttctc attttgcgat tttggtcagg agtggtgtgg 30300
   cgagttgttt taatgccgtt gtttcaatga ttggtgacat tggacattct ctgaaggact 30360
   ttgagaacct cataggcgtc atttgtccca cagggatatg gcaggatcgg ttctgagtct 30420
   aagagctgga tcaggactgg aaaatggata gctctgagtt ttcagactta ctcagttctc 30480
   agtctcccac caataggcca gaatgtacgt cagggccata gtttccaaag ctctgaggct 30540
   gaacgatgaa tatggtttta aaacagcatc acttgcccca gggagaagac tgagatgaag 30600
   gggagaagga gaacccgtgg ccccatgctt gtccccacct tgatggactg tgcctgagct 30660
   ccactggaca ggctgcacag cctcttaccc cagatgtgac cttctgggta cacagattac 30720
   catgttatgc ttgtggctgt ggccataggg ataaaccaaa tttggggctt gtagtacact 30780
   tgcctgttga tcctaatgag catccaggag ctgcaccaag agtttaccca gtacctgtaa 30840
   aaccagggct ctgatggtgg ctagagaggc ctggctgggt aagatcagta acatttgctg 30900
   gggtctgggc cctcatgtct tggctgaggt ctaagcatgg ggagttgtgg aaaaggagac 30960
   tgttagggaa ctttgacgta agtggggtcg aagactgtgg ccttcagata atgacagggg 31020
   cctcctcagt gataccagga actcaaggca gagacagtga gggtcagggg catgaaaccc 31080
   agtgatagaa actaggtgcc atggctgttg cagacagggc cacagggcct cctgggatct 31140
   cagagcaaag aggccagatg atgaggacag taagtcaggg cagacatggc tggtggggca 31200
   ggaggaacta gcataggatt tgaagtcagc aatttaaatc gtggtatcaa aggactccgc 31260
   aaacttggga acaaataagc agttatggat actcaaagat attgttggta ggagcagaag 31320
   agtgatttgt gcaaccaaag gaggtcttgg agtcccaggg gcctctccat gttgaagcag 31380
   gatgctaggg gatgagagga cacagggcca atgtataata taggtagcaa ggcccagcct 31440
   cttggaggag aattggtgga agctggctgg gggatcatca ctccagacta cacctggact 31500
   tgggaaggac cctgtctaca gaggccaagc caggatcatg accatcaggc taaggaggaa 31560
   gtgtgcatgt tgctggggta gcaggtgtct tgggcccact aaggtcctcc aggctttggg 31620
   aaggaagcag actccctttc cctctcattc ctggcctgct ccccatgggg ccagggcatt 31680
   ggtggtgctg gagcgggtgg taatggacag aggagacatg gtgtgtgccc caggttcaac 31740
   aatgggagtg acacagagtt aagaagtggt ttcgtgtgag tcttagattc aggtagacct 31800
   ataccttact gtgtgacctc agttcttgag cctcactttc ttttctctaa aatggggata 31860
   gtcgtacctg ccttataggg cagtcaagaa gattgaatga gaaaataata catgacagca 31920
   ctcagcaaga atagttgtta acaaatgtca gtttctttcc atttgcctct tttagtacct 31980
   agagcagagt tgagtgtatc tgtgggccca aataggcctc cactctagac gaggtggagg 32040
   gtaaaggaag gggcagcatg ccctctggta agcctaggag cagagctgga agagcagctc 32100
   tgcttcaggg tttatctgac ccaagggtaa tgatgaattt ggtgacgtgg aatagttaaa 32160
   gtctggcaag tttctgagcc tcgatttcct tatctgaaaa ataagttgtg tgtggccttg 32220
   tgacagtgca tggaaagccc ctagcaccac tcctgcacat agtaggaggt ccacacatac 32280
   atgacagtgt ggattctgtg gttgcagaca ctcctcttgc cctcagagtc atatcgcggt 32340
   gaagcgtttc tctctgtgag ctcagagtcc agcctgggat tggtcagaca tgaaataggt 32400
   tgtgcatggt ggtcagtgct tgggcggtgg ctaatcatgt caccccagag ttggccgagg 32460
   aggaaggggc ccggggggag gctgccctga ggctgtgggg gctccaaggg tactgccagg 32520
   ggaagaatgg gcactctgca gttctctcct ctagcccagg gtgttctgag agtgtccaga 32580
   accaacaggt ctgctcagac gctggacatg aggttccact tggcacacag catgacagcc 32640
   ttttcccctg caggagcgtt accagaggca ggcaggcagc atggaggtga agggaattgg 32700
   aagggcctgg catgagtcag gaatgagtga ggatgggggc gaaagcaact ccgtgcagtc 32760
   cagagggagc tggctctggg ttcagcccct gtacagctcc acagcctgtg ccctgacccg 32820
   gatcccagtc ccagccagtt actgtgcaca ggggactaag tgatgaacct cgttctctgg 32880
   aggcagcagg gctgtacaga gggtccagac aacctggaga gagagaggct gaagctgcct 32940
   cagacttccc tgggcagcca ccctgcttgg ggcaacaccg ctcagcagct agaggagttc 33000
   agggttctga atcccaatct gtcgcctgcg agctggagac ctggggcaag cctagcctgt 33060
   ctgactcctc ctgccctctt cagagctcac gtctaggaga ggtgtaataa ctgcgggtgt 33120
   ttgacgactt cagcactggt tatggaagca ggttgaagag ttgtgacgag acctgcatga 33180
   acagcctacc aggcgtgcag cccctgcact ttgcaaatga cctctagtcc ttgtcaccct 33240
   gggacaaccc tggatctttc tggacctgcc tgaatcctct ggataacctt tgcagcccat 33300
   tacctccccc aaatgcacac acattcacac acacagccat caccgtgtac agatggagct 33360
   ccttcctctc agtaagtttc tgacctccat cctgcccctc ccgctccagg cagctccact 33420
   cccggctcct gatctgctcc cattcctcca ccgtctggcc tcaccaaata ctggtctgtg 33480
   aatggcctca ctccgtaagt ctgacgccaa tgaggtccag cttctccagc tcattcttct 33540
   ggctccccac ctcctccagc aagatttaaa aatactgcgc atatgagagc cggcacaggg 33600
   cattgttgct ccccgtttcc tttcttgctg agtcagggtc taggagagca cccacaggga 33660
   gtcctgcagc cccaggtttg gcccctggct ctgctcctca tgaggtgtgt gaactcagcc 33720
   agttgtttta ccttttgaga cctgcttcct gatctataaa ctggaaataa taatagtacc 33780
   ttccctccag gctgtcgtaa agattaaatg ggaccctata tgctgttagc atggtgcctg 33840
   gcacagggta agcatttaat aaatgttagc tactgttatt attttgtgcc taacaaaata 33900
   cttagagtga atgacaggaa aaattctcct catgtattca cccgacaaat atttattaaa 33960
   ctcctgtttt cttccaggca ctgtactagg cactggggat acagcaggga gcaaaacagt 34020
   gaaaacccct gtcctcaagg agcttctatg ctggattcca cagccacaca ctcaagtctt 34080
   tgttgtcagc cctcctctcc cttagtctgc accctcccct gagcctctgc ctcagcctcc 34140
   tcccgggctc aggattccct gaatttccct ctcctcacac atcctgtctc taagctccag 34200
   atgcgctttt ccagtcactt gcaatggggt gacctgcaga cgctcaaacc tggcacatct 34260
   gaatagtaac tcacaatgcc caaccccttc tcaaacctgc tattcccact gggttccctg 34320
   aggagccact gtccagaccc ctcttcctcc aacatgcagc tcttctatcg ccatcgccac 34380
   catgaataga gcacttagtg caggcaccat gctaaaagct tttatttatt aaatcatgga 34440
   atcccctgac gtccctaaaa accattccac ttgtttgcag ataagaaaac cgaggcggaa 34500
   atcgttaaac agccttaggt tacacagtta ggcagagccc ggacaaagag cccaggctgt 34560
   ctggcctcag ttgtgctttt cctgccgctg tgctgtcctg cctcgggtcc catgggcttg 34620
   cctcagctgt accactgacc tcaggcccct cctgggccgc atctagctct gccctgggct 34680
   attgaagagc ctgctcactg gtatctctaa ttccctctgg cttcaccaca gaccctgctg 34740
   ccagggctgc ctatgatccc ctcccttaca ggcgggatca cttccccagc accaccacac 34800
   ccctgcaccc ctgcaatttt ccattgttta caggcagcct ggccctgttc cagaccaact 34860
   tccccagtcc catataacct ctccaagacc cttcttctcc ctactcattc cccacgtgtt 34920
   acagccaggc aggccgtcca ccccaatgct ttcctaactc ccgtggcttc ctcaccccgt 34980
   ctacctttac tgagctctgt atcctttaat acccagcctc aaaggccgtc tctgctgcaa 35040
   agccctgcct ggttccctgt cagactcctc tggctctctc tgctgtgccc ccacgggtct 35100
   tcacctctgg ctgggttgac ttcataccac ctgggattat tgtttgttat ctgtgtccat 35160
   ctgtgtcctc cagtaaccca tgatcttcac aggccagtgc cctgtgttag tcatctctgg 35220
   gtccccgtga ctgacggggc tgggtgcagt gcgagcctca gtactgaaat gaacagggcc 35280
   ggcctacgag gtcactccgc gatttggggc aggaaagatc tgagtttctt tctgcactcc 35340
   cgtcgagtcc tgctgtagaa accacaaatg gggaatgatc gttgaatccc agaagactga 35400
   gcaggaaggg ccgctgcagg tcatctgtgt taattcttta tctttgaaat gttggagtga 35460
   ggcccagaaa tgagaaagac ctagcccacg atcactccac agggaggagt tgtggttgga 35520
   attcagaccc ctggcttcta gcccagtgtg ctttctgctt tgctgaacca ttccatcttt 35580
   gctttttctc tacacaaacc acctactaca ataacaacaa ttgcttatgc tgaaaatcag 35640
   cccagtctca tagctctgga acctctcttt gtccccttcc agataaccac aggacacttt 35700
   attttacact tttgtcataa tgattttaca ctctcatttt taaatttagt attctttgta 35760
   ttctttcggg gggggggggt aattaaccct aaattcttgt agagaaatta agttcttaga 35820
   gtagatttct gggttgcttg ttttcttcca ttgacgtttc acatgacatt cctgtccacc 35880
   gtcacactgc ttcagttgtt gcagatgtgt attttcctat ctaatgggat tggtgctcct 35940
   ctcattagcc gtcatcagaa atgtctttta ttttcctatc taatgggatt ggcgctcctc 36000
   tcattagccg tcatcggaaa tgtctttgct tttcttacct gttttgtttg tttttgtttt 36060
   ttgctgttgt ttttttttcc ttccagataa tctatacctg ggttatctag aggaattagg 36120
   ggcagacgag tagcttatcc cagtctgccc taatcatggg catccacaca ggtcagctga 36180
   attggccact cttaattcat ggctctgaaa atggtaaaaa cggccattga ttgagagaaa 36240
   caggcaaaaa agagagtagt gggggcacca atgccctgac taccccaaac tctcactgct 36300
   gcccactaat ctgccatcct gggatgtggg gcaggatggc cgcaccgtgt gggcatgctc 36360
   tcacgggaaa atggaggtgg tgttgctgat cgacactagc cagcgaggca acatggccac 36420
   actggctttc ttatcacaga gcccacgttc acaaaccatc ttggggctcg ccctcctttc 36480
   cttaaatcat caccacaggg aagcataaaa aaatctctgt acattatcaa gtttcagaat 36540
   ccccttggaa ttttgctttg aactaaatga agtctcattt aggaagaatg actgtcttta 36600
   aaacagtctt cctatcatat agaaatacat taggaatctc catttcttca agttcttttt 36660
   ataaatctca gttgcatttt ataatcttct ttatatgaat cacaaatcag tctcattggt 36720
   attattttaa aacttttcat atgtagtctt gtaccactgt aaatgagatc tttttcattc 36780
   tcattttgca aatgataatt tttttataga aatgttcttg gctatttttc catcttttta 36840
   tctggcaact ttactcaact ttctgattct gggggttttc ttgttgattc ccttacattt 36900
   tctcaggtcc agtacagtaa tcatgtgcaa taatataaac catgagtcct ttctaacata 36960
   gtagactaat aactgttcat caatgccccc ctcaggaact ttaaagaaat acaagtagtt 37020
   gggccctcct cggtccccac tgaaccataa cttcagtctc agccataggg ctttggcttg 37080
   cggggcaggg atgggaagtg gattccagac ccaaaagtct gcaaaggtgg agaaagcctg 37140
   gcttctctaa aagcatacca gtatagctac agaccagaaa cttcattgct ttgtaggaac 37200
   tttttaaaat tttggtctgt gagcaaggga agtgagtgaa ggatttgaag cagcaggtgc 37260
   aaggatagat ttgcattatg aaaaaatcac tctcactgcc atgtgagaac agactgcagg 37320
   ggggctggag ggaaaggggg tgcagatcat tatgtaacca cagctaactc ttaccgcctg 37380
   ttttaagtgg tttatatata ttaactatta agtccccagg acagcccaag gcatacaggt 37440
   ggttcatggc agagcacatt tgaattgttc ctaagcactc atcacctcga ctctttttca 37500
   cggagagggt gggagggact tcagacccac ttacggagtg catcctgcgc tgccatctgt 37560
   cttctgggtg tgtgactggg tgagggtggg tctgtactca ccgtgccttt ttcatagggg 37620
   taggtctagc ctgagtgttc tgtacactgc tctcccaggg aagtgggagc gggaggtgag 37680
   aaatgtgtat ccttttaaca taaattatca gatggggccc atgatgatgg agctctagtg 37740
   atgttgggtg ccttggacct tatctaatcc aaccccctca tcttatacac tgacacacta 37800
   aggccaagga aattgggaag gcacttcttt atggtcatat aggaggctgg ggacttaggc 37860
   ctaaacttgg gattcctgac atccaagaca ctaatggtgt ttttagatca agtgagaaca 37920
   tgggcctaca acatcagcaa atgtctggtc acctgtaagg agcaagggaa gttttatatg 37980
   agattcatgt ccttcaccaa accgcttcag ttgtctgtca gagccaggag gatggggtgg 38040
   aatacagttc agaataggca ggctgagatg cagatatggg gagccacagc atgacagaga 38100
   gagctggggt gtgtgcggtg cagggccagg aggctggggc caagcagagg atgacctgag 38160
   ctagaggaac acacagtggc ccgaggcccc tcccactggg gaagagagac ctctgaagtc 38220
   catctgaggg ccaacccaac ttgcctgtct ccagggagac cccagaaatc ctcagaagtg 38280
   taagggctgg gacagggcca cctggcatga gatgcccagc tcagaatccc agggcctcgt 38340
   cctgctaagc tgagccccca tcctcagagc tcccacctgt gccttcctca ggatggaggt 38400
   acaggaaggg gccactctgc agcagcggag agggtggtga cagctcagca gacacagaga 38460
   catggagagc aggtgggctg tgatcggacc aggagtcggg agagagacac agtgatgcaa 38520
   aggacgcaga gggggaagga ggcagaggcg ggtactgccc cacagcccca ccacccctgg 38580
   gcgtgatctc acctgaggaa gcctagaagg agtcttcacg cgtcgcctcc tccaggccag 38640
   agctcagctt ggagatgcag agggggaagg gaccctctcc cttccttccc ttcctccctc 38700
   ctagagccta ggcagggcag cagtggtggg accacctgtt ctcaccctca tctgcggtgg 38760
   gcggcggagg ccccatgcca caggcttggt gaggctgagt aaatccttgc tgctgtgacc 38820
   acccacagtg cttctgcttc cttctgtata atagctcctc ggggagctgc cagcaatcaa 38880
   tgagacaata caagcgagca cttagcacag tgctggccct gtgtgagggc tcagtgagtg 38940
   ggagctctaa gtactaaatg ttgtcccatc tccgtggtgc tttggacttc cagcacagca 39000
   ttcacagaca cacgtccctt ctcagttcat cctcacacac tatgtgcagt aggcaaggaa 39060
   tacgttgtct ccaatcaaca gatgaggaaa ctgaggcgag cctggggaaa gtggtctctg 39120
   gtcacccagt gagttagcag cagagaggat aagaattcag ctcccctcag tcttgtgtgt 39180
   tttcacagct gtgtttctta cataaaatac aagaatgcta acacaaaaaa caataaagcg 39240
   agaattctca ccttatttca gagttgagta aactataatc atcttctttt gtctcctaac 39300
   agccctgctt taaagagtca aagtatcaga gaacgtttag tgctaaaagg agccttagag 39360
   acagtcccac tctctttcat gaagcacaga gagaggacat cctcacttaa ggtcacacag 39420
   cacattagca acttagccag gattggaacc tggcactggt gttctttcct ctatagctgc 39480
   tgcctccata ggtcttggaa cttttatccc cacttcagag tctggaaaac tcagctcaca 39540
   gcttgactcc aatcccatag caccaccttt tttgggactc tgcctcctca gtggtggctc 39600
   tagagtctga tatttagaat cagcagcagc aaaagggaag ggccatggag caaaaaggtg 39660
   gtgtgttggg agtaataaat tttcaaagag tttaaattgc atttcaggga ctgattcctc 39720
   ttgttggaga gattggtttg tgaattcaga ataggattgt ttaatatgca tttcacattt 39780
   taaataatca tggttttctt agagcaggga gaaaaacagg tcaaaaacac aaactcatag 39840
   gcttttctga aaggttacaa aataaaatgt tctttgctgg aggatataaa ggaaatggct 39900
   cttgtttgac aaaggagagg gctctgctag ataaattctg catttatgaa taaatgaaag 39960
   ctctctcccg ctcttcctga tcaccttaat tccttgttgc ctatctgcaa atccaataat 40020
   gctggtgaaa caaacccctc ctggaggctg agcacagggg cctgagcgct cagcctcaga 40080
   accactcttt ctatttataa tctcagcctg tgctccccag agagctgggc ttagatgccc 40140
   cctgggcaag tgggcaagag gacttgctgg gaacagaacc ttccccgggt cagagaccct 40200
   gtactccagc cctgggtctc cctgcctagg aagcactcag caaatgcctc agaataggta 40260
   aagagcaggt aaagagcaga accgtagttc agcatcatcc ttcctgcact tcctctgctc 40320
   ccttccaaga taaataacca ggctaagaat tgcaggtgtt tcttgcagcc agttggctat 40380
   tgtcttagtg acacaccata ttacactagg caactgagga tatgtcaact cgtaaggcgt 40440
   tactatatat atgggttttt gtggtttttt tttttaccaa atctattttg taccaatagt 40500
   gtgtgtttat cgtctgtctt ctcttaacat tgggggtggg gatgttgtct gcactgttca 40560
   gggatcacta ggaggtctta ggggccaaag ctggagctaa gaggaggttc aaacacctgg 40620
   aagaaggggc cacctggaat ggaggatgtg gcccctaggg tggcaaagct gagacactgg 40680
   actgtgtctc tcactctagg catctttgta tgaaaccaca tatgtgtcag ctggtggggc 40740
   ttttttatag agcattatac tcacctaaaa tattgtattc catctgataa atccattact 40800
   ttgtcattaa aaattctcta taaggctggg tgcgatggct cacacctgta atcctaatgc 40860
   tgtgggaggc tgaggcagga gggttgtttg actgaggagt ccaagaccag cctgggagac 40920
   atagcaatac tcaatctcta caaaagattt tttaaaatta gccaggtgtg gtggttcaca 40980
   cctgtagtcc cagctactct ggaggctgag gcaggaggat cacttgagcc caggagttca 41040
   aggctgcagt gagctacaat agcgccactt atactccagc ctgggtgata gagtgagacc 41100
   tcgtgtcttt tttttttttt tttttttttt ttgagacgga gtctcgctct gtcaccgaga 41160
   ctggagtgca gtggcacaat cttggctcac tacaacctcc gcctcccggg ttcaagtgat 41220
   tctcctgcct cagcctccca agtagctggg attacaggtg cccaccacca cgcccagcta 41280
   atttttgtaa ttttagtaga gacgggtttg ccatgttgcc caggctgctc ttgaactcct 41340
   ggcctcatgt gatccaccca cctcagcctc ccaaagtgct ggggttacag gcaggagcca 41400
   ctgtgccagg ccaaccctgt ctcataaaaa caaaaattcc ccataaagga tgcttagtga 41460
   tccatcctat gaacactgaa ttatttactt aaaggagtcc taattaatag acattttgtt 41520
   tatcttcccc cagccaattt cccctattag caataaagtt tccatcagta tctttgtaca 41580
   taaacttttg acttcgtttt caatttcctt tgactagttt tctagaatag gaattaatga 41640
   gtcaaagggc taaggcgccc atcatctggt tttctctgga ggaaggtagt tctctgggct 41700
   ctacaatgct catctgcctg aatctgaatt tgaactctat cacttcttat cagtgtgact 41760
   ttcagcagat tatatataac cttttagttg cttcatttgt aaaatgggga ttataatagt 41820
   acctaccttg aagtgttgct ctgaggatta aatgggagaa gtgatgtgta attgttaagt 41880
   tgaataggca taggcatgac tcatgagatg taatcataaa gcaaacttta tttttgtctc 41940
   ccacagtctc tttaaattta ttctataggg ctgttttccc ttctaaaatt atattaaatt 42000
   tagggtaagt cccatgactt ttctgaccat tttcaggtaa ccagaggttg tcttgtcagt 42060
   aattaatgca tcctgtctgc tgtaataaaa aaaaaaatct tgaaacgtca gagttacacc 42120
   atctctttcc cagcacggtc tgctgtaggc cagctgcctg tggggacctt gcacatgaca 42180
   gtcctgaccc tttccagggt ccaaggtgga ggggcaggta ggtaggagag gagatggttg 42240
   ggaaaggtct tcaacgactg tactctgatg agttgctgtc ccgttgttct ctgggccctt 42300
   tctctcactg ggctgttttt gtgggttggt ggagaccacc ccatcgctgg agtctcaccc 42360
   tgcacctccc ttgatgaggg tgatactttc tccccgtagc cgatcctatg gtttctttct 42420
   caggctctgg gaaacctggt ctccctcagt gtcacctcct cccttggagg agccctcttg 42480
   tcctggatct aagacagtca catctggctc tcttgctggg tgctacctct gtccacaaga 42540
   gacactcatg tgtgctttgc cctgcaaacc agggagcaca ggctggtcct gaagcagcag 42600
   cactcttgct gctctttgga tttctccact gtggcaccaa gaccattttg gccacatatc 42660
   aactgactta ggggtctcct caaggcccca ctgtctggct ttgaggtgaa ggtgatagca 42720
   ccctgtaccc ctcccattta tttgggatca ctggacccca aaggctgtct ctgaaattca 42780
   ttctaaacct ccaacaacct tttttcaacc tctctcccat tttgtatgca ttagagatgg 42840
   gtgtcagcta atggcatgga cccaggtttt cgccacctct tttccaagag gtgtatccca 42900
   cttcactttg gaatgtggta tctgttcatt tggagcccca agtggatcct agactggaag 42960
   catcccatct catcctccca ttgccatatt tgtctttatg aaacacacag aggagcacct 43020
   ggcatagagc atgcactcaa caaatgatag ctgtgtatga tccaggctca cggactctct 43080
   agttacattc acctcataac cattcgatct ggttttattc ctattacttt gttttatgcc 43140
   ctatgcattt tatgtttcct gtagattatg cttttgtgtg tttgtgtgtg tggaaatgat 43200
   ttgctattat tttcagcagt gatttggaaa atagacattg cttttaattt tactagtggt 43260
   tgacctacat tttttttcag acattgtaag cctatttatc tcaatcatca acagtggtgc 43320
   aatggtcttt gtatattctg cagttattta aatgggaatt acagagggct ttatctggcc 43380
   acccatagtt atgctaccat cttgccatga tgtctaccca cagcttcaaa atggcacttg 43440
   actctgtttt agggctggct catctttgtc ctggacttta ctcatagctg tctagcagtt 43500
   tgccatacca ctagcctttc tcccgcaatc ctccattctg ctcccaatta tttctttcta 43560
   aaggttacat ctgggcagat tattcttggc ttaaaaccct ctagtggtcc cccactggga 43620
   accacatcct tagaacagag tctataggat atttccaaat tataggataa ttgcaaacgc 43680
   cctagcttga tgttcaggat gacatatgac ctggccctgc cttaactttc taggcccatt 43740
   tcctgccaca ccgaaccatt cacctattgg ttgaatgctg ccatgctggt ttgcaccttg 43800
   gttttcaagt gcattgggca tgtactctca tgtctgcgta actttgctca tgtcatcctc 43860
   tgtctgtatg gccctttggt aaaagaccct agcacagtgc ccggtcaatt gtagaatctc 43920
   agtaactgtt acgtttccct cttcctttgt tatcagctta tcaatatcct attcatcttt 43980
   cagggcccaa cacaactatc atccttttcc actcttggga aggcttgtcc taaccttatc 44040
   tccccccttc tagggacttc cattgactgc ctcttttctg tgtccccttg cccccagcac 44100
   attgtacact tgtgttgtaa caccaacacc ttaatgtagg gtacagtcat tatttcttcc 44160
   tatctccctt gccagaccga aagctccctg agagcagcca tgcacctgac ccccactaag 44220
   gccactgcag ccctgcatca gcctttgagg ggctagttga tgtttgattg atttgaattg 44280
   ccttcctcag actgcaaggt cacaatgcca agccaacaga cagggcccaa tttgaatgga 44340
   tatcatttat cattcatatc cctttgcttc caaaattgac tagagatggc ccacactcaa 44400
   taaaaatgtc accttaagta tagcacatat aagactccat ataggtggct aagtcccaaa 44460
   atgctaacct tcttccctgg gtgaactgat tctgcttggg agagggggtg gccctggggt 44520
   cctcaagctc aggtggcaga tgtgagggtc tctgttgggc tgcttcactc cccatgcatt 44580
   ccttcactcc ccctcacaaa catttcatca ccacgtttga aaatgcctaa aatgcaaaca 44640
   ggatgattta aattcatagc aaatatctta cccttgcagg atttgggcag gtggtgaggg 44700
   aggcatgttg ggattgctgt gtggtggtgt ttcagtccag ataaccttgt tcctggggct 44760
   aaatttgaaa agcttatgcc cttcttcgag ggtcatggtt atagtgcagt tgtaaaaata 44820
   taacttttga tcatggccta gtataatata catcatatga ctttctggag tagctttttg 44880
   atggaagcca aggacgtaaa cttaaaatgt caaccacgga agtcatttga gtggaaaaag 44940
   ccatcatgca ttctgagtag aatgttaaca ctcatgcact ccatagaact cgaagaagca 45000
   cacacgtcat tctcctgcac gcgtaccatc caacaaatgt tcttgtgcag tcctgttcag 45060
   actgcagggc catctctcac acacatacac acattgctgc gtacactgaa acagtcctgc 45120
   tgcccccaac ttcccatgca cacacacact caaggcatac actggcacac agtcccttgt 45180
   gtgccagtgt atgggactca cacaacccat gtgcatacac acatgtacac acatacacac 45240
   tcatattcat gcgtacactg gcgctcagtc ccactggctc ccccacatgt acacacactc 45300
   acactcatgc atacactggc gctcagtccc atggttcccc cacatcccat gtacctatgt 45360
   aacttctgca gaaagagact tggaaagcca tcttacttct gcacctcact ctgcctccag 45420
   attttgctcc cttaggacag agcctaaact gtctatttaa tagattcaat tactccaggc 45480
   tcctgagacc ttccaactaa attctgattg ctttaaggtg tcaggtaatt tgggaataaa 45540
   gggaggaaaa cagcttcaaa ttcttgaagg gaaagtcttt tagactctag ctgtctctga 45600
   catggtagct actaattgca actcaggaag gggtggcctg gagggcgcgg cctccagggg 45660
   tcaaagcttc tgatctctag gcaaggttct gccctgaagg acagaggcct ctggggcaat 45720
   gctttgctgc tctgctgcgg tctccagggt agggaccaat ctctgctgcc cttcctggaa 45780
   ggccagcaga gtccaggcca agcatcccag caggcaacaa aacagccctg ctttctgcct 45840
   tctgcacccc actcccaggt gttttatttt gtctgctctc tgtccccagt gacagtgaca 45900
   gctcttggag gttgataaat aatcagaggt catagtgttc agagagcatc aatgttgtgc 45960
   ccgacagaga tggatctgac ctgtcctgag ctccagaagc ctggagagag aggccatgtg 46020
   tgtgtgcatg catgtgtgta ggtgtgtgta ggtgtgcgtg tgtgtgtgca tgtgtgtagg 46080
   ggtgtgtgtg tgtgcactac atgaactgtg attggccaaa aggaggctga gggtaagtgc 46140
   tgtagaccca gaccttttgt gtgtgaatgc tagcttcatc attgaccagc tgtgtgactt 46200
   tgggcaagat ccttgccttc tctgtgactc agttcctctg ctgtgaaatg ggtatgctaa 46260
   tggtgcctac cttctagggt ggctatgagg attaaacgaa ttataatatg tacgtagcac 46320
   ctaacacagc tcctgacgcc cagcaaataa tccaaatatt gtgggttatt gtgttttata 46380
   attaatgggc aagagaaggc aaggctgaag ggctgggaga gccctggggt gggggtctgg 46440
   ctgtagcccc agggcagcag gatgagcccc ctccctcagc ccccttggct gttctcacac 46500
   tctctgcggg gcagtcctga ccgcctattt atcatcagaa gatccccgca caacactctt 46560
   ctggcgcggt tggcgcggca gaatccagga tgcacacgag gacgcccacg gggcgtgtgg 46620
   ggaccgtgcc cctccgcaga cctctcccgg atcacctgct ccacacgcag gccttggtga 46680
   cccctcagcc tgctgctcct tgcttccgcc cgccctgctg tgggtcccgc accttctcag 46740
   tgtgccatgc cttgcattca agctggtcct agtcacagcc aggacctcct cagcgcgcag 46800
   agactttcag atgatctttg taggggtttt tgcctccggt caactgcgcc ccttaggaag 46860
   ggctttggag gacacagatc acagaacagt ttcagctcca ccacttccta gctttacggc 46920
   cttgggcatc tcagcattcc tattctctgg gttataggga tatgatgttt gtctctcaag 46980
   gctactgtgg agattacata ctagaaattt gcaagggctg ggaatgcaat agaccgtaaa 47040
   tgttcccctt cccttgacgc tatagcttag atgggaggga gccactgctg aggaaacacc 47100
   tccttaaccc aaagacagag ggttccccag gcgggggtgt gggaactcgg aggtgggggt 47160
   gagggaaggc caacctccac gcttcggccc ggaggttccc ccgcctcttc ctgcacgact 47220
   ggattccttc tccatccgcg cctccggccc ggtccctgcc ccgccaaatc cgcgcatcgg 47280
   tttcccatcc catagcgcag ataggtaggg caggtacaca gggaggtgtt cgaatgatcc 47340
   ccgtttcaca gaagacgaaa ctgaggctgg gaggcctggg gactggcccg gctgcagcgc 47400
   cgccgttagc cgaaggtggg agccggcgcg gtcacgcgca tggagcgtgg atcgcgacga 47460
   tttcggggga gggggcaagg gcgctccgca ggccctgagc cggccggggg gtggggtctc 47520
   tgcggggcgg ggcggggcgg ggcgagggag aaggggcagg ccgccgctgc cacgcagtac 47580
   tcccgccttg gcgccagagg ggcgcgggac ggtgcggtcg gcctccccgc ctccacgcgc 47640
   gcgcacgacc cgcgcactcc cggagcttcg cccatctcgc ctcgccgcgc cgcgcaggca 47700
   gccgctcgca gagccagcag ccggggccct ggcgtgcagc gcgggcctcg gcggggccca 47760
   gcgccccggc ccgggaggat gcggcccggg gcggcccggg agctgagcag ggcccccgcg 47820
   ccggcccctc cgggccccgg cctccagagc cgcagccacc gccccgcccc cgagagacat 47880
   gacttccaag ccgcattccg actggattcc ctacaggtac gcgggcgccg ggccggggcg 47940
   cccaccactc ccgactcggg acgtgagctg gctggggata cgcggccggg gcgcagggca 48000
   ccgctgccct ccccacctat cccagcactg gggcgcgggt tggagtcgct ggtccttcct 48060
   ccctcaactt tgagggcccc tgcggactgc tcccgcctgc cccgctccac ccccttatct 48120
   gccagggttg ctggaagggg cctgtgtggg ccttgttggg tcaggcagca ccatgtggcc 48180
   gcctggggaa ggagtccaca ctgctctgag ccccctccta ggcccagaac cccagtcaca 48240
   cgcctgtgca gggtgcgcgc acacgctcat acatctgcac gcatgcaccc acaggacgtc 48300
   cctgccttgg gccaaagacg ctcctgctgg tcggttcaca ctctcaaagc atggatccgt 48360
   gaagacagtt attctgaacc aagagcacac cagtggtcat gtggacatgc acccgtatat 48420
   tctacacaca agaattcttg ggctcacaaa gacacatgcc tgcatatctg tgcacgtcca 48480
   cctcctgtat ctggagaata actgacacct gcttctcact cacaagtgct actcagtctc 48540
   tttcagacac tgaataatct acttgctagc aggtctatac ctagacacac accagctcac 48600
   agtacataga actgccctca catgcagaaa ggcactcaag aatccaggca cactcaggga 48660
   agccccccac cctctgctgc caggcactgt ggtctatctc aaggatggct gggctttccc 48720
   atttgcctgg gaagggggcg gggaggtggt ctctgggggg cccctcacag gtgcaggtct 48780
   ttccctatcc actctactcc attctattac cagggaaggg agctcatcgc catggcagcg 48840
   ccagattctt ggccacccat tggctccttg gttgccatgg gctacggggc tgtctctgtg 48900
   gaaaatgggt gctggactcc taaggcccac tgtgttattg tcgacccagg tgcacgtgct 48960
   agcggacggc aggatgagat cctcaggtcc cgccttgtaa cacagcttgc tctctgttag 49020
   agcctcatac cagtcactga tagaaaatag ttctgacagg gctcttctct gagtctgtcc 49080
   agacagcgtc ccaaatggaa accagctgaa accgcccaag gcttcttaaa agcagatcct 49140
   tctgaaaaca ggcacgtggc ctgggaactc agggtttctc ttgagaattg ttacctctaa 49200
   tctcagctcc tgtgggggat tcaggggttt ccaggttatt ttgtgtctct ccccacaacc 49260
   accagcaaca ccctcacacg tgcgcacata caggtcaccc acaggctctc ctgcagacac 49320
   atgtagtcac acttcagtct cacatggatt agggagctgt ttccatcatg gaaccaggga 49380
   ctggggctgt gctgactgag aagagctgct gcgccagaca gacgtccagg ctggggcaca 49440
   ggtagtttct ggaggatcct ggcctttctt ggcacatgtg ggctgaggac cttctagaag 49500
   acagtgcagc cccatggagc gtcatcaacc ttttctagcc acgcggcaga ggtgcctggg 49560
   cggtgcttgt gggctgagtg gactccggtg ctgtggaagt acctgtgtgc gggggtccag 49620
   atggcagggt ggtggccatg gaccgaggag ggtgggaggg gcagcactta cggctgtgga 49680
   gattatgacg tggttaggat ctggcaaact ggaggttggg ccaggccaag acatggttct 49740
   agaaagcttc tcccagggag ccagggacta aagccacttg tagagagtgt gcaggggtct 49800
   tagagaaaat atgcctcaaa cggaatggct taagcctgtt cctgggaaag gtggcccagg 49860
   aaggtagaac tgtctctagg aaatgatcct gttctagcaa gtgcctaggg ccctggcatc 49920
   ctgcaaggag tgatttggca cttgcctcag cccaggtcca actactatct cccctccacc 49980
   gccacccctc caattccttt cctgggccgc agactcagcc ctgaagcata gaatcctgcc 50040
   tccctggaag cctggccccg aggagcgtct caacaccctt atagccctgc actgcttgct 50100
   ttccccatgc ctgtcactcc acctcctcgt tctgttgtag agaggggggt gtgtagctgg 50160
   aggcaggcta tgtcagcagt tgccaggatc ccccctgccc tgctggggtc tttctgggca 50220
   cgcactgagt gtggcaaagt actggaactg gggtgcagac ttccaaaatg ggagacttgg 50280
   gctgagagga ggagattcaa attgcctctt gcattaactg ggctgctgat tgcagtcaaa 50340
   gccacatgct gtctgatcac agagaaaact ggggcagaag acagacaaag cattttactt 50400
   gcagatggga gtgggggcag ggggtggcat gcacgtgctt tgactggtgt gaggtttccc 50460
   taatgtcaca gcatgtcaga gctggaatgt ggcatttggt ttgggagaga ggggttggct 50520
   ggcttcttgc tggttctgct ggtgagggtt ttactccctc tctaatggtt acaccttgca 50580
   ttccctgggc ccagcttatt ttctttagag aaatgatagt tggtgataat cttcttatgg 50640
   gtaattttgg ctcagtgtat aatgatcatg gattaatctc acattctgag ttcaaaggga 50700
   gccattgagt ttataaagtt taacaattgg aatgtttttg tcttttgttt ctgttagtac 50760
   taataatttc taattctgcc agtttctctt tacctgtgtt cttgaggaat attaaatgcc 50820
   cttagtacat ttacagcctt ttataagttg taagtggagc agtgagagtc aggaaggact 50880
   gcggcccacg gcgtattgga ggactatgct gggtgggatt ggggtggccc cgtcctcagt 50940
   gcccttgtcc tcatggtcct gaggccattg cctggtgttt ctctcacttg gtcatgaact 51000
   aattgagatc agggaactta tctggttcct ggtcatgtcc ccagcacaga caagacgtcc 51060
   acttctttga atcaaagtgg catcccggga tagcctctag ggcttccttg gctttcccag 51120
   ccccacacct gggttctaag cacacctgag ggatacagta ctctcacatc accatgggac 51180
   ctcctggagc actgcttgaa aagcattctg taggtcacag aaagagaatc tcagactgga 51240
   agagccttta caggtcatct agttctccgt gtggatgctc cctgtagcat ccctgccaca 51300
   cggcctccca tcagatggct gtgatttctt tcaagttctt ccttaggttg aacctaagca 51360
   agtctccggg ttccccctcc tcctggccct tgttaggatc ctcccaccac ttcctttgtt 51420
   ttgtagggag tagtgggact cctgacgcct tccgttctgc tgctaggttc ggcctccgag 51480
   gtgccctggg cacttggacc caccacttta cttctggcag ccttataacg actccctgat 51540
   tttcttgttg ttctttagag atcaccagct gtggctcgtg gtcgcatttg catggctgag 51600
   cattagaatc acccatggca cttgctttta caaagagaga gaggagtggg ttgattctgg 51660
   gggatgttcc ccagcagttc tgaatgacca agtcagggct tagtcccgcc agcagtttga 51720
   atgcacctga tacatttggg aactgctggc tggaatgggg acggcacatc ctgggaggct 51780
   ggaactcctg tgagcagtgt ggtcctctgc cttctggagc tgcactggtt cccactgtgt 51840
   cccagtaagc tgccaggcga cagaagccac agatctggac ttgccggggg tccagggttg 51900
   cagccagggg cacagggttg cagccagatg ccctggtggc ctttcctctc ctgacagccc 51960
   acatctgtga tttcctgggt ccagcagggg tagcttccac tgtgactgag gggaaagaat 52020
   gagggcagga agaggagaag gtgaagtcaa catggaaagt gtggccagag ggttgggtct 52080
   gtccctagat tccctgaccc ctgggcctct ggttagcact gatggagtag ctggagcatg 52140
   taaggtagag tctctgaaga gagggagttc ggaaacaggc tagaggatgt tggctgtgtg 52200
   tgcttttcca gggcacagaa ttgtccttgt gtccttgccc cttgttgtca atcgtggatg 52260
   actgctctga tatctgtggg gtgccttggg ggaagtgagt ggatgtagag gggcaggttc 52320
   cctgggaggt gaagctgcca cctcgttggt gctggactga gcagatggat tggtggacac 52380
   tgaagcctgg gtttctctgc cctcatgagc cagtggcttt cctgagcctc cctgttcagg 52440
   cccccagcag gaggtgcccg tgatgtagga gactccaggg tcagctgaaa gacctatcca 52500
   tcagcgccct gtctcttgtc tgagtctcct ggcggggctg cagctggcag cctgggcttg 52560
   gcagttattc cataattgga gctgaagtca ttaaaaaacc attactgtct ccttgtgtgg 52620
   ttatcgttta ggtcagccta gaacagggcc catcagaaac aggcaggctg gggaagcgtc 52680
   ggtgagaaag gaagatgcct taggaatggt gctggggtgg agatgaggag gaagggccat 52740
   ctgggtgggg gcggagaagg gaactgggcc tattgaggaa gccaggggag cttttggtgc 52800
   tgctcttcct ggagacagcc atcgcagttg cccttggtct ccacaacagc tgctgttggc 52860
   tggggattct ttgttattca ccagggagag ttgttgcgca cttgtcgtta gctaggaaca 52920
   cccacctggt acaagcttgt cactgctagg taatcgtggt cccatctcgt ccccttgcag 52980
   ctcttctaga acactgcttt accaggaatc gtgcccactc tcagagcctc cccattttag 53040
   tagatttagt gtagaccctc ctagctgggt cttgctcagc caggcacagg tggcttctcc 53100
   ccggtggccg ccccccagct gtgcatgctc ctttccttct gtgtgcttgc acatgcccgc 53160
   tggtgggtga ggtggtaggt ggctctctag gtactttggg gatgtctgag gtttctggag 53220
   cctcttcagt agcctagccc ttgtgttcat ccatatttct ctgaaggcca gtggttaagt 53280
   cctttctccc tgttatctta cctagtaatc agggtcaccg aagcctgtgg ctctttatca 53340
   ttctccccca tcaggaaatg gaggtggagg cagaggagga gaaaggaatg ttttcatgga 53400
   aagcagcctc ctgcctctgg cataactgtg catagtccat ggcaagtggg atggtggcct 53460
   ctggtaggga aatgtgcccc ctgcatggat gagagcatta aaaggcactg ggcagtgagg 53520
   ccactatggc tggaaatacc tttagtcagt tgagcagctg ctgtgaccca gcatttccgg 53580
   aggtggcatt gctgatgtga gtcttacctc tcagctggat gtcccctttt ctaaatgact 53640
   ctagatgtga gcgtcagcac catggaagtc atcttctttg tcttgctcag ccaagcccag 53700
   ggctggggat ggcccaggtg actgcccagt ccctgccctc tgaaagtgtg agtggcttgc 53760
   gacaacctag aaaactgtgg aatggcccct cctgctggca caaagggaag ccagcactgt 53820
   cttgtggagg ttgcacaggg gccaggccac tggcttatga cagtggggtt tatagctgcc 53880
   ctgtgcccag gcacagagct tggcaagcgg agggagcaca gttggattcc agaccctgtt 53940
   gctcctgtca gccaggggcc actgtacaga gcttagactg catttttcac gtgtgtcctg 54000
   aaagttgtga tgtgtatgca ctgtgcgtgt ggctgaccac gggggacact ggtgttcggg 54060
   tctgaccctt tcaagtccgt ctgtgggcaa atgctttgac tttgcttatt cagtcaggct 54120
   gttggcttga gctgtagaga tgggagggtt gtagcctctc aggcctggca gggccctcag 54180
   gcattttgcg ctctggtcct gaaccctttc ttcagaagtc ttcactgcag tcctccagcc 54240
   tgggtgtgtg ccttccacaa tgggatgccc agcctctcag ccagctggaa tatttaggga 54300
   cctggttgca tcctgtggtc ctggcaatgc cctcagggag ccccaaatgc agctgtgccc 54360
   tcttaggcag atgggctgtt tcaagaagac caaggcctag aagtatggct cttgagcggt 54420
   gttttctcct gtgctctggc agctgagggt ttggtgaatg aggctgttgt gcacacagcc 54480
   ccagatcaca gctggggctc ctctgacatg cagagcagct cgtgggtaca aggaaagacc 54540
   ttcccagatg tcaagtaggc aggacacctg ctagttaaaa aacatgcata gtcctcatag 54600
   atcgtggcca aggtggtggg cagtctgtgt tatgggggct tccttctacc ctctgactgg 54660
   ctcccttgga ggtgcaagta gttgaaggct catgtaggtg gtcaatgatt gttccagcta 54720
   gaaagggcta gccatcagga ggaggtgaaa gtggcttcac tttgtcctca gggtgatgtg 54780
   acctcctgtt tcccctttgc acgcacactg cgctcacctt cacacgagca cacccacttc 54840
   ttcacaggtg tctgcatgca catgtgcaca cacatgagat attcacacac ttccttatgg 54900
   cctcacacaa acctaatgca ctcgggcacg cctgtgcagg aacctggaca cagtctggtt 54960
   caggggcagc ttcagaataa ctgggccagg gccgcctggg gcttccccag agctcagatc 55020
   ctcaggctcc aacctcacct catgcagctg tccacacctt ggcctttata gcatttgggc 55080
   ctaggtctca tgcatgtgtc tctggctagc aaaaagctaa gtgatttgag ttaccagttc 55140
   cctacttgtg ccctgccttc ctcagaggca tggccagtgc gggtgagatg aggctccctg 55200
   tcccaagact ggtggggctc aagaatttga tgtccccagg gctgcctgtg ccatgtctgc 55260
   agcccgggaa ggacctggta gctggttgct gggctgtaca ttgatgtgtt gtgtccaccc 55320
   tgcagacacc tctgtgtggc ccaaaccctg taggctgagg gactggttgg tctcttagct 55380
   tcattctctc aaatgagaag gaggaccctc tgccctcttt aagtggggaa ggggagtaga 55440
   caaagtcagg actctactct gcccacctgg aaagctagga gtttgctttc aacggatggg 55500
   gaaagggtct gccccagagg acgcagcctc ctctagaatg gtgggaagga aggaaaaggc 55560
   tggggctggt gagatcccag tagggagctg gagggagcag ccccctgggt ttgtttgaag 55620
   cctggcctcg ggcctggctt gttgctggga tgcctctgga tagttctctt ggatctcagt 55680
   caggcccggc cctgctcagg gatctgaggg gagggtctca tccggtattt actggggagt 55740
   ccctggggaa acaggggtcc tagcaggctt ccaaagtggc agctcgagcc cttttggttc 55800
   aagctggttt caagatggca ggctccaccc ttcctccttc taccatgtgg gctcactgag 55860
   tcccagcagc cgtgatggga tgggtttaac gggcccagat atgctggggg tgggctctgg 55920
   gctgtatatc ctggcacctc acgggcaagc cctgaaaacc cctctttcgc tctgcagtgt 55980
   cttagatgat gagggcagaa acctgaggca gcagaagctt gatcggcagg tgagtaggcc 56040
   tggggccggg tagaagggaa gagtctgggc tgggatctct gagggcagag gggcagggct 56100
   gtccatcttc cctggatgat cccgtctgat tgggggatgg gattcccatg tggaagaaga 56160
   gaggtgcact ctctcccagc tcagcactaa ccctgccgcg gcacatgggc tcctgccttc 56220
   aggcagctgg gtgactgcag ttggagggct ggctgtgctg tgatcctgcc agggcagcca 56280
   cctgggccct gttttgcctc cctttcctgg accccaagtg ttggggtgcc aaggacatgg 56340
   ggccttggcc caaggtgggc tgtggacccc ccgataactg ggagcccgcc cttcctggtg 56400
   gaggcagtgg gtcagccaac ctctgtgcct ccatagcggg ccctgctgga gcagaagcag 56460
   aagaagaagc gccaggagcc cctgatggtg caggccaatg cagatgggcg gccccggagc 56520
   cggcgggccc ggcagtcaga ggaacaagcc cccctggtgg agtcctacct cagcagcagt 56580
   ggcagcacca gctaccaagg tataccttgc ctgctgcccc acatcccgtc actgcttcgg 56640
   ggagcccgtc acttcctgcc cacctaggca ggtgcggact ggtcctgacg ggggatgccc 56700
   aggctgtgta tggcttccag cccaggcaga cagctgagta gggagggcag agggaaggtg 56760
   gacctgtagc cttgggcgcc tgtgtatgct ccagctgaca cctcctgaat caggcactga 56820
   ggccagaagc ctaaaccttt tatgaggcca ggagttcaga agtccaagtt ttgatctgga 56880
   gcatttcact gagtgcagga ggtagcttct aagagtagat gacctgctcc ccaccagtat 56940
   tctacattct gagaggaatc cgtctcactg tcctgcccca tatcaaccag tgtctcagta 57000
   caaacacctt tttccttccc tctctgcacc gtgtctgcct ggcttctgac ttgcagatcc 57060
   ctctctctgg ctcacaactc cttaagagct gtagtgacct ggagcctgca gctcccatcc 57120
   ttaaatgttc caggtctcgg cagagcccag ttgggagaaa ttctctgcag ctcatcctgg 57180
   agagagcaca tctccagaga tggctgcagc acatctctgt tctcaggacc ctccttcctg 57240
   cttgggttag gtgaagagta ggccaggcca gactgagggg ttgggggtct ctgagcttga 57300
   aaggaggact cagactcagt ctccactgcc ccctgctggt gctagtaatc caggctgcta 57360
   cttcatcaaa tagtaattct aaaaaggttg caggcatctg gcttttgcgg ttaggaagaa 57420
   ccttagcagt cgtggtgctc ccctccttct gtggcttcgt tatgacctgg agcctccaga 57480
   tacatcgaac tactggtagt tgcctcacct cagggctgag cttcttcatc taactgctgg 57540
   cccataccct gcctcgcagt attttaggtt cttctctggg ttccactgac ccatagctcc 57600
   ctgagccctt tgtctgcaca ttggagggca gcaggtgggt ctcacttgcc tggtgcccag 57660
   tttgtggctg ggcacaccat acaggctcgg tcccctgtgt gcatgtgtac cctccgagga 57720
   agcctactcc gtttcttcta acaaaggctg gctgtacatc tgccagtggc taagtacagt 57780
   gctagaggct ttacctgcat tatcttgttt catcctcaca ggaactcatt atcctcacat 57840
   catcagtaag gatgtgggct ctgaggggct cagtgacctg ctcatagtac tctgctagtg 57900
   agcagcaggg ctagaatttg aacccagacc taacagacgc caaatggtct ctcctccata 57960
   ttatgttgcc tccctgatga aaagctcctt catgctaatt cccctgtgtg tgtgtcagtt 58020
   cagcaccctg gccatacctg gtatgcctgt actctacttg agaggccttc agaaaagggg 58080
   accagggacc tcagcccagc tgtctcacag aggcagcccc agctgctcct cacagagctg 58140
   gccccagccc caccctttgc tggcagccag ctcctccact tcctttgtga gtgccatccc 58200
   aagggtggta gtggtaccct gccaggaagg tggccaatgt tctgggtcat atggcgtgtg 58260
   gcctgtggct gtgtggcatc acagccccag ctctgccaag cctggcacct gggcgagtga 58320
   ggctgcatac agaatagggg cctgcaccca ggattgtggg ccctgccaat ttgtgccaga 58380
   ggccaacatc gtgagaggtc attgacgtgc agtcgttttg gaaggtattt cttggcagct 58440
   gtgttccaga tgatgtcagg acatcagaaa gactgtggta caactagcgc ctctggcgta 58500
   agtgcaggac tgaagtcaaa agacagtttc tgtgctaggt ggctcacacc tgtaatccca 58560
   acactttggg aggccaaggc aggaggattg cttgagggcg gaagttcagg actagcctgg 58620
   gtaacatagt gagaccccat ctttaaaaaa agtgagcagg gtgtggtgac acctgcctgt 58680
   agtcccagct acgtaggagg atcacttgag cccaggagtt caagactgca gtgagctatg 58740
   atcgtgccac tgcaatccag caagaaaaaa aaaagaaaaa gatagtttct ggtcgtgcca 58800
   ctaccagtta gttacctgct cgcttgtgac tggggcaagc catttaccct ctctggatct 58860
   gtttcctcca ttataaaatg gggaaacttc tctgtcttag ggctttggaa ctcgaaggag 58920
   ccacggtgtg ttccaaggtc caaaatcagg gaattgctat tccctgacat tccagagtgg 58980
   gctgggcctc cacgaccttt ttcacagagg acatttgaac atgtgacatt tgatttctgg 59040
   gaggtgccta ctgcttctct aggggcccag tctttgtcca ggttcaaatg gtggacaaag 59100
   ctctggggag gctgctgcac acacgcgtgg cctacccagg aactggtgtt tccaggccac 59160
   tcccaccccg cctacagcat tagttgcctc cctgtctgcc ctctactggc tacaggcgtt 59220
   aaattcaggg gatcattcca agaggaaaag gagggagtct gatacacatc agacctcaca 59280
   gtttatcctg gtttttgacc tgctggctat actcattcat gttacacatt cattcagttc 59340
   ttaaatattt attgaggttt gtgtcaggct ctggattaga ggctgtaggt cccaagtgag 59400
   cacaaatggg tgtggtccct gctttcacgg accttacagt ctaggtggag agacaatgtt 59460
   aaacagatgg tcacggggtt gctttgtgga aggaagccaa attctgtgag tagatgacaa 59520
   cacctgctct gacgagggag gcaggcacag cgtccctcgg gaggtgacat ccaagctgaa 59580
   atctgcagga tgagtgggag ttgatcactg aggggggcga gggggagggc agtgcaggca 59640
   gcaggagcaa gggccctgtg gcaagaggga gtgcagtgca tgtgaggcaa cagaccagcc 59700
   tggtgtggct gcaactcttg gtgccccaga ggtggggagt atgtgtttat tttagaccta 59760
   ggggaaggga gagaatgttg gtcttcagct taagagcaat cagaaaccat tcaaggattt 59820
   tgagcagatt ggggttctgc agtatggaga atttggaggc caccagctgg gacccttctg 59880
   agacctcact gttggtgcct gtagcttctt gtgtcccaga gttgttgttc tgagcactcc 59940
   tacaccccag cctcccatgg gctcatcccc gcagagcccc ctcgtaggct ccctggagtg 60000
   cttgccctgg ttgtggtctc ttccagaaga tgcagccttt gttctttctt ccccgtcttc 60060
   aatggccata tcctctcaga cattctccct gcccctgttc aggctcagta tggtttggtg 60120
   gcccagaact tttgagtgcg cactttgttg gctgatcaca ggttttggtc tggctgttga 60180
   cttgattaca ctgggctggg ggtgccgcct ccttccagct gggcctgccc tggaatcttt 60240
   gcctagtaga gatgagtggg cctgatcctg tgggctgtag aagtggtaca ggggccctgg 60300
   tgggactcgg ggtgcagtca aaaatgctgt gctggggact gtgttcaggt gggagctctg 60360
   gtctcaccca ctgcctgttt ctctctctcc atctggggat gtttcctgag cagttcaaga 60420
   ggccgactca ctcgccagtg tgcagctggg agccacgcgc ccaacagcac cagcttcagc 60480
   caagagaacc aaggcggcag ctacagcagg gggccagggt ggcgccgcta ggaaggagaa 60540
   gaagggaaag cacaaaggtc agctcacatt ctctacagcc ctccccagca ggcctggcct 60600
   ccactgtagg gctggggaag gtttgtcctc ctgacttgga ggggagggat aggatgaaca    60660
   gcctcaggga agacacagac tgccactctg ggcaccccct caggtggctc acaggcctca    60720
   tctagcttgg gaggtgcctg ggctgcctct gggtgtgggc atgcctacca cactgccagg 60780
   aagtgaagtc ctgctcagct gtggcccaga accaccgtcc cccacctcag ctaccttggc 60840
   ctgagcaccc tttatcatga cccctcaagg aggatctcac ccagcctgga ctaggacact 60900
   cactgcactg ggggctgact gcttcccagg cagcttgccc catccttatg cagccctcgc 60960
   cgtcagctct tcgtataatg tggacttccc ttgactccat attctagtcc aggttccacc 61020
   ctctggacca cagaccagcc tgttccctct gctcctcttt ctccagaaaa gacccctaaa 61080
   gattaaggac agtgattggc ggtacctgcc ccaggtcctg tctcttggct acacaccccg 61140
   tttcctcaac ttttgttcac ggacagtggt ttccaggcca ctgcgtcacg gttggacagt 61200
   gatgtgtcac aggccatggg cttctggagc tggccgagca ctctgggcat agctcagtag 61260
   gcgggcaggg tggctgccac ctgtgtgggt caggccttgc ttctgttgct gaagctctgt 61320
   ggtaacgggc aggaaagctg acgtaggatg ggagatgctc catttgggaa ggcgagtgta 61380
   tgccaattgc aatctttctc aaggggccca cgtttttaat gatgtatgtg agttactgca 61440
   cgtgtgtatg ttgtgtctgt ttgtggaaga aaacaccagc attgatggct tgtagctggt 61500
   cgtgtccatg aatgattgct ggccttgcct atggtctgga tcagtccttg ttctccatct 61560
   tgtttttcca ctgtgcagtt ggttttgtag atggctgccg tctgctttaa ggacgtgagg 61620
   tgttgtaaac caaccctcgg caattaattt gggggaagag cagaagaaat gaagcccaca 61680
   tcccttacta gcttaccagt tgttaacagg ctggtgcaat cattagtttt ataaaaatca 61740
   cttttgcaaa taaagttttg cagagggttt ccccactctt ccctcatccc cttcatggac 61800
   gtctgagaat ccaggccctc ctctcctcct cctggatgta actcaggcgt gtccgtggcc 61860
   tgcaggcacc agcgggccag cagcactggc agaagacaag tctgaggccc aaggcccagt 61920
   gcagattctg actgtgggcc agtcagacca cgcccaggac gcaggggaga cggcagctgg 61980
   tgggggcgaa cggcccagcg ggcaggatct ccgtgccacg atgcagagga agggtgagcc 62040
   ccatggggac ccagtgatac ccccaaaact cagtcccagg ttctcagatg cacctttctc 62100
   tgggagcatg gccttcctgt gtccaaaccc ctccctggca atggtgggtg agggtggggc 62160
   acacttcgga gacaaatgag aaactcttag gcagggcccc tgctaaggcc ccagggaggc 62220
   cctgatttgg gggcagattt ccttttcctt ttgctcctta gaaaaagctc cttttgcaag 62280
   aagtttccca tatgtttgct gagctactgg gagcagggcc aggataccaa gagacggggt 62340
   agatcctccg tggaaacctg ctgttcagtt ttcccaccac aagccctgcc ctagggagcc 62400
   cctggtgcag ggtggcagag ggaagaagcc tggcagtgcc acacattgtg aggaatctaa 62460
   tcaaggatgg cttcctgtac aaaggacttt gagacaactt gaggatgggg atggcattcc 62520
   cagggaatgg caggcgggag gacagctgag atagaagctc agggcaaaga tgaggagtca 62580
   gtgggtcccc tccacattgg gctgaggaag gagctgaggc ctgtgccctg gcagattccg 62640
   tggtctatgc cagccaaggc ctctaatagg cctgaggtca gaaatggaga ttcccagttc 62700
   aaggtgagct cagtgcaggc cttgatacac aagagacagt ggtagggtgg ctgctaggta 62760
   gtggggtaat gtagggactg agctgaaact gggtggtggg gatatatcct gaggattgtg 62820
   gccagccccg gctcatgtgt gtacctgaga gaatatcctt ttatatctgg acatgtgtgg 62880
   gaatatatgt gtgaatggga gtctatatgt gtagatatgg ctaagagtgt gtgcataagt 62940
   ttgtgggggt acaggtgagt cagtgtctga acatgagtat gtgaccatgt gtatttcagg 63000
   ggcagcgtag acttctcctc cttcatccct tcttcttctc tccttggccc aggcatctcc 63060
   agcagcatga gctttgacga ggatgaggag gatgaggagg agaatagctc cagctcctcc 63120
   cagctaaata gtaacacccg ccccagctct gctactagca ggaagtccgt cagggtgagt 63180
   gagtgagtct gcatccacag cagtttttgg aggactgctc atccgttaga ggtggactgc 63240
   atgtgaagag atggactcgt atgcctttag gagcttctct gctggcctct tatgtccctc 63300
   taccttgcct cctaacctct tcagctaggc cagcagggtg atgtatgggg ggagatgcag 63360
   ttggacagga tgacctctga ggacctcccg tatctcccat ctccacctct aggaactgtt 63420
   gagggcaggg ctgggaagat agcttctgac cccaggccca ggctggccag gccccaatcc 63480
   caggatcctt ccctctctcc caccgccacg ttaggaggca gatttggatc ccagaccacc 63540
   aatttgggct gcttagggtc cttggggctc aggcacctat tctgcatccc cataggaggc 63600
   agcctcagcc cctagcccaa cagctccaga gcaaccagtg gacgttgagg tccaggatct 63660
   tgaggagttt gcactgaggc cggcccccca gggtatcacc atcaaatgcc gcatcactcg 63720
   ggacaagaaa gggatggacc ggggcatgta ccccacctac tttctgcacc tggaccgtga 63780
   ggatgggaag aaggtaaggt tggtctgggc atgttatcat ctaggcttta cagccctttg 63840
   aaatcctagg ggctgaaatg tgactggaag tctcatatct accactgacc tctcagttcc 63900
   tcaaagaaac tgccttcgtg tctggtctgt gcacatcttt gtgttttcca gtgcatttgt 63960
   gtgtgtgcac atatgtgcgt ttgggagctg acgcaacgga gagagtctgt gtgagtggct 64020
   ctcatgactg tgtgcagacc agaggctgag tctggaatat gacctcattc cactccccaa 64080
   ggtgttcctc ctggcgggaa ggaagagaaa gaagagtaaa acttccaatt acctcatctc 64140
   tgtggaccca acagacttgt ctcgaggagg ggacagctat atcgggaaac tgcggtacta 64200
   gcattccccc aggaagcagg cgggagtggg agggaggggc aggggcaagc tgtctgtaga 64260
   gggcctgaat cttcctgaag gagatctagg ccagggatgg atactctccc aggatcctct 64320
   ctgataatca catccaactg gaggcctatg tctatgccag cctagagcca gacttggaga 64380
   tgggaatcac acacccgacc ccaagctgtt cccaggaggt gggtgcaggc ccaccaagag 64440
   tgatggatcc aaccccaggg tgtcactgat aacgcaggcc accatggaag agttgccttg 64500
   gctccatggt caatgccaag ggacagggct gagagtgagc tcttgaggga agagacacac 64560
   agcaatcttg gggtgggggg gcagtggtga aggggcagga aggctctgag cctaggcctc 64620
   caggtggggg cagtggggag gtagggtttg ctgaggaact gagtaccaga tttggggagc 64680
   ataaataaag atgagaggtc aggagctaaa gctggagatg gggctggact gagacttagg 64740
   ctggctgcga cagaggagat ctcatcctct ctccacgggt gctaagcctc ttccactgtc 64800
   ttatcagatg ccattctgtt tgctcacctc ccatgaggag aactcccatg ttcccccaga 64860
   taaatcttct gaagaatcct gattgacctc cctgaattgc tctcactgaa ctgaaatgca 64920
   ctttgagtca actcagagca agtccaggcc ttctgcccac gaagtgtctt caaagatgtg 64980
   gattcagtga gcagtatgcc tccctgggcc tgctcctgtt ccagcccaga atgttttgca 65040
   ggctcctcat aggacagacg atgagctgtt ccctgctctg gggcagaggg tgcatgactc 65100
   tatactgatt gtgcctttat ttcaggtcca acttgatggg caccaagttc actgtttatg 65160
   acaatggagt caaccctcag aaggcctcat cctccacttt ggaaagtgga accttacgtc 65220
   aggagctggc agctgtgtgc tacgtgagtc ctaggttcgg gggtctctga tttccaaggt 65280
   agatatgaaa tccaggactt gatgcctgat ctaggggcta tcccatccat cttagtgggt 65340
   agacaaggct gtgtggagag gggctgtcct ctgtggagtg ttcctggcct aggacagggg 65400
   ctctggctct ctcctcctga cttcagagct cacaccacag ccggccatct gcttggggag 65460
   ctcagaaaac caggcctggc tctctgcagt ccaggccagg agtctcttag acagaaggag 65520
   acggtcgccc tgggttcttg tctgtgcatt tcccagatca tccctctggc atgccaggtt 65580
   ctacatggat tacacggcac tttgtcatct cactgaacgt tcaacctaag aggtagatcc 65640
   tattttcttt atagatgagg aaactgaagc tcagctgcag atgtcacaca gctcaaaaac 65700
   gaccaaactg gaatataaat ataggggatt actctacccc tttgaacctt tttcttcatt 65760
   tttaaaatca ggggtgggta tctcatcaac ctgggcatgg gcacttcttg gaggccatca 65820
   gtaagtaatc cacagctact ttgtatgatc ctgtggtgcc acctgctgac cagttttggg 65880
   gaggcagcct gagtcttcaa gttcttactg agctccaact aagcatattg tttgaggcac 65940
   taaggatata gtaatgaaca gacataaatg tataccttta tgggacttaa agccctagca 66000
   gggcagacaa gctatgaaac agatctatca gtgaagtaag tatgttcttt ataaacacta 66060
   agaagaaaaa gtaggggagg ggagataaga aatgtcaagg tatgtgtgag aatttcagaa 66120
   tggcagggag ggcgtcactg aggtagctct tgagtaaaga ccagaaggaa gtgaaaaaca 66180
   gcagtgtacg tgtctagggg acacagtgag gacaaaagtc ctgaatgcct ggcatgaaga 66240
   aggaatggag tggataccag gcagtaaatg gtgaggagag gaaggtcatg gggccacgtg 66300
   caggtgaagt cacatctcca gctttaactt ggagtgagct ggagagccac tggagagttc 66360
   tgagcagagg agaaagatca tctgacttaa tgtgcctgag cactggctgc tgtgttgaga 66420
   acagatggtg ggaaggagga ataggagggc caacaacaga aaccaggagt ccaggcaaag 66480
   gctgttagaa tgatcttggt gagagaagct agtggtgaaa agtggtggaa gtctgggtat 66540
   aattggaagg tggagctgac agaaactgct gatggattag acgtaggatg tagaagagag 66600
   gactcgagtg tgaccaagta gtttgccgga gcgagtggaa gaacagcatt gccgtgagca 66660
   gaagcaagga tgacgcataa gaggagctag ttctggcagg gtagagaccc aggggctcag 66720
   ttctggccgt gttaggttta gagggatgtg tgttagactt cggagtggag atggtgggaa 66780
   ctagctcttc ctctttattc ccgtcccccc caccttctcc agtaggtaaa tagacgcctc 66840
   aggtggccag tgttgcgttc tctttcccag gagacaaacg tcttaggctt caaggggcct 66900
   cggaagatga gcgtgattgt cccaggcatg aacatggttc atgagagagt ctctatccgc 66960
   ccccgcaacg tgagtgtcta ccccttcctc ccctctttcc ccatcatcct agtctctgca 67020
   tgagcttcta agggcagaac tccagctgat gtgtgtatgt ggaggggtac catgtgagaa 67080
   agccctggag gtctagggaa atccaaggac ccccattccc gggatagatc cctttctggg 67140
   gtggtcatgg tgccaaaggc ctgggcctgg ctcaggtgag gctgccctcc caggagcatg 67200
   agacactgct agcacgctgg cagaataaga acacggagag tatcatcgag ctgcaaaaca 67260
   agacacctgt ctggaatgat gacacacagt cctatgtact caacttccat gggcgcgtca 67320
   cacaggcctc cgtgaagaac ttccagatca tccatggcaa tgaccgtgag tgtttctgtc 67380
   cctactcatt atggtccgta ggatacccaa ggcccttagc gtagggttca gcccacctag 67440
   ccctgcctac actggctaga gtttaagaat gtgagctata cagctaaggt tagatgtatg 67500
   gaactttcta accctaatga ctgggaggtc ctggaagaac cttctttgca gccctggtcc 67560
   tagattctgt gtattcaacg gagtctcagg cacgggaaca ccctttaaaa ggacttttcc 67620
   tcttttctgt cccctggtgt tcacatgcat cttactttgt cctttgccat ctgccacctc 67680
   tttcctgcca cttctcccaa ttggcctttg ttttacttcc ctttgtgatt cccctggcat 67740
   ctctgcttct cacttgttct tccctcatgt ggtttgggtg tctgtctatc cttccctggc 67800
   tctaccattc ctgtcctgtc cttttctctg tctgtgcctg tgcttggccc cagcggacta 67860
   catcgtgatg cagtttggcc gggtagcaga ggatgtgttc accatggatt acaactaccc 67920
   gctgtgtgca ctgcaggcct ttgccattgc cctgtccagc ttcgacagca agctggcgtg 67980
   cgagtagagg cctcttcgtg ccctttgggg ttgcccagcc tggagcggag cttgcctgcc 68040
   tgcctgtgga gacagccctg cctatcctct gtatataggc cttccgccag atgaagcttt 68100
   ggccctcagt gggctccctg gcccagccag ccaggaactg gctcctttgc ctctgctact 68160
   gaggcagggg agtagtggag agcgggtggg tgggtgtgaa gggatgagaa taattctttc 68220
   catgccacga gatcaacaca cactcccacc cttggggtag tagtgtgttg tagtcgtact 68280
   taccaagctg agcaacctct tcagctggga aggccgcaag aggcatagag ggagaggaag 68340
   cacactgcag ggctgctgtg gcccagtcgt ccgctcagcc aaggagtcag atggcaatgg 68400
   gtactccagc aggtaggggc acagtgaatg tgtgtatgta tgaaggccac atcaacttta 68460
   tgtagcaaag ggcttggtgg ccaagcctgg cccttaaaca actgcagaaa gcccttcaac 68520
   ttcagaaggc ctcactcaag cctgagagaa gttgggaggg tggtggggac aggtaagtgg 68580
   caggaccctg tcaggattgc aggtgcctgg cttgctgtgg ctatgggaat cagctggtgg 68640
   ctaggtttct agcgcatttg atttctccag gtttgctgtg tctcacagag gcagtaggaa 68700
   cccagctctc agggctgtct tggtggatgg gccctgcaag acacaggctc agcatgcaga 68760
   agtgcatgaa cagggtccct ggatcagggt tgttctggga gtcctgtcag cttccccagg 68820
   agctctctgc tgagcagccc agcacaaccc ccaggaaaca caaatggggt ccaggtcacc 68880
   agcctgactg cacacagcta ggcatgcctg ggaatcctgc tgccagagaa ccattcccaa 68940
   gccatggcat gctccttgaa gaatctctcc tctctctctc tctctggaaa gacccaactt 69000
   tctcactgct gtcagccaag tcatggttgg taaccatgta ggttcttggg agggaatggg 69060
   acagggtgaa taaagcaggg aatattttag aaccacaaga gatcagcagt ggcaggaccc 69120
   ttaggaatct agtacaacct tgttgcttta ggtgagtcac actcagaaaa tggggcttgc 69180
   cctgggtcac ctagctggtt aatggcagca ttcagaactt caagttctct tgatttcttt 69240
   gttcccactg tcccccaaga aactagtatc tctggcctcc tgggggccca ttctgcatgc 69300
   cctccccact tcccccgatt cagagactta gaagcatcag agttgataaa ttagattgaa 69360
   accattccag taggaaatca ggcagttccc tgttgaaagt tgtctggctt tttgcacgtg 69420
   agtatgatcc aggattggcc tgtgtctggc ccctccgcga gggctactca gcagtaagca 69480
   ggccttagta cggctgctgc acacttctcg gtctaggtgg gagagagtgc ataaaggttc 69540
   tggggctggg cctgagccta actccctcca ctaccgctat ttctccttgg atggcaccat 69600
   ccaccctgca ggcttgggaa ctgtaagctc aagcttttgc tcatttgtca tttaccctgg 69660
   tggaggcttc ttgcccagat agagatctga aattggtggg aagcaagcag gggtcgtacc 69720
   ttgctttaga gtagatgttt ggaaaagcta agtagaaatt ttttaaaata gaagttgtat 69780
   tttaaacgtg ttaggcttta taggtgaagg acttgttttg acttaagtag aagtaatatg 69840
   ctctcatttc tttgtaatct agattttcct atgttgaatg cttaaagcaa ggcatacctg 69900
   tcctagcagg atctgcccac acctcggccg tacatctctg tgagagaccc cctccaacga 69960
   ttagcttttg caacatggtc cagtttctag agggcactag aactttgtgc cagcgctggc 70020
   gagataccat ggtgccatcc tcctttctat agttccagtt tagcccttcc ctgcctttaa 70080
   ggagaagaga tcttcatggt gacatttgag atgatggaaa ctagatcgtc tctaatgtta 70140
   ggtcaagcta tttctctgga tctatgattt taagatagaa caggctggtt ctgagttctg 70200
   gtgagacgga gaccccggtg gcagtggaaa aatcagaagc acaaaggaaa actcttggcc 70260
   tctgctttct cctgtagcac aaagagggag ttggctggat cttttggtgg agtcaggaaa 70320
   gacaggctga gctttctagc ctaagtgtgg agaaggatag cctcagccac aaaactctgt 70380
   caggcattga atgacaagca tcagtagctg agtgcactcc tggtgatcct ggttttcctt 70440
   gaccactggc cctggggcca tgggtgcatg gactcgtaaa gctgctaggg cccctagaaa 70500
   ccacctgggt tcagcctctc ccacaataag agttcttgcc cttcataccc tctacctgga 70560
   tggatggtct ttgggcaggg aattaatgac agaatgagca gtccctaatg gtattgggca 70620
   gttgggagtg agtttaggca gcttctctgt aaccgcttct ggggccacac cccaaaactc 70680
   tacaccctct gccccagaac aattctgcag agtggtgaag ttcacttccc caactcttct 70740
   ccaagtctag ctttcctttc cttctggctc caaggtgctc tgtgtccgtc tgtgtatgtg 70800
   tgtgtctgtg cgtctgtgtg tttatttggg gaaaagggca atcagcaatc aactgaggtt 70860
   tcttaattgt gattatattc ctgagaatga atgagtagga aaggaataag gatgttgtta 70920
   tgatcgcctg tggattttgt ccattcatct catgctctct gaacagaagg tatacctggg 70980
   aagataccag ctttttctct cagggttctg agtcagagga caccatccaa gaatgttgtt 71040
   agcttttcag ttcccgctct gcattgcctg aaaaaactgg tatgttgcac gtatgctttt 71100
   ggttgaaatt gaacatactc tagtgaatgt gccttaaaaa tcaagacact agttcagagg 71160
   aagcacataa tgtccagatc tatggcgaac acaggtgttg gaagccagga ctgtgataat 71220
   cagaagctat aagagaactt tcggagcctg cctccttcag tgacaagtag ggcacaaaat 71280
   tctagaagca gaaggttgtt tttttttttt tttctccatt ctgaaaatag caggacattt 71340
   acctcttaaa taaacttagc atttagaggt aattctaaat ttaacaagtg acagggttgg 71400
   tttcaaagaa aaaggtccat gctttgctta caatggagtc tgcagtgagg ggagatgctg 71460
   ggatagccat ttccatggct ctgttatgca agcacaaatt tcatctccta gatggacttc 71520
   ctggttttct cttactgcag taacactggc cttcccttct ctaattcctt accccagctg 71580
   cggcatccct gtgttaactc aggatgccaa gtggccctca gattacactt ctccagatag 71640
   ctgaatgagt ctgctttcac tgtgactggg acctgaatga cctgcagtca gggcccagag 71700
   ttgggactct atactaccct gggctctggt ctgtaggttt gtagtagcca ccggtaataa 71760
   gccaagggct aggctcttgt ttgagtttat ggccacctgg aattttcagt catctcatga 71820
   tacaggcggg aggggcagaa cagatagatt acgacaggtt tggtttttaa attttccaac 71880
   caagtggaaa ggcaagttgg tcttatagaa agcactactg cacttagtag ctatgtgatt 71940
   ttgagcaaac cacataatct ctctaggtcc attttcctaa ccacaagata aagatgttac 72000
   attgtcaaag cttgccgtag gatttggggt gaatgaaaat tattccttgc tttcatcact 72060
   acctttatag ctctcatcac tacctttata gctcatcact gtgccttttt ttctttccta 72120
   agaaagacat cacatccctc tcctctcctc ctctgtgctc ctgtccctcc ctccccctag 72180
   caaggtccag gcaaagctgg agatgaggct gagatccaga gtttcctaga acgcaactta 72240
   ggatggctag gaaagggaag cctgactgct cggtcaggag ggtgcagtat ctcttgctgg 72300
   gaacacagcc agttttcaca atgcctagac tgtgtatgtc tatttgcaca agattgtctt 72360
   ttcctatttt ggagtggtca gacattttat ttttgttcaa gattatctgg cgttttagac 72420
   aaatttgcaa aactgtgctt ttattgactt tttgaataaa ctttggtatt ctggagcaaa 72480
   tgtatttatt tattggtatg tgcaatgaca aacttggtat tttcccatgt tgacattatg 72540
   tatgttgtag aatttagtgt ttgtctaagt acacacatat atcaacaaat taaacttgaa 72600
   tcgtttcaac acttctgtgt acttttttca taaaggggaa aaaagccctg tttacttcct 72660
   aatttttttc tatacctttc attcattgtt tcctaggagc ctcaggtggg taagtaggtg 72720
   ataatagcaa agagatcata acctcaccaa tgacatgtgg gtcaacactg tccttcctct 72780
   ggcctgtgcc tttcaagttt aaggttcatc agaaccacac ccagaggctg gaaaaatggt 72840
   gaagtcagaa ggtacttaag ttagagcagt tctcttcagg gtctgctgtg ccgcctgaag 72900
   gggctggtgg ttcctcagta tcccctgtgg gcctggcctt aggaagagaa cagggctgta 72960
   gagatgaaaa aggtataaaa gacccaacca cttgatgtct cagggcttgc ttggacccta 73020
   acccatttaa acaggacctc gagatgctta ggaatctttt tttgttgccg ggcagctgtg 73080
   gcagatttga aggcaaaagg aagggaggct acagttgtag ctagaagcag acatgaaagc 73140
   taatggtctg acccagacaa gaggttataa ctaaaggcac ccggagactg cagaagggcc 73200
   ctgggccact cctggcaatc agggtagcct tagagggtgt caggtgtgac atctcagcaa 73260
   cactggactt gacagagtgg tgacagggat catgctgtca ccaagcacct accacatgct 73320
   ttgccctggc acagacactt tataaacagc atcttattta atccaaacag ccctgtaaat 73380
   agagcattat tcccaattca tggggaaact ggttctctga gaggttaagg aatgacctaa 73440
   gaggagaaat ttgggctatt ttctttgtat aaaaaaaggg aaataaagga aaaggaagca 73500
   cattttagaa aagtcaatgg tcaaggcaaa gtcataaatg tgttcatgtt ttttctcaat 73560
   attgctgaat agctaaagca ctaaattttt ctttggaata caaatagtcc agattgaaga 73620
   gaactttgag agtgtggtaa taattcgggt ttgtttattc ccccagatct agagttctga 73680
   tgtgaccaag agcatttcct acctgaacca tggcctatct cctgtggcct ctgcctctgg 73740
   tgggaaatag cgcatacagg atattctaaa aaggaaagag ggttttgttt gtttagtcat 73800
   caggttcaaa aatcttaaat ggctatagac aggggtgacc ctacgtccag gacagtctta 73860
   gtttatgcct atcaccttgg cataattata ttaaagctcc ctttttccaa agtgtttgaa 73920
   tttggacctt aagttatttg atcacccaag ccatataagg tgcattgtat ggttctcaaa 73980
   ggccgttgat tctacaggcc aaatacacct ccactcctca tcacgactcc ctgggcccag 74040
   cttctggagc tagtcatgga gataatggtt tctttgtgct cctgttgtga ccaggaaagg 74100
   ggatctcctg gctgcaccac ccacctgatt taccaagagt gatcccagat tatgcagttg 74160
   ttttgtgagc taactgtcca taaaggtatg acttgtgtac tctgccacag tgccaaaagg 74220
   ccaggggact gatcaagttc ccagcaccct cgtgagaaat gaagaccgca agaaaagaga 74280
   cagcagccct ggcaacaaaa accggaccca tgattttgtc atcctgggtt gtatatacac 74340
   atataactgt catccccgaa ggataaaata attctggaaa atccaaaaag aattttctaa 74400
   atcctaaata actgaaaggt acatccaaat gtctgatata taaatacaca aatatattcc 74460
   ttatatataa accaa 74475
<210> 2
   <211> 74475
   <212> DNA
   <213> homo sapiens
<220>
   <221> misc_feature
   <223> Mutated TUB gene
<400> 2
<210> 3
   <211> 561
   <212> PRT
   <213> homo sapiens
<220>
   <221> misc_feature
   <223> humanTUB561
<400> 3
   Met Gly Ala Arg Thr Pro Leu Pro Ser Phe Trp Val Ser Phe Phe Ala
   1               5                   10                  15
   Glu Thr Gly Ile Leu Phe Pro Gly Gly Thr Pro Trp Pro Met Gly Ser
               20                  25                  30
   Gln His Ser Lys Gln His Arg Lys Pro Gly Pro Leu Lys Arg Gly His
           35                  40                  45
   Arg Arg Asp Arg Arg Thr Thr Arg Arg Lys Tyr Trp Lys Glu Gly Arg
       50                  55                  60
   Glu Ile Ala Arg Val Leu Asp Asp Glu Gly Arg Asn Leu Arg Gln Gln
   65                  70                  75                  80
   Lys Leu Asp Arg Gln Arg Ala Leu Leu Glu Gln Lys Gln Lys Lys Lys
   85 90 95
   Arg Gln Glu Pro Leu Met Val Gln Ala Asn Ala Asp Gly Arg Pro Arg
   100 105 110
   Ser Arg Arg Ala Arg Gln Ser Glu Glu Gln Ala Pro Leu Val Glu Ser
   115 120 125
   Tyr Leu Ser Ser Ser Gly Ser Thr Ser Tyr Gln Val Gln Glu Ala Asp
   130 135 140
   Ser Leu Ala Ser Val Gln Leu Gly Ala Thr Arg Pro Thr Ala Pro Ala
   145 150 155 160
   Ser Ala Lys Arg Thr Lys Ala Ala Ala Thr Ala Gly Gly Gln Gly Gly
   165 170 175
   Ala Ala Arg Lys Glu Lys Lys Gly Lys His Lys Gly Thr Ser Gly Pro
   180 185 190
   Ala Ala Leu Ala Glu Asp Lys Ser Glu Ala Gln Gly Pro Val Gln Ile
   195 200 205
   Leu Thr Val Gly Gln Ser Asp His Ala Gln Asp Ala Gly Glu Thr Ala
   210 215 220
   Ala Gly Gly Gly Glu Arg Pro Ser Gly Gln Asp Leu Arg Ala Thr Met
   225 230 235 240
   Gln Arg Lys Gly Ile Ser Ser Ser Met Ser Phe Asp Glu Asp Glu Glu
   245 250 255
   Asp Glu Glu Glu Asn Ser Ser Ser Ser Ser Gln Leu Asn Ser Asn Thr
   260 265 270
   Arg Pro Ser Ser Ala Thr Ser Arg Lys Ser Val Arg Glu Ala Ala Ser
   275 280 285
   Ala Pro Ser Pro Thr Ala Pro Glu Gln Pro Val Asp Val Glu Val Gln
   290 295 300
   Asp Leu Glu Glu Phe Ala Leu Arg Pro Ala Pro Gln Gly Ile Thr Ile
   305 310 315 320
   Lys Cys Arg Ile Thr Arg Asp Lys Lys Gly Met Asp Arg Gly Met Tyr
   325 330 335
   Pro Thr Tyr Phe Leu His Leu Asp Arg Glu Asp Gly Lys Lys Val Phe
               340                 345                 350
   Leu Leu Ala Gly Arg Lys Arg Lys Lys Ser Lys Thr Ser Asn Tyr Leu
           355                 360                 365
   Ile Ser Val Asp Pro Thr Asp Leu Ser Arg Gly Gly Asp Ser Tyr Ile
       370                 375                 380
   Gly Lys Leu Arg Ser Asn Leu Met Gly Thr Lys Phe Thr Val Tyr Asp
   385                 390                 395                 400
   Asn Gly Val Asn Pro Gln Lys Ala Ser Ser Ser Thr Leu Glu Ser Gly
                   405                 410                 415
   Thr Leu Arg Gln Glu Leu Ala Ala Val Cys Tyr Glu Thr Asn Val Leu
               420                 425                 430
   Gly Phe Lys Gly Pro Arg Lys Met Ser Val Ile Val Pro Gly Met Asn
           435                 440                 445
   Met Val His Glu Arg Val Ser Ile Arg Pro Arg Asn Glu His Glu Thr
       450                 455                 460
   Leu Leu Ala Arg Trp Gln Asn Lys Asn Thr Glu Ser Ile Ile Glu Leu
   465                 470                 475                 480
   Gln Asn Lys Thr Pro Val Trp Asn Asp Ala Thr Gln Ser Tyr Val Leu
                   485                 490                 495
   Asn Phe His Gly Arg Val Thr Gln Ala Ser Val Lys Asn Phe Gln Ile
               500                 505                 510
   Ile His Gly Asn Asp Pro Asp Tyr Ile Val Met Gln Phe Gly Arg Val
           515                 520                 525
   Ala Glu Asp Val Phe Thr Met Asp Tyr Asn Tyr Pro Leu Cys Ala Leu
       530                 535                 540
   Gln Ala Phe Ala Ile Ala Leu Ser Ser Phe Asp Ser Lys Leu Ala Cys
   545                 550                 555                 560
   Glu
<210> 4
   <211> 561
   <212> PRT
   <213> homo sapiens
<220>
   <221> misc_feature
   <223> Mutated TUB561
<400> 4
   Met Gly Ala Arg Thr Pro Leu Pro Ser Phe Trp Val Ser Phe Phe Ala
   1               5                   10                  15
   Glu Thr Gly Ile Leu Phe Pro Gly Gly Thr Pro Trp Pro Met Gly Ser
               20                  25                  30
   Gln His Ser Lys Gln His Arg Lys Pro Gly Pro Leu Lys Arg Gly His
           35                  40                  45
   Arg Arg Asp Arg Arg Thr Thr Arg Arg Lys Tyr Trp Lys Glu Gly Arg
       50                  55                  60
   Glu Ile Ala Arg Val Leu Asp Asp Glu Gly Arg Asn Leu Arg Gln Gln
   65                  70                  75                  80
   Lys Leu Asp Arg Gln Arg Ala Leu Leu Glu Gln Lys Gln Lys Lys Lys
                   85                  90                  95
   Arg Gln Glu Pro Leu Met Val Gln Ala Asn Ala Asp Gly Arg Pro Arg
               100                 105                 110
   Ser Arg Arg Ala Arg Gln Ser Glu Glu Gln Ala Pro Leu Val Glu Ser
           115                 120                 125
   Tyr Leu Ser Ser Ser Gly Ser Thr Ser Tyr Gln Val Gln Glu Ala Asp
       130                 135                 140
   Ser Leu Ala Ser Val Gln Leu Gly Ala Thr Arg Pro Thr Ala Pro Ala
   145                 150                 155                 160
   Ser Ala Lys Arg Thr Lys Ala Ala Ala Thr Ala Gly Gly Gln Gly Gly
                   165                 170                 175
   Ala Ala Arg Lys Glu Lys Lys Gly Lys His Lys Gly Thr Ser Gly Pro
               180                 185                 190
   Ala Ala Leu Ala Glu Asp Lys Ser Glu Ala Gln Gly Pro Val Gln Ile
           195                 200                 205
   Leu Thr Val Gly Gln Ser Asp His Ala Gln Asp Ala Gly Glu Thr Ala
       210                 215                 220
   Ala Gly Gly Gly Glu Arg Pro Ser Gly Gln Asp Leu Arg Ala Thr Met
   225 230 235 240
   Gln Arg Lys Gly Ile Ser Ser Ser Met Ser Phe Asp Glu Asp Glu Glu
   245 250 255
   Asp Glu Glu Glu Asn Ser Ser Ser Ser Ser Gln Leu Asn Ser Asn Thr
   260 265 270
   Arg Pro Ser Ser Pro Thr Ser Arg Lys Ser Val Arg Glu Ala Ala Ser
   275 280 285
   Ala Pro Ser Pro Thr Ala Pro Glu Gln Pro Val Asp Val Glu Val Gln
   290 295 300
   Asp Leu Glu Glu Phe Ala Leu Arg Pro Ala Pro Gln Gly Ile Thr Ile
   305 310 315 320
   Lys Cys Arg Ile Thr Arg Asp Lys Lys Gly Met Asp Arg Gly Met Tyr
   325 330 335
   Pro Thr Tyr Phe Leu His Leu Asp Arg Glu Asp Gly Lys Lys Val Phe
   340 345 350
   Leu Leu Ala Gly Arg Lys Arg Lys Lys Ser Lys Thr Ser Asn Tyr Leu
   355 360 365
   Ile Ser Val Asp Pro Thr Asp Leu Ser Arg Gly Gly Asp Ser Tyr Ile
   370 375 380
   Gly Lys Leu Arg Ser Asn Leu Met Gly Thr Lys Phe Thr Val Tyr Asp
   385 390 395 400
   Asn Gly Val Asn Pro Gln Lys Ala Ser Ser Ser Thr Leu Glu Ser Gly
   405 410 415
   Thr Leu Arg Gln Glu Leu Ala Ala Val Cys Tyr Glu Thr Asn Val Leu
   420 425 430
   Gly Phe Lys Gly Pro Arg Lys Met Ser Val Ile Val Pro Gly Met Asn
   435 440 445
   Met Val His Glu Arg Val Ser Ile Arg Pro Arg Asn Glu His Glu Thr
   450 455 460
   Leu Leu Ala Arg Trp Gln Asn Lys Asn Thr Glu Ser Ile Ile Glu Leu
   465 470 475 480
   Gln Asn Lys Thr Pro Val Trp Asn Asp Asp Thr Gln Ser Tyr Val Leu
   485 490 495
   Asn Phe His Gly Arg Val Thr Gln Ala Ser Val Lys Asn Phe Gln Ile
   500 505 510
   Ile His Gly Asn Asp Pro Asp Tyr Ile Val Met Gln Phe Gly Arg Val
   515 520 525
   Ala Glu Asp Val Phe Thr Met Asp Tyr Asn Tyr Pro Leu Cys Ala Leu
   530 535 540
   Gln Ala Phe Ala Ile Ala Leu Ser Ser Phe Asp Ser Lys Leu Ala Cys
   545 550 555 560
   Glu
<210> 5
   <211> 506
   <212> PRT
   <213> homo sapiens
<220>
   <221> misc_feature
   <223> TUB506
<400> 5
   Met Thr Ser Lys Pro His Ser Asp Trp Ile Pro Tyr Ser Val Leu Asp
   1 5 10 15
   Asp Glu Gly Arg Asn Leu Arg Gln Gln Lys Leu Asp Arg Gln Arg Ala
   20 25 30
   Leu Leu Glu Gln Lys Gln Lys Lys Lys Arg Gln Glu Pro Leu Met Val
   35 40 45
   Gln Ala Asn Ala Asp Gly Arg Pro Arg Ser Arg Arg Ala Arg Gln Ser
   50 55 60
   Glu Glu Gln Ala Pro Leu Val Glu Ser Tyr Leu Ser Ser Ser Gly Ser
   65 70 75 80
   Thr Ser Tyr Gln Val Gln Glu Ala Asp Ser Leu Ala Ser Val Gln Leu
   85 90 95
   Gly Ala Thr Arg Pro Thr Ala Pro Ala Ser Ala Lys Arg Thr Lys Ala
               100                 105                 110
   Ala Ala Thr Ala Gly Gly Gln Gly Gly Ala Ala Arg Lys Glu Lys Lys
           115                 120                 125
   Gly Lys His Lys Gly Thr Ser Gly Pro Ala Ala Leu Ala Glu Asp Lys
       130                 135                 140
   Ser Glu Ala Gln Gly Pro Val Gln Ile Leu Thr Val Gly Gln Ser Asp
   145                 150                 155                 160
   His Ala Gln Asp Ala Gly Glu Thr Ala Ala Gly Gly Gly Glu Arg Pro
                   165                 170                 175
   Ser Gly Gln Asp Leu Arg Ala Thr Met Gln Arg Lys Gly Ile Ser Ser
               180                 185                 190
   Ser Met Ser Phe Asp Glu Asp Glu Glu Asp Glu Glu Glu Asn Ser Ser
           195                 200                 205
   Ser Ser Ser Gln Leu Asn Ser Asn Thr Arg Pro Ser Ser Ala Thr Ser
       210                 215                 220
   Arg Lys Ser Val Arg Glu Ala Ala Ser Ala Pro Ser Pro Thr Ala Pro
   225                 230                 235                 240
   Glu Gln Pro Val Asp Val Glu Val Gln Asp Leu Glu Glu Phe Ala Leu
                   245                 250                 255
   Arg Pro Ala Pro Gln Gly Ile Thr Ile Lys Cys Arg Ile Thr Arg Asp
               260                 265                 270
   Lys Lys Gly Met Asp Arg Gly Met Tyr Pro Thr Tyr Phe Leu His Leu
           275                 280                 285
   Asp Arg Glu Asp Gly Lys Lys Val Phe Leu Leu Ala Gly Arg Lys Arg
       290                 295                 300
   Lys Lys Ser Lys Thr Ser Asn Tyr Leu Ile Ser Val Asp Pro Thr Asp
   305                 310                 315                 320
   Leu Ser Arg Gly Gly Asp Ser Tyr Ile Gly Lys Leu Arg Ser Asn Leu
                   325                 330                 335
   Met Gly Thr Lys Phe Thr Val Tyr Asp Asn Gly Val Asn Pro Gln Lys
               340                 345                 350
   Ala Ser Ser Ser Thr Leu Glu Ser Gly Thr Leu Arg Gln Glu Leu Ala
   355 360 365
   Ala Val Cys Tyr Glu Thr Asn Val Leu Gly Phe Lys Gly Pro Arg Lys
   370 375 380
   Met Ser Val Ile Val Pro Gly Met Asn Met Val His Glu Arg Val Ser
   385 390 395 400
   Ile Arg Pro Arg Asn Glu His Glu Thr Leu Leu Ala Arg Trp Gln Asn
   405 410 415
   Lys Asn Thr Glu Ser Ile Ile Glu Leu Gln Asn Lys Thr Pro Val Trp
   420 425 430
   Asn Asp Asp Thr Gln Ser Tyr Val Leu Asn Phe His Gly Arg Val Thr
   435 440 445
   Gln Ala Ser Val Lys Asn Phe Gln Ile Ile His Gly Asn Asp Pro Asp
   450 455 460
   Tyr Ile Val Met Gln Phe Gly Arg Val Ala Glu Asp Val Phe Thr Met
   465 470 475 480
   Asp Tyr Asn Tyr Pro Leu Cys Ala Leu Gln Ala Phe Ala Ile Ala Leu
   485 490 495
   Ser Ser Phe Asp Ser Lys Leu Ala Cys Glu
   500 505
<210> 6
   <211> 506
   <212> PRT
   <213> homo sapiens
<220>
   <221> misc_feature
   <223> Mutated TUB506
<400> 6
   Met Thr Ser Lys Pro His Ser Asp Trp Ile Pro Tyr Ser Val Leu Asp
   1 5 10 15
   Asp Glu Gly Arg Asn Leu Arg Gln Gln Lys Leu Asp Arg Gln Arg Ala
   20 25 30
   Leu Leu Glu Gln Lys Gln Lys Lys Lys Arg Gln Glu Pro Leu Met Val
   35 40 45
   Gln Ala Asn Ala Asp Gly Arg Pro Arg Ser Arg Arg Ala Arg Gln Ser
       50                  55                  60
   Glu Glu Gln Ala Pro Leu Val Glu Ser Tyr Leu Ser Ser Ser Gly Ser
   65                  70                  75                  80
   Thr Ser Tyr Gln Val Gln Glu Ala Asp Ser Leu Ala Ser Val Gln Leu
                   85                  90                  95
   Gly Ala Thr Arg Pro Thr Ala Pro Ala Ser Ala Lys Arg Thr Lys Ala
               100                 105                 110
   Ala Ala Thr Ala Gly Gly Gln Gly Gly Ala Ala Arg Lys Glu Lys Lys
           115                 120                 125
   Gly Lys His Lys Gly Thr Ser Gly Pro Ala Ala Leu Ala Glu Asp Lys
       130                 135                 140
   Ser Glu Ala Gln Gly Pro Val Gln Ile Leu Thr Val Gly Gln Ser Asp
   145                 150                 155                 160
   His Ala Gln Asp Ala Gly Glu Thr Ala Ala Gly Gly Gly Glu Arg Pro
                   165                 170                 175
   Ser Gly Gln Asp Leu Arg Ala Thr Met Gln Arg Lys Gly Ile Ser Ser
               180                 185                 190
   Ser Met Ser Phe Asp Glu Asp Glu Glu Asp Glu Glu Glu Asn Ser Ser
           195                 200                 205
   Ser Ser Ser Gln Leu Asn Ser Asn Thr Arg Pro Ser Ser Pro Thr Ser
       210                 215                 220
   Arg Lys Ser Val Arg Glu Ala Ala Ser Ala Pro Ser Pro Thr Ala Pro
   225                 230                 235                 240
   Glu Gln Pro Val Asp Val Glu Val Gln Asp Leu Glu Glu Phe Ala Leu
                   245                 250                 255
   Arg Pro Ala Pro Gln Gly Ile Thr Ile Lys Cys Arg Ile Thr Arg Asp
               260                 265                 270
   Lys Lys Gly Met Asp Arg Gly Met Tyr Pro Thr Tyr Phe Leu His Leu
           275                 280                 285
   Asp Arg Glu Asp Gly Lys Lys Val Phe Leu Leu Ala Gly Arg Lys Arg
       290                 295                 300
   Lys Lys Ser Lys Thr Ser Asn Tyr Leu Ile Ser Val Asp Pro Thr Asp
   305                 310                 315                 320
   Leu Ser Arg Gly Gly Asp Ser Tyr Ile Gly Lys Leu Arg Ser Asn Leu
                   325                 330                 335
   Met Gly Thr Lys Phe Thr Val Tyr Asp Asn Gly Val Asn Pro Gln Lys
               340                 345                 350
   Ala Ser Ser Ser Thr Leu Glu Ser Gly Thr Leu Arg Gln Glu Leu Ala
           355                 360                 365
   Ala Val Cys Tyr Glu Thr Asn Val Leu Gly Phe Lys Gly Pro Arg Lys
       370                 375                 380
   Met Ser Val Ile Val Pro Gly Met Asn Met Val His Glu Arg Val Ser
   385                 390                 395                 400
   Ile Arg Pro Arg Asn Glu His Glu Thr Leu Leu Ala Arg Trp Gln Asn
                   405                 410                 415
   Lys Asn Thr Glu Ser Ile Ile Glu Leu Gln Asn Lys Thr Pro Val Trp
               420                 425                 430
   Asn Asp Asp Thr Gln Ser Tyr Val Leu Asn Phe His Gly Arg Val Thr
           435                 440                 445
   Gln Ala Ser Val Lys Asn Phe Gln Ile Ile His Gly Asn Asp Pro Asp
       450                 455                 460
   Tyr Ile Val Met Gln Phe Gly Arg Val Ala Glu Asp Val Phe Thr Met
   465                 470                 475                 480
   Asp Tyr Asn Tyr Pro Leu Cys Ala Leu Gln Ala Phe Ala Ile Ala Leu
                   485                 490                 495
   Ser Ser Phe Asp Ser Lys Leu Ala Cys Glu
               500                 505
<210> 7
   <211> 6432
   <212> DNA
   <213> homo sapiens
<220>
   <221> misc_feature
   <223> Tuba
<400> 7
   cttgaggatt cagtctggtc ctgaagggtt tggggggaga ctgcgaccag aagatgtttc 60
   catgtcctaa ttaatgggtg atggtggttg ttagtctgac tgttgccacg gtgatgaagg 120
   gagacatcca agtgctggtt tcagtactga ggcgaataca gggaatttca acaggctcca 180
   ggtcttacta tgcagcctga agtgggacca tcccttaaac ccactccatc ctgtggccac 240
   gatgggggcc aggacacctt tgccttcttt ctgggtttct ttctttgccg agacagggat 300
   tttgttccca ggaggcactc cctggcccat gggatctcag cattcaaagc agcacaggaa 360
   acctgggccc ctgaaacggg gccaccgaag agatcggaga acaaccagga ggaagtactg 420
   gaaggaagga agggagatcg ctcgtgtctt agatgatgag ggcagaaacc tgaggcagca 480
   gaagcttgat cggcagcggg ccctgctgga gcagaagcag aagaagaagc gccaggagcc 540
   cctgatggtg caggccaatg cagatgggcg gccccggagc cggcgggccc ggcagtcaga 600
   ggaacaagcc cccctggtgg agtcctacct cagcagcagt ggcagcacca gctaccaagt 660
   tcaagaggcc gactcactcg ccagtgtgca gctgggagcc acgcgcccaa cagcaccagc 720
   ttcagccaag agaaccaagg cggcagctac agcagggggc cagggtggcg ccgctaggaa 780
   ggagaagaag ggaaagcaca aaggcaccag cgggccagca gcactggcag aagacaagtc 840
   tgaggcccaa ggcccagtgc agattctgac tgtgggccag tcagaccacg cccaggacgc 900
   aggggagacg gcagctggtg ggggcgaacg gcccagcggg caggatctcc gtgccacgat 960
   gcagaggaag ggcatctcca gcagcatgag ctttgacgag gatgaggagg atgaggagga 1020
   gaatagctcc agctcctccc agctaaatag taacacccgc cccagctctg ctactagcag 1080
   gaagtccgtc agggaggcag cctcagcccc tagcccaaca gctccagagc aaccagtgga 1140
   cgttgaggtc caggatcttg aggagtttgc actgaggccg gccccccagg gtatcaccat 1200
   caaatgccgc atcactcggg acaagaaagg gatggaccgg ggcatgtacc ccacctactt 1260
   tctgcacctg gaccgtgagg atgggaagaa ggtgttcctc ctggcgggaa ggaagagaaa 1320
   gaagagtaaa acttccaatt acctcatctc tgtggaccca acagacttgt ctcgaggagg 1380
   ggacagctat atcgggaaac tgcggtccaa cttgatgggc accaagttca ctgtttatga 1440
   caatggagtc aaccctcaga aggcctcatc ctccactttg gaaagtggaa ccttacgtca 1500
   ggagctggca gctgtgtgct acgagacaaa cgtcttaggc ttcaaggggc ctcggaagat 1560
   gagcgtgatt gtcccaggca tgaacatggt tcatgagaga gtctctatcc gcccccgcaa 1620
   cgagcatgag acactgctag cacgctggca gaataagaac acggagagta tcatcgagct 1680
   gcaaaacaag acacctgtct ggaatgatga cacacagtcc tatgtactca acttccatgg 1740
   gcgcgtcaca caggcctccg tgaagaactt ccagatcatc catggcaatg acccggacta 1800
   catcgtgatg cagtttggcc gggtagcaga ggatgtgttc accatggatt acaactaccc 1860
   gctgtgtgca ctgcaggcct ttgccattgc cctgtccagc ttcgacagca agctggcgtg 1920
   cgagtagagg cctcttcgtg ccctttgggg ttgcccagcc tggagcggag cttgcctgcc 1980
   tgcctgtgga gacagccctg cctatcctct gtatataggc cttccgccag atgaagcttt 2040
   ggccctcagt gggctccctg gcccagccag ccaggaactg gctcctttgc ctctgctact 2100
   gaggcagggg agtagtggag agcgggtggg tgggtgtgaa gggatgagaa taattctttc 2160
   catgccacga gatcaacaca cactcccacc cttggggtag tagtgtgttg tagtcgtact 2220
   taccaagctg agcaacctct tcagctggga aggccgcaag aggcatagag ggagaggaag 2280
   cacactgcag ggctgctgtg gcccagtcgt ccgctcagcc aaggagtcag atggcaatgg 2340
   gtactccagc aggtaggggc acagtgaatg tgtgtatgta tgaaggccac atcaacttta 2400
   tgtagcaaag ggcttggtgg ccaagcctgg cccttaaaca actgcagaaa gcccttcaac 2460
   ttcagaaggc ctcactcaag cctgagagaa gttgggaggg tggtggggac aggtaagtgg 2520
   caggaccctg tcaggattgc aggtgcctgg cttgctgtgg ctatgggaat cagctggtgg 2580
   ctaggtttct agcgcatttg atttctccag gtttgctgtg tctcacagag gcagtaggaa 2640
   cccagctctc agggctgtct tggtggatgg gccctgcaag acacaggctc agcatgcaga 2700
   agtgcatgaa cagggtccct ggatcagggt tgttctggga gtcctgtcag cttccccagg 2760
   agctctctgc tgagcagccc agcacaaccc ccaggaaaca caaatggggt ccaggtcacc 2820
   agcctgactg cacacagcta ggcatgcctg ggaatcctgc tgccagagaa ccattcccaa 2880
   gccatggcat gctccttgaa gaatctctcc tctctctctc tctctggaaa gacccaactt 2940
   tctcactgct gtcagccaag tcatggttgg taaccatgta ggttcttggg agggaatggg 3000
   acagggtgaa taaagcaggg aatattttag aaccacaaga gatcagcagt ggcaggaccc 3060
   ttaggaatct agtacaacct tgttgcttta ggtgagtcac actcagaaaa tggggcttgc 3120
   cctgggtcac ctagctggtt aatggcagca ttcagaactt caagttctct tgatttcttt 3180
   gttcccactg tcccccaaga aactagtatc tctggcctcc tgggggccca ttctgcatgc 3240
   cctccccact tcccccgatt cagagactta gaagcatcag agttgataaa ttagattgaa 3300
   accattccag taggaaatca ggcagttccc tgttgaaagt tgtctggctt tttgcacgtg 3360
   agtatgatcc aggattggcc tgtgtctggc ccctccgcga gggctactca gcagtaagca 3420
   ggccttagta cggctgctgc acacttctcg gtctaggtgg gagagagtgc ataaaggttc 3480
   tggggctggg cctgagccta actccctcca ctaccgctat ttctccttgg atggcaccat 3540
   ccaccctgca ggcttgggaa ctgtaagctc aagcttttgc tcatttgtca tttaccctgg 3600
   tggaggcttc ttgcccagat agagatctga aattggtggg aagcaagcag gggtcgtacc 3660
   ttgctttaga gtagatgttt ggaaaagcta agtagaaatt ttttaaaata gaagttgtat 3720
   tttaaacgtg ttaggcttta taggtgaagg acttgttttg acttaagtag aagtaatatg 3780
   ctctcatttc tttgtaatct agattttcct atgttgaatg cttaaagcaa ggcatacctg 3840
   tcctagcagg atctgcccac acctcggccg tacatctctg tgagagaccc cctccaacga 3900
   ttagcttttg caacatggtc cagtttctag agggcactag aactttgtgc cagcgctggc 3960
   gagataccat ggtgccatcc tcctttctat agttccagtt tagcccttcc ctgcctttaa 4020
   ggagaagaga tcttcatggt gacatttgag atgatggaaa ctagatcgtc tctaatgtta 4080
   ggtcaagcta tttctctgga tctatgattt taagatagaa caggctggtt ctgagttctg 4140
   gtgagacgga gaccccggtg gcagtggaaa aatcagaagc acaaaggaaa actcttggcc 4200
   tctgctttct cctgtagcac aaagagggag ttggctggat cttttggtgg agtcaggaaa 4260
   gacaggctga gctttctagc ctaagtgtgg agaaggatag cctcagccac aaaactctgt 4320
   caggcattga atgacaagca tcagtagctg agtgcactcc tggtgatcct ggttttcctt 4380
   gaccactggc cctggggcca tgggtgcatg gactcgtaaa gctgctaggg cccctagaaa 4440
   ccacctgggt tcagcctctc ccacaataag agttcttgcc cttcataccc tctacctgga 4500
   tggatggtct ttgggcaggg aattaatgac agaatgagca gtccctaatg gtattgggca 4560
   gttgggagtg agtttaggca gcttctctgt aaccgcttct ggggccacac cccaaaactc 4620
   tacaccctct gccccagaac aattctgcag agtggtgaag ttcacttccc caactcttct 4680
   ccaagtctag ctttcctttc cttctggctc caaggtgctc tgtgtccgtc tgtgtatgtg 4740
   tgtgtctgtg cgtctgtgtg tttatttggg gaaaagggca atcagcaatc aactgaggtt 4800
   tcttaattgt gattatattc ctgagaatga atgagtagga aaggaataag gatgttgtta 4860
   tgatcgcctg tggattttgt ccattcatct catgctctct gaacagaagg tatacctggg 4920
   aagataccag ctttttctct cagggttctg agtcagagga caccatccaa gaatgttgtt 4980
   agcttttcag ttcccgctct gcattgcctg aaaaaactgg tatgttgcac gtatgctttt 5040
   ggttgaaatt gaacatactc tagtgaatgt gccttaaaaa tcaagacact agttcagagg 5100
   aagcacataa tgtccagatc tatggcgaac acaggtgttg gaagccagga ctgtgataat 5160
   cagaagctat aagagaactt tcggagcctg cctccttcag tgacaagtag ggcacaaaat 5220
   tctagaagca gaaggttgtt tttttttttt tttctccatt ctgaaaatag caggacattt 5280
   acctcttaaa taaacttagc atttagaggt aattctaaat ttaacaagtg acagggttgg 5340
   tttcaaagaa aaaggtccat gctttgctta caatggagtc tgcagtgagg ggagatgctg 5400
   ggatagccat ttccatggct ctgttatgca agcacaaatt tcatctccta gatggacttc 5460
   ctggttttct cttactgcag taacactggc cttcccttct ctaattcctt accccagctg 5520
   cggcatccct gtgttaactc aggatgccaa gtggccctca gattacactt ctccagatag 5580
   ctgaatgagt ctgctttcac tgtgactggg acctgaatga cctgcagtca gggcccagag      5640
   ttgggactct atactaccct gggctctggt ctgtaggttt gtagtagcca ccggtaataa      5700
   gccaagggct aggctcttgt ttgagtttat ggccacctgg aattttcagt catctcatga      5760
   tacaggcggg aggggcagaa cagatagatt acgacaggtt tggtttttaa attttccaac      5820
   caagtggaaa ggcaagttgg tcttatagaa agcactactg cacttagtag ctatgtgatt      5880
   ttgagcaaac cacataatct ctctaggtcc attttcctaa ccacaagata aagatgttac      5940
   attgtcaaag cttgccgtag gatttggggt gaatgaaaat tattccttgc tttcatcact      6000
   acctttatag ctctcatcac tacctttata gctcatcact gtgccttttt ttctttccta      6060
   agaaagacat cacatccctc tcctctcctc ctctgtgctc ctgtccctcc ctccccctag      6120
   caaggtccag gcaaagctgg agatgaggct gagatccaga gtttcctaga acgcaactta      6180
   ggatggctag gaaagggaag cctgactgct cggtcaggag ggtgcagtat ctcttgctgg      6240
   gaacacagcc agttttcaca atgcctagac tgtgtatgtc tatttgcaca agattgtctt      6300
   ttcctatttt ggagtggtca gacattttat ttttgttcaa gattatctgg cgttttagac      6360
   aaatttgcaa aactgtgctt ttattgactt tttgaataaa ctttggtatt ctggaaaaaa      6420
   aaaaaaaaaa aa                                                          6432
<210> 8
   <211> 6175
   <212> DNA
   <213> homo sapiens
<220>
   <221> misc_feature
   <223> Tubb
<400> 8
   tggcgtgcag cgcgggcctc ggcggggccc agcgccccgg cccgggagga tgcggcccgg      60
   ggcggcccgg gagctgagca gggcccccgc gccggcccct ccgggccccg gcctccagag     120
   ccgcagccac cgccccgccc ccgagagaca tgacttccaa gccgcattcc gactggattc     180
   cctacagtgt cttagatgat gagggcagaa acctgaggca gcagaagctt gatcggcagc     240
   gggccctgct ggagcagaag cagaagaaga agcgccagga gcccctgatg gtgcaggcca     300
   atgcagatgg gcggccccgg agccggcggg cccggcagtc agaggaacaa gcccccctgg    360
   tggagtccta cctcagcagc agtggcagca ccagctacca agttcaagag gccgactcac     420
   tcgccagtgt gcagctggga gccacgcgcc caacagcacc agcttcagcc aagagaacca     480
   aggcggcagc tacagcaggg ggccagggtg gcgccgctag gaaggagaag aagggaaagc     540
   acaaaggcac cagcgggcca gcagcactgg cagaagacaa gtctgaggcc caaggcccag     600
   tgcagattct gactgtgggc cagtcagacc acgcccagga cgcaggggag acggcagctg     660
   gtgggggcga acggcccagc gggcaggatc tccgtgccac gatgcagagg aagggcatct 720
   ccagcagcat gagctttgac gaggatgagg aggatgagga ggagaatagc tccagctcct 780
   cccagctaaa tagtaacacc cgccccagct ctgctactag caggaagtcc gtcagggagg 840
   cagcctcagc ccctagccca acagctccag agcaaccagt ggacgttgag gtccaggatc 900
   ttgaggagtt tgcactgagg ccggcccccc agggtatcac catcaaatgc cgcatcactc 960
   gggacaagaa agggatggac cggggcatgt accccaccta ctttctgcac ctggaccgtg 1020
   aggatgggaa gaaggtgttc ctcctggcgg gaaggaagag aaagaagagt aaaacttcca 1080
   attacctcat ctctgtggac ccaacagact tgtctcgagg aggggacagc tatatcggga 1140
   aactgcggtc caacttgatg ggcaccaagt tcactgttta tgacaatgga gtcaaccctc 1200
   agaaggcctc atcctccact ttggaaagtg gaaccttacg tcaggagctg gcagctgtgt 1260
   gctacgagac aaacgtctta ggcttcaagg ggcctcggaa gatgagcgtg attgtcccag 1320
   gcatgaacat ggttcatgag agagtctcta tccgcccccg caacgagcat gagacactgc 1380
   tagcacgctg gcagaataag aacacggaga gtatcatcga gctgcaaaac aagacacctg 1440
   tctggaatga tgacacacag tcctatgtac tcaacttcca tgggcgcgtc acacaggcct 1500
   ccgtgaagaa cttccagatc atccatggca atgacccgga ctacatcgtg atgcagtttg 1560
   gccgggtagc agaggatgtg ttcaccatgg attacaacta cccgctgtgt gcactgcagg 1620
   cctttgccat tgccctgtcc agcttcgaca gcaagctggc gtgcgagtag aggcctcttc 1680
   gtgccctttg gggttgccca gcctggagcg gagcttgcct gcctgcctgt ggagacagcc 1740
   ctgcctatcc tctgtatata ggccttccgc cagatgaagc tttggccctc agtgggctcc 1800
   ctggcccagc cagccaggaa ctggctcctt tgcctctgct actgaggcag gggagtagtg 1860
   gagagcgggt gggtgggtgt gaagggatga gaataattct ttccatgcca cgagatcaac 1920
   acacactccc acccttgggg tagtagtgtg ttgtagtcgt acttaccaag ctgagcaacc 1980
   tcttcagctg ggaaggccgc aagaggcata gagggagagg aagcacactg cagggctgct 2040
   gtggcccagt cgtccgctca gccaaggagt cagatggcaa tgggtactcc agcaggtagg 2100
   ggcacagtga atgtgtgtat gtatgaaggc cacatcaact ttatgtagca aagggcttgg 2160
   tggccaagcc tggcccttaa acaactgcag aaagcccttc aacttcagaa ggcctcactc 2220
   aagcctgaga gaagttggga gggtggtggg gacaggtaag tggcaggacc ctgtcaggat 2280
   tgcaggtgcc tggcttgctg tggctatggg aatcagctgg tggctaggtt tctagcgcat 2340
   ttgatttctc caggtttgct gtgtctcaca gaggcagtag gaacccagct ctcagggctg 2400
   tcttggtgga tgggccctgc aagacacagg ctcagcatgc agaagtgcat gaacagggtc 2460
   cctggatcag ggttgttctg ggagtcctgt cagcttcccc aggagctctc tgctgagcag 2520
   cccagcacaa cccccaggaa acacaaatgg ggtccaggtc accagcctga ctgcacacag 2580
   ctaggcatgc ctgggaatcc tgctgccaga gaaccattcc caagccatgg catgctcctt 2640
   gaagaatctc tcctctctct ctctctctgg aaagacccaa ctttctcact gctgtcagcc 2700
   aagtcatggt tggtaaccat gtaggttctt gggagggaat gggacagggt gaataaagca 2760
   gggaatattt tagaaccaca agagatcagc agtggcagga cccttaggaa tctagtacaa 2820
   ccttgttgct ttaggtgagt cacactcaga aaatggggct tgccctgggt cacctagctg 2880
   gttaatggca gcattcagaa cttcaagttc tcttgatttc tttgttccca ctgtccccca 2940
   agaaactagt atctctggcc tcctgggggc ccattctgca tgccctcccc acttcccccg 3000
   attcagagac ttagaagcat cagagttgat aaattagatt gaaaccattc cagtaggaaa 3060
   tcaggcagtt ccctgttgaa agttgtctgg ctttttgcac gtgagtatga tccaggattg 3120
   gcctgtgtct ggcccctccg cgagggctac tcagcagtaa gcaggcctta gtacggctgc 3180
   tgcacacttc tcggtctagg tgggagagag tgcataaagg ttctggggct gggcctgagc 3240
   ctaactccct ccactaccgc tatttctcct tggatggcac catccaccct gcaggcttgg 3300
   gaactgtaag ctcaagcttt tgctcatttg tcatttaccc tggtggaggc ttcttgccca 3360
   gatagagatc tgaaattggt gggaagcaag caggggtcgt accttgcttt agagtagatg 3420
   tttggaaaag ctaagtagaa attttttaaa atagaagttg tattttaaac gtgttaggct 3480
   ttataggtga aggacttgtt ttgacttaag tagaagtaat atgctctcat ttctttgtaa 3540
   tctagatttt cctatgttga atgcttaaag caaggcatac ctgtcctagc aggatctgcc 3600
   cacacctcgg ccgtacatct ctgtgagaga ccccctccaa cgattagctt ttgcaacatg 3660
   gtccagtttc tagagggcac tagaactttg tgccagcgct ggcgagatac catggtgcca 3720
   tcctcctttc tatagttcca gtttagccct tccctgcctt taaggagaag agatcttcat 3780
   ggtgacattt gagatgatgg aaactagatc gtctctaatg ttaggtcaag ctatttctct 3840
   ggatctatga ttttaagata gaacaggctg gttctgagtt ctggtgagac ggagaccccg 3900
   gtggcagtgg aaaaatcaga agcacaaagg aaaactcttg gcctctgctt tctcctgtag 3960
   cacaaagagg gagttggctg gatcttttgg tggagtcagg aaagacaggc tgagctttct 4020
   agcctaagtg tggagaagga tagcctcagc cacaaaactc tgtcaggcat tgaatgacaa 4080
   gcatcagtag ctgagtgcac tcctggtgat cctggttttc cttgaccact ggccctgggg 4140
   ccatgggtgc atggactcgt aaagctgcta gggcccctag aaaccacctg ggttcagcct 4200
   ctcccacaat aagagttctt gcccttcata ccctctacct ggatggatgg tctttgggca 4260
   gggaattaat gacagaatga gcagtcccta atggtattgg gcagttggga gtgagtttag 4320
   gcagcttctc tgtaaccgct tctggggcca caccccaaaa ctctacaccc tctgccccag 4380
   aacaattctg cagagtggtg aagttcactt ccccaactct tctccaagtc tagctttcct 4440
   ttccttctgg ctccaaggtg ctctgtgtcc gtctgtgtat gtgtgtgtct gtgcgtctgt 4500
   gtgtttattt ggggaaaagg gcaatcagca atcaactgag gtttcttaat tgtgattata 4560
   ttcctgagaa tgaatgagta ggaaaggaat aaggatgttg ttatgatcgc ctgtggattt 4620
   tgtccattca tctcatgctc tctgaacaga aggtatacct gggaagatac cagctttttc 4680
   tctcagggtt ctgagtcaga ggacaccatc caagaatgtt gttagctttt cagttcccgc 4740
   tctgcattgc ctgaaaaaac tggtatgttg cacgtatgct tttggttgaa attgaacata 4800
   ctctagtgaa tgtgccttaa aaatcaagac actagttcag aggaagcaca taatgtccag 4860
   atctatggcg aacacaggtg ttggaagcca ggactgtgat aatcagaagc tataagagaa 4920
   ctttcggagc ctgcctcctt cagtgacaag tagggcacaa aattctagaa gcagaaggtt 4980
   gttttttttt ttttttctcc attctgaaaa tagcaggaca tttacctctt aaataaactt 5040
   agcatttaga ggtaattcta aatttaacaa gtgacagggt tggtttcaaa gaaaaaggtc 5100
   catgctttgc ttacaatgga gtctgcagtg aggggagatg ctgggatagc catttccatg 5160
   gctctgttat gcaagcacaa atttcatctc ctagatggac ttcctggttt tctcttactg 5220
   cagtaacact ggccttccct tctctaattc cttaccccag ctgcggcatc cctgtgttaa 5280
   ctcaggatgc caagtggccc tcagattaca cttctccaga tagctgaatg agtctgcttt 5340
   cactgtgact gggacctgaa tgacctgcag tcagggccca gagttgggac tctatactac 5400
   cctgggctct ggtctgtagg tttgtagtag ccaccggtaa taagccaagg gctaggctct 5460
   tgtttgagtt tatggccacc tggaattttc agtcatctca tgatacaggc gggaggggca 5520
   gaacagatag attacgacag gtttggtttt taaattttcc aaccaagtgg aaaggcaagt 5580
   tggtcttata gaaagcacta ctgcacttag tagctatgtg attttgagca aaccacataa 5640
   tctctctagg tccattttcc taaccacaag ataaagatgt tacattgtca aagcttgccg 5700
   taggatttgg ggtgaatgaa aattattcct tgctttcatc actaccttta tagctctcat 5760
   cactaccttt atagctcatc actgtgcctt tttttctttc ctaagaaaga catcacatcc 5820
   ctctcctctc ctcctctgtg ctcctgtccc tccctccccc tagcaaggtc caggcaaagc 5880
   tggagatgag gctgagatcc agagtttcct agaacgcaac ttaggatggc taggaaaggg 5940
   aagcctgact gctcggtcag gagggtgcag tatctcttgc tgggaacaca gccagttttc 6000
   acaatgccta gactgtgtat gtctatttgc acaagattgt cttttcctat tttggagtgg 6060
   tcagacattt tatttttgtt caagattatc tggcgtttta gacaaatttg caaaactgtg 6120
   cttttattga ctttttgaat aaactttggt attctggaaa aaaaaaaaaa aaaaa 6175
<210> 9
   <211> 6432
   <212> DNA
   <213> homo sapiens
<220>
   <221> misc_feature
   <223> Mutated Tuba
<400> 9
   cttgaggatt cagtctggtc ctgaagggtt tggggggaga ctgcgaccag aagatgtttc 60
   catgtcctaa ttaatgggtg atggtggttg ttagtctgac tgttgccacg gtgatgaagg 120
   gagacatcca agtgctggtt tcagtactga ggcgaataca gggaatttca acaggctcca 180
   ggtcttacta tgcagcctga agtgggacca tcccttaaac ccactccatc ctgtggccac 240
   gatgggggcc aggacacctt tgccttcttt ctgggtttct ttctttgccg agacagggat 300
   tttgttccca ggaggcactc cctggcccat gggatctcag cattcaaagc agcacaggaa 360
   acctgggccc ctgaaacggg gccaccgaag agatcggaga acaaccagga ggaagtactg 420
   gaaggaagga agggagatcg ctcgtgtctt agatgatgag ggcagaaacc tgaggcagca 480
   gaagcttgat cggcagcggg ccctgctgga gcagaagcag aagaagaagc gccaggagcc 540
   cctgatggtg caggccaatg cagatgggcg gccccggagc cggcgggccc ggcagtcaga 600
   ggaacaagcc cccctggtgg agtcctacct cagcagcagt ggcagcacca gctaccaagt 660
   tcaagaggcc gactcactcg ccagtgtgca gctgggagcc acgcgcccaa cagcaccagc 720
   ttcagccaag agaaccaagg cggcagctac agcagggggc cagggtggcg ccgctaggaa 780
   ggagaagaag ggaaagcaca aaggcaccag cgggccagca gcactggcag aagacaagtc 840
   tgaggcccaa ggcccagtgc agattctgac tgtgggccag tcagaccacg cccaggacgc 900
   aggggagacg gcagctggtg gggcgaacgg gcccagcggg caggatctcc gtgccacgat 960
   gcagaggaag ggcatctcca gcagcatgag ctttgacgag gatgaggagg atgaggagga 1020
   gaatagctcc agctcctccc agctaaatag taacacccgc cccagctctc ctactagcag 1080
   gaagtccgtc agggaggcag cctcagcccc tagcccaaca gctccagagc aaccagtgga 1140
   cgttgaggtc caggatcttg aggagtttgc actgaggccg gccccccagg gtatcaccat 1200
   caaatgccgc atcactcggg acaagaaagg gatggaccgg ggcatgtacc ccacctactt 1260
   tctgcacctg gaccgtgagg atgggaagaa ggtgttcctc ctggcgggaa ggaagagaaa 1320
   gaagagtaaa acttccaatt acctcatctc tgtggaccca acagacttgt ctcgaggagg 1380
   ggacagctat atcgggaaac tgcggtccaa cttgatgggc accaagttca ctgtttatga 1440
   caatggagtc aaccctcaga aggcctcatc ctccactttg gaaagtggaa ccttacgtca 1500
   ggagctggca gctgtgtgct acgagacaaa cgtcttaggc ttcaaggggc ctcggaagat 1560
   gagcgtgatt gtcccaggca tgaacatggt tcatgagaga gtctctatcc gcccccgcaa 1620
   cgagcatgag acactgctag cacgctggca gaataagaac acggagagta tcatcgagct 1680
   gcaaaacaag acacctgtct ggaatgatga cacacagtcc tatgtactca acttccatgg 1740
   gcgcgtcaca caggcctccg tgaagaactt ccagatcatc catggcaatg acccggacta 1800
   catcgtgatg cagtttggcc gggtagcaga ggatgtgttc accatggatt acaactaccc 1860
   gctgtgtgca ctgcaggcct ttgccattgc cctgtccagc ttcgacagca agctggcgtg 1920
   cgagtagagg cctcttcgtg ccctttgggg ttgcccagcc tggagcggag cttgcctgcc 1980
   tgcctgtgga gacagccctg cctatcctct gtatataggc cttccgccag atgaagcttt 2040
   ggccctcagt gggctccctg gcccagccag ccaggaactg gctcctttgc ctctgctact 2100
   gaggcagggg agtagtggag agcgggtggg tgggtgtgaa gggatgagaa taattctttc 2160
   catgccacga gatcaacaca cactcccacc cttggggtag tagtgtgttg tagtcgtact 2220
   taccaagctg agcaacctct tcagctggga aggccgcaag aggcatagag ggagaggaag 2280
   cacactgcag ggctgctgtg gcccagtcgt ccgctcagcc aaggagtcag atggcaatgg 2340
   gtactccagc aggtaggggc acagtgaatg tgtgtatgta tgaaggccac atcaacttta 2400
   tgtagcaaag ggcttggtgg ccaagcctgg cccttaaaca actgcagaaa gcccttcaac 2460
   ttcagaaggc ctcactcaag cctgagagaa gttgggaggg tggtggggac aggtaagtgg 2520
   caggaccctg tcaggattgc aggtgcctgg cttgctgtgg ctatgggaat cagctggtgg 2580
   ctaggtttct agcgcatttg atttctccag gtttgctgtg tctcacagag gcagtaggaa 2640
   cccagctctc agggctgtct tggtggatgg gccctgcaag acacaggctc agcatgcaga 2700
   agtgcatgaa cagggtccct ggatcagggt tgttctggga gtcctgtcag cttccccagg 2760
   agctctctgc tgagcagccc agcacaaccc ccaggaaaca caaatggggt ccaggtcacc 2820
   agcctgactg cacacagcta ggcatgcctg ggaatcctgc tgccagagaa ccattcccaa 2880
   gccatggcat gctccttgaa gaatctctcc tctctctctc tctctggaaa gacccaactt 2940
   tctcactgct gtcagccaag tcatggttgg taaccatgta ggttcttggg agggaatggg 3000
   acagggtgaa taaagcaggg aatattttag aaccacaaga gatcagcagt ggcaggaccc 3060
   ttaggaatct agtacaacct tgttgcttta ggtgagtcac actcagaaaa tggggcttgc 3120
   cctgggtcac ctagctggtt aatggcagca ttcagaactt caagttctct tgatttcttt 3180
   gttcccactg tcccccaaga aactagtatc tctggcctcc tgggggccca ttctgcatgc 3240
   cctccccact tcccccgatt cagagactta gaagcatcag agttgataaa ttagattgaa 3300
   accattccag taggaaatca ggcagttccc tgttgaaagt tgtctggctt tttgcacgtg 3360
   agtatgatcc aggattggcc tgtgtctggc ccctccgcga gggctactca gcagtaagca 3420
   ggccttagta cggctgctgc acacttctcg gtctaggtgg gagagagtgc ataaaggttc 3480
   tggggctggg cctgagccta actccctcca ctaccgctat ttctccttgg atggcaccat 3540
   ccaccctgca ggcttgggaa ctgtaagctc aagcttttgc tcatttgtca tttaccctgg 3600
   tggaggcttc ttgcccagat agagatctga aattggtggg aagcaagcag gggtcgtacc 3660
   ttgctttaga gtagatgttt ggaaaagcta agtagaaatt ttttaaaata gaagttgtat 3720
   tttaaacgtg ttaggcttta taggtgaagg acttgttttg acttaagtag aagtaatatg 3780
   ctctcatttc tttgtaatct agattttcct atgttgaatg cttaaagcaa ggcatacctg 3840
   tcctagcagg atctgcccac acctcggccg tacatctctg tgagagaccc cctccaacga 3900
   ttagcttttg caacatggtc cagtttctag agggcactag aactttgtgc cagcgctggc 3960
   gagataccat ggtgccatcc tcctttctat agttccagtt tagcccttcc ctgcctttaa 4020
   ggagaagaga tcttcatggt gacatttgag atgatggaaa ctagatcgtc tctaatgtta 4080
   ggtcaagcta tttctctgga tctatgattt taagatagaa caggctggtt ctgagttctg 4140
   gtgagacgga gaccccggtg gcagtggaaa aatcagaagc acaaaggaaa actcttggcc 4200
   tctgctttct cctgtagcac aaagagggag ttggctggat cttttggtgg agtcaggaaa 4260
   gacaggctga gctttctagc ctaagtgtgg agaaggatag cctcagccac aaaactctgt 4320
   caggcattga atgacaagca tcagtagctg agtgcactcc tggtgatcct ggttttcctt 4380
   gaccactggc cctggggcca tgggtgcatg gactcgtaaa gctgctaggg cccctagaaa 4440
   ccacctgggt tcagcctctc ccacaataag agttcttgcc cttcataccc tctacctgga 4500
   tggatggtct ttgggcaggg aattaatgac agaatgagca gtccctaatg gtattgggca 4560
   gttgggagtg agtttaggca gcttctctgt aaccgcttct ggggccacac cccaaaactc 4620
   tacaccctct gccccagaac aattctgcag agtggtgaag ttcacttccc caactcttct 4680
   ccaagtctag ctttcctttc cttctggctc caaggtgctc tgtgtccgtc tgtgtatgtg 4740
   tgtgtctgtg cgtctgtgtg tttatttggg gaaaagggca atcagcaatc aactgaggtt 4800
   tcttaattgt gattatattc ctgagaatga atgagtagga aaggaataag gatgttgtta 4860
   tgatcgcctg tggattttgt ccattcatct catgctctct gaacagaagg tatacctggg 4920
   aagataccag ctttttctct cagggttctg agtcagagga caccatccaa gaatgttgtt 4980
   agcttttcag ttcccgctct gcattgcctg aaaaaactgg tatgttgcac gtatgctttt 5040
   ggttgaaatt gaacatactc tagtgaatgt gccttaaaaa tcaagacact agttcagagg 5100
   aagcacataa tgtccagatc tatggcgaac acaggtgttg gaagccagga ctgtgataat 5160
   cagaagctat aagagaactt tcggagcctg cctccttcag tgacaagtag ggcacaaaat 5220
   tctagaagca gaaggttgtt tttttttttt tttctccatt ctgaaaatag caggacattt 5280
   acctcttaaa taaacttagc atttagaggt aattctaaat ttaacaagtg acagggttgg 5340
   tttcaaagaa aaaggtccat gctttgctta caatggagtc tgcagtgagg ggagatgctg 5400
   ggatagccat ttccatggct ctgttatgca agcacaaatt tcatctccta gatggacttc 5460
   ctggttttct cttactgcag taacactggc cttcccttct ctaattcctt accccagctg 5520
   cggcatccct gtgttaactc aggatgccaa gtggccctca gattacactt ctccagatag 5580
   ctgaatgagt ctgctttcac tgtgactggg acctgaatga cctgcagtca gggcccagag 5640
   ttgggactct atactaccct gggctctggt ctgtaggttt gtagtagcca ccggtaataa 5700
   gccaagggct aggctcttgt ttgagtttat ggccacctgg aattttcagt catctcatga 5760
   tacaggcggg aggggcagaa cagatagatt acgacaggtt tggtttttaa attttccaac 5820
   caagtggaaa ggcaagttgg tcttatagaa agcactactg cacttagtag ctatgtgatt 5880
   ttgagcaaac cacataatct ctctaggtcc attttcctaa ccacaagata aagatgttac 5940
   attgtcaaag cttgccgtag gatttggggt gaatgaaaat tattccttgc tttcatcact 6000
   acctttatag ctctcatcac tacctttata gctcatcact gtgccttttt ttctttccta 6060
   agaaagacat cacatccctc tcctctcctc ctctgtgctc ctgtccctcc ctccccctag 6120
   caaggtccag gcaaagctgg agatgaggct gagatccaga gtttcctaga acgcaactta 6180
   ggatggctag gaaagggaag cctgactgct cggtcaggag ggtgcagtat ctcttgctgg 6240
   gaacacagcc agttttcaca atgcctagac tgtgtatgtc tatttgcaca agattgtctt 6300
   ttcctatttt ggagtggtca gacattttat ttttgttcaa gattatctgg cgttttagac 6360
   aaatttgcaa aactgtgctt ttattgactt tttgaataaa ctttggtatt ctggaaaaaa 6420
   aaaaaaaaaa aa 6432
<210> 10
   <211> 6175
   <212> DNA
   <213> homo sapiens
<220>
   <221> misc_feature
   <223> Mutated Tubb
<400> 10
   tggcgtgcag cgcgggcctc ggcggggccc agcgccccgg cccgggagga tgcggcccgg 60
   ggcggcccgg gagctgagca gggcccccgc gccggcccct ccgggccccg gcctccagag 120
   ccgcagccac cgccccgccc ccgagagaca tgacttccaa gccgcattcc gactggattc 180
   cctacagtgt cttagatgat gagggcagaa acctgaggca gcagaagctt gatcggcagc 240
   gggccctgct ggagcagaag cagaagaaga agcgccagga gcccctgatg gtgcaggcca 300
   atgcagatgg gcggccccgg agccggcggg cccggcagtc agaggaacaa gcccccctgg 360
   tggagtccta cctcagcagc agtggcagca ccagctacca agttcaagag gccgactcac 420
   tcgccagtgt gcagctggga gccacgcgcc caacagcacc agcttcagcc aagagaacca 480
   aggcggcagc tacagcaggg ggccagggtg gcgccgctag gaaggagaag aagggaaagc 540
   acaaaggcac cagcgggcca gcagcactgg cagaagacaa gtctgaggcc caaggcccag 600
   tgcagattct gactgtgggc cagtcagacc acgcccagga cgcaggggag acggcagctg 660
   gtgggggcga acggcccagc gggcaggatc tccgtgccac gatgcagagg aagggcatct 720
   ccagcagcat gagctttgac gaggatgagg aggatgagga ggagaatagc tccagctcct 780
   cccagctaaa tagtaacacc cgccccagct ctcctactag caggaagtcc gtcagggagg 840
   cagcctcagc ccctagccca acagctccag agcaaccagt ggacgttgag gtccaggatc 900
   ttgaggagtt tgcactgagg ccggcccccc agggtatcac catcaaatgc cgcatcactc 960
   gggacaagaa agggatggac cggggcatgt accccaccta ctttctgcac ctggaccgtg 1020
   aggatgggaa gaaggtgttc ctcctggcgg gaaggaagag aaagaagagt aaaacttcca 1080
   attacctcat ctctgtggac ccaacagact tgtctcgagg aggggacagc tatatcggga 1140
   aactgcggtc caacttgatg ggcaccaagt tcactgttta tgacaatgga gtcaaccctc 1200
   agaaggcctc atcctccact ttggaaagtg gaaccttacg tcaggagctg gcagctgtgt 1260
   gctacgagac aaacgtctta ggcttcaagg ggcctcggaa gatgagcgtg attgtcccag 1320
   gcatgaacat ggttcatgag agagtctcta tccgcccccg caacgagcat gagacactgc 1380
   tagcacgctg gcagaataag aacacggaga gtatcatcga gctgcaaaac aagacacctg 1440
   tctggaatga tgacacacag tcctatgtac tcaacttcca tgggcgcgtc acacaggcct 1500
   ccgtgaagaa cttccagatc atccatggca atgacccgga ctacatcgtg atgcagtttg 1560
   gccgggtagc agaggatgtg ttcaccatgg attacaacta cccgctgtgt gcactgcagg 1620
   cctttgccat tgccctgtcc agcttcgaca gcaagctggc gtgcgagtag aggcctcttc 1680
   gtgccctttg ggttgcccca gcctggagcg gagcttgcct gcctgcctgt ggagacagcc 1740
   ctgcctatcc tctgtatata ggccttccgc cagatgaagc tttggccctc agtgggctcc 1800
   ctggcccagc cagccaggaa ctggctcctt tgcctctgct actgaggcag gggagtagtg 1860
   gagagcgggt gggtgggtgt gaagggatga gaataattct ttccatgcca cgagatcaac 1920
   acacactccc acccttgggg tagtagtgtg ttgtagtcgt acttaccaag ctgagcaacc 1980
   tcttcagctg ggaaggccgc aagaggcata gagggagagg aagcacactg cagggctgct 2040
   gtggcccagt cgtccgctca gccaaggagt cagatggcaa tgggtactcc agcaggtagg 2100
   ggcacagtga atgtgtgtat gtatgaaggc cacatcaact ttatgtagca aagggcttgg 2160
   tggccaagcc tggcccttaa acaactgcag aaagcccttc aacttcagaa ggcctcactc 2220
   aagcctgaga gaagttggga gggtggtggg gacaggtaag tggcaggacc ctgtcaggat 2280
   tgcaggtgcc tggcttgctg tggctatggg aatcagctgg tggctaggtt tctagcgcat 2340
   ttgatttctc caggtttgct gtgtctcaca gaggcagtag gaacccagct ctcagggctg 2400
   tcttggtgga tgggccctgc aagacacagg ctcagcatgc agaagtgcat gaacagggtc 2460
   cctggatcag ggttgttctg ggagtcctgt cagcttcccc aggagctctc tgctgagcag 2520
   cccagcacaa cccccaggaa acacaaatgg ggtccaggtc accagcctga ctgcacacag 2580
   ctaggcatgc ctgggaatcc tgctgccaga gaaccattcc caagccatgg catgctcctt 2640
   gaagaatctc tcctctctct ctctctctgg aaagacccaa ctttctcact gctgtcagcc 2700
   aagtcatggt tggtaaccat gtaggttctt gggagggaat gggacagggt gaataaagca 2760
   gggaatattt tagaaccaca agagatcagc agtggcagga cccttaggaa tctagtacaa 2820
   ccttgttgct ttaggtgagt cacactcaga aaatggggct tgccctgggt cacctagctg 2880
   gttaatggca gcattcagaa cttcaagttc tcttgatttc tttgttccca ctgtccccca 2940
   agaaactagt atctctggcc tcctgggggc ccattctgca tgccctcccc acttcccccg 3000
   attcagagac ttagaagcat cagagttgat aaattagatt gaaaccattc cagtaggaaa 3060
   tcaggcagtt ccctgttgaa agttgtctgg ctttttgcac gtgagtatga tccaggattg 3120
   gcctgtgtct ggcccctccg cgagggctac tcagcagtaa gcaggcctta gtacggctgc 3180
   tgcacacttc tcggtctagg tgggagagag tgcataaagg ttctggggct gggcctgagc 3240
   ctaactccct ccactaccgc tatttctcct tggatggcac catccaccct gcaggcttgg 3300
   gaactgtaag ctcaagcttt tgctcatttg tcatttaccc tggtggaggc ttcttgccca 3360
   gatagagatc tgaaattggt gggaagcaag caggggtcgt accttgcttt agagtagatg 3420
   tttggaaaag ctaagtagaa attttttaaa atagaagttg tattttaaac gtgttaggct 3480
   ttataggtga aggacttgtt ttgacttaag tagaagtaat atgctctcat ttctttgtaa 3540
   tctagatttt cctatgttga atgcttaaag caaggcatac ctgtcctagc aggatctgcc 3600
   cacacctcgg ccgtacatct ctgtgagaga ccccctccaa cgattagctt ttgcaacatg 3660
   gtccagtttc tagagggcac tagaactttg tgccagcgct ggcgagatac catggtgcca 3720
   tcctcctttc tatagttcca gtttagccct tccctgcctt taaggagaag agatcttcat 3780
   ggtgacattt gagatgatgg aaactagatc gtctctaatg ttaggtcaag ctatttctct 3840
   ggatctatga ttttaagata gaacaggctg gttctgagtt ctggtgagac ggagaccccg 3900
   gtggcagtgg aaaaatcaga agcacaaagg aaaactcttg gcctctgctt tctcctgtag 3960
   cacaaagagg gagttggctg gatcttttgg tggagtcagg aaagacaggc tgagctttct 4020
   agcctaagtg tggagaagga tagcctcagc cacaaaactc tgtcaggcat tgaatgacaa 4080
   gcatcagtag ctgagtgcac tcctggtgat cctggttttc cttgaccact ggccctgggg 4140
   ccatgggtgc atggactcgt aaagctgcta gggcccctag aaaccacctg ggttcagcct 4200
   ctcccacaat aagagttctt gcccttcata ccctctacct ggatggatgg tctttgggca 4260
   gggaattaat gacagaatga gcagtcccta atggtattgg gcagttggga gtgagtttag 4320
   gcagcttctc tgtaaccgct tctggggcca caccccaaaa ctctacaccc tctgccccag 4380
   aacaattctg cagagtggtg aagttcactt ccccaactct tctccaagtc tagctttcct 4440
   ttccttctgg ctccaaggtg ctctgtgtcc gtctgtgtat gtgtgtgtct gtgcgtctgt 4500
   gtgtttattt ggggaaaagg gcaatcagca atcaactgag gtttcttaat tgtgattata 4560
   ttcctgagaa tgaatgagta ggaaaggaat aaggatgttg ttatgatcgc ctgtggattt 4620
   tgtccattca tctcatgctc tctgaacaga aggtatacct gggaagatac cagctttttc 4680
   tctcagggtt ctgagtcaga ggacaccatc caagaatgtt gttagctttt cagttcccgc 4740
   tctgcattgc ctgaaaaaac tggtatgttg cacgtatgct tttggttgaa attgaacata 4800
   ctctagtgaa tgtgccttaa aaatcaagac actagttcag aggaagcaca taatgtccag 4860
   atctatggcg aacacaggtg ttggaagcca ggactgtgat aatcagaagc tataagagaa 4920
   ctttcggagc ctgcctcctt cagtgacaag tagggcacaa aattctagaa gcagaaggtt 4980
   gttttttttt ttttttctcc attctgaaaa tagcaggaca tttacctctt aaataaactt 5040
   agcatttaga ggtaattcta aatttaacaa gtgacagggt tggtttcaaa gaaaaaggtc 5100
   catgctttgc ttacaatgga gtctgcagtg aggggagatg ctgggatagc catttccatg 5160
   gctctgttat gcaagcacaa atttcatctc ctagatggac ttcctggttt tctcttactg 5220
   cagtaacact ggccttccct tctctaattc cttaccccag ctgcggcatc cctgtgttaa 5280
   ctcaggatgc caagtggccc tcagattaca cttctccaga tagctgaatg agtctgcttt 5340
   cactgtgact gggacctgaa tgacctgcag tcagggccca gagttgggac tctatactac 5400
   cctgggctct ggtctgtagg tttgtagtag ccaccggtaa taagccaagg gctaggctct 5460
   tgtttgagtt tatggccacc tggaattttc agtcatctca tgatacaggc gggaggggca 5520
   gaacagatag attacgacag gtttggtttt taaattttcc aaccaagtgg aaaggcaagt 5580
   tggtcttata gaaagcacta ctgcacttag tagctatgtg attttgagca aaccacataa 5640
   tctctctagg tccattttcc taaccacaag ataaagatgt tacattgtca aagcttgccg 5700
   taggatttgg ggtgaatgaa aattattcct tgctttcatc actaccttta tagctctcat 5760
   cactaccttt atagctcatc actgtgcctt tttttctttc ctaagaaaga catcacatcc 5820
   ctctcctctc ctcctctgtg ctcctgtccc tccctccccc tagcaaggtc caggcaaagc 5880
   tggagatgag gctgagatcc agagtttcct agaacgcaac ttaggatggc taggaaaggg 5940
   aagcctgact gctcggtcag gagggtgcag tatctcttgc tgggaacaca gccagttttc 6000
   acaatgccta gactgtgtat gtctatttgc acaagattgt cttttcctat tttggagtgg 6060
   tcagacattt tatttttgtt caagattatc tggcgtttta gacaaatttg caaaactgtg 6120
   cttttattga ctttttgaat aaactttggt attctggaaa aaaaaaaaaa aaaaa 6175

## Description

The present invention provides methods for diagnosing a new syndrome comprising diabetic retinopathy, massive human obesity, maculopathy and endocochlear deafness, and early type II diabetes mellitus in a human subject. The invention moreover provides methods for selecting an active compound for the prevention and/or the treatment of said syndrome.

The term obesity actually encompasses several complex metabolic diseases that result from the additive and interactive effects of common genetic variants. Multiple frequent gene variants having separately minor effects might be involved in the determinism of different forms of obesity. There are non-syndromic obesities and syndromic obesities. The predisposition to overweight in a given environment could depend upon multiple rare mutations or nucleotide variants in a large set of genes. Some mutations can lead to the development of monogenic human obesity forms with different penetrance and expressivity. In addition, genetic variations in the same distinct genes, including those involving monogenic obesities, could participate to polygenic obesities and their related phenotypes.

Numerous human gene polymorphisms affecting the regulation of food intake and energy homeostasis (Chagnon et al., 1997; Inoue et al., 1997; Karvonen et al., 1998; Mayfield et al., 2001; Wauters et al., 2001) or peripheral regulation of energy expenditure have been reported (Clement et al., 1995; Dionne et al., 2001; Loktionov, 2003; Ristow et al., 1998; Walston et al., 1995; Widen et al., 1995).

Moreover, some mutations also induce very specific syndromes, such as e.g. Prader-Willi, Bardet-Biedl, Cohen, Ayazi, and MOMO. Obese subjects displaying such syndromes are generally referred to as patients affected by syndromic obesity, in contrast to patients which obesity is apparently not linked to other symptoms.

Prader-Willi syndrome (PWS) is a rare genetic disorder in which seven genes on chromosome 15 (q 11-13, including SNRPN and NECDIN) are deleted on the inherited paternal chromosome. This syndrome is characterized by poor muscle tone, low levels of sex hormones and a constant feeling of hunger.

Bardet-Biedl syndrome (BBS) is a ciliopathic human genetic disorder due to mutations in genes BBS1 to BBS17) characterized principally by obesity, retinitis pigmentosa, polydactyly, hypogonadism, arterial hypertension and renal failure in many cases. Most patients affected by Bardet-Biedl syndrome experience progressive retinal degeneration, with night blindness usually appearing by age 9 and legal blindness often occurring by age 15. Of utmost importance, the triallelic mode of inheritance is a feature of the genetic mode of transmission in many families affected by some forms of BBS. This mode of inheritance constitutes a bridge with the multiallelic inheritance or polygenism characterizing most non syndromic obesities.

Cohen syndrome is a rare genetic disorder due to a mutation in the COH1 gene, which main features are obesity, variable mental retardation with occasional seizure and deafness.

Ayazi syndrome is a syndrome characterized by choroideremia (a recessive retinal degenerative disease), congenital deafness and obesity.

MOMO syndrome is a genetic disorder belonging to the overgrowth syndromes which has been diagnosed in six cases around the world only. The name is an acronym of the four primary aspects of the disorder: Macrosomia (excessive birth weight), Obesity, Macrocephaly (excessive head size) and Ocular abnormalities.

While patients affected by syndromic obesity have so far no etiologic therapy available, several existing treatments can improve their obesity as well as other symptoms and their complications. It is thus of particular importance to establish an early diagnosis in each affected patient and to determine the prognosis for each specific form of syndromic obesity, in order to implement as soon as possible the preventive measures and the appropriate treatments. The prognostic tests should be able to evaluate the risk of developing syndromic obesity before the occurrence of the first symptoms.

However,the tests available do not allow to distinguish between all of the syndromic obesities and exclude irrelevant diagnosis. Physicians are thus generally compelled to use differential diagnosis procedures.

A differential diagnosis procedure is a systematic diagnostic method used to identify the presence of a medical condition where multiple alternatives are possible. Such a procedure is essentially a process of elimination or at least of obtaining information that shrinks the "probabilities" of candidate conditions to negligible levels.

However, as far as syndromic obesities as concerned, differential diagnosis procedures may be difficult to apply. Indeed, some symptoms may not be present at the time when the diagnosis is made. As those different syndromes share obesity as one of their many symptoms, there is a risk that they be confused with each other, with mere obesity, or with any other syndrome that may also comprise obesity.

There is thus still a need for methods for diagnosing syndromic obesity, and more particularly for diagnosing the new syndrome comprising massive human obesity, maculopathy and endocochlear deafness.

**DESCRIPTION** The present invention relates to new methods and tools for diagnosing or assessing the risk for a subject of developing specific syndromic forms of obesity.

The inventors have discovered a novel syndromic form of obesity. This novel human syndrome associates early massive morbid obesity, diabetes mellitus and early diabetic retinopathy. Diabetic retinopathy is a special form of maculopathy recognized as an occult maculopathy and endo-cochlear deafness.

Surprisingly, the inventors have also discovered that the mutation 97 G->C in exon 7 of the human TUB gene is responsible for this novel syndrome. This mutation, which is a transversion, has been found to be inherited according to an autosomal dominant mode in a unique family.

In humans, the TUB gene (NCBI reference: GENE ID: 7275), normally encodes primarily a protein called Tubby protein homolog isoform a (NCBI reference: NP_003311) of 561 amino-acid residues called TUB 561 and a shorter protein called Tubby protein homolog isoform b (NCBI reference: NP_813977) of 506 amino-acid residues, called TUB 506.

The inventors have discovered that the mutation 97 G->C in exon 7 of the human TUB gene leads to the insertion of a proline instead of an alanine at position 277 of the Tubby protein homolog isoform a, also called TUB 561 (hereafter "A277P-TUB 561"), and to the insertion of a proline instead of an alanine at position 222 of the Tubby protein homolog isoform b, also called TUB 506 (hereafter "A222P-TUB 506"). Each of these two mutated TUBBY proteins isoforms appears to contribute to the pathophysiology of the novel syndrome recently discovered. Thus, the mutation can be detected either from DNA, mRNA or protein samples from a subject.

The new syndrome caused by a specific mutation in the human TUBBY gene belongs to the family of syndromic obesities, and has never been described before. It is a very serious condition with a number of highly debilitating features. It would thus be highly advantageous to be capable of diagnosing early this syndrome, since it would permit the prescription of treatments for alleviating the symptoms, if not for curing the said syndrome. However, since this syndrome has never been identified before, there is thus, to this day, no available specific diagnosis method.

Interestingly, in contrast with the above-mentioned genes SNRPN, NECDIN, BBS1 to BBS12 and COH1, known to be directly involved in syndromic forms of obesity, the TUB gene has never been linked to syndromic obesity. Indeed, while several association studies have found that some SNPs of the TUB gene are associated with obesity, no study has ever disclosed any point mutation in this gene to be responsible for syndromic obesity (Shiri-Sverdlov et al., 2006; Snieder et al., 2008; van Vliet-Ostaptchouk et al., 2008).

Moreover, the transversion 97 G->C in exon 7 of the TUB gene has not been found in humans who are simply obese, indicating that it does not merely correlate with obesity at large, but rather is specific of said new syndrome.

Moreover, subjects displaying diverse forms of known syndromic obesities did not harbor the mutation either, thus further emphasizing the specific correlation of the said mutation with the above-mentioned syndrome. As disclosed in the examples, this specific point mutation in the TUB gene is associated with unexpected features compared to what was known on TUB mutations in both mice and humans.

More precisely, while the A277P mutation has been characterized as recessive in knock-out and splice site mice mutants, it is dominant in humans. Further, the human syndrome associated with the A277P mutation is characterized by an early-onset massive obesity, while it is associated with late-onset, moderate obesity in mice models.

Moreover, a type 2 diabetes Mellitus (T2DM) is susceptible to appear quite early in human patients carrying the A277P mutation, which was not described in the mice models.

Finally, the experimental results obtained by the inventors suggest that the gene MATP1A is not involved in the human syndrome observed in A277P human mutant patients. On the contrary, in mice, this gene has been involved in the "*tubby mouse neurosensory phenotype*". The "*tubby mouse neurosensory phenotype*" is characterized by adult-onset obesity, insulin resistance, and neurosensory deficits. Interestingly, it is possible to diagnose the said new syndrome by detecting the presence of the transversion 97 G->C in exon 7 of the TUB gene. This method of diagnosis is highly specific, and thus can be used to detect humans at risk of developing a syndrome comprising massive human obesity, maculopathy and endocochlear deafness. The method of the invention can be used as a differential diagnosis help for physicians, since it enables a clear distinction between subjects that are at risk of developing a mere obesity, and subjects that are at risk of developing a syndrome comprising massive human obesity, maculopathy and endocochlear deafness. The method is highly specific of the newly discovered syndrome, and therefore enables distinguishing this syndrome from other forms of syndromic obesities.

In a first aspect, the invention thus provides a method for diagnosing a syndrome characterized by a group of features, said features comprising human obesity, maculopathy and endocochlear deafness.

In a first embodiment, the invention thus provides a method for diagnosing in a subject a syndrome characterized by a group of features, said features comprising diabetic retinopathy, massive human obesity, maculopathy and endocochlear deafness, and early type II diabetes mellitus, said method comprising a step of detecting the transversion 97 G->C in exon 7 in the TUB gene, in a biological sample of the said subject.

Another object of the invention is a method for assessing the risk for a subject of developing a syndrome comprising diabetic retinopathy, massive human obesity, maculopathy and endocochlear deafness, and early type II diabetes mellitus for a human subject, said method comprising a step of detecting the transversion 97 G->C in exon 7 of the TUB gene, in a biological sample from the subject.

As used hereafter, "diagnosing" a syndrome in a subject means identifying or detecting the presence of the said syndrome in the said subject. Thus, in the context of the invention, a "diagnosis" informs on whether a subject is actually presenting said syndrome at the moment when the diagnosis is made.

On the other hand, "assessing the risk of developing" a syndrome for a subject means predicting that the said subject will develop the said syndrome in the future. Thus, in the context of the invention, a "risk assessment" informs whether a subject will develop the said syndrome after the risk assessment is made, i.e. in the future. According to the invention, a positive diagnosis of the syndrome of the invention in a subject means that the transversion 97 G->C in exon 7 of the TUB gene can be actually detected in a biological sample of the said subject.

Likewise, a positive assessment of a risk of developing the said syndrome of the invention in a subject means that the transversion 97 G->C in exon 7 of the TUB gene can be actually detected in a biological sample of the said subject.

In the context of the invention, the terms "a syndrome comprising massive human obesity, maculopathy and endocochlear deafness" are used in the present application to designate an association of medical conditions comprising massive human obesity, maculopathy and endocochlear deafness, wherein said conditions may occur concurrently, separately or successively in said subject.

By "massive human obesity", it is herein referred to a medical condition wherein the subject has accumulated excess body fat to the extent that it has an adverse effect on his or her health. Human subjects are considered massively obese when their body mass index (BMI) exceeds 45 kg/m². The body mass index is the ratio of the person's weight in kilograms by the square of the person's height in meters.

By "maculopathy", it is herein referred to a medical condition wherein the subject presents a pathological condition of the macula. The macula is an area at the center of the retina that is associated with highly sensitive, accurate vision. The term maculopathy encompass any pathological condition of the macula. Yet, in the context of the invention, the inventors have found that the maculopathies developed by the subjects presenting a syndrome comprising massive human obesity, maculopathy and endocochlear deafness especially affect the faveola. The foveola is located within the macula, more particularly within the fovea. The foveola is approximately 0.35 mm in diameter and lies in the center of the fovea. Thus, preferably, the term "maculopathy" refers to any pathological condition of the foveola.

By "endocochlear deafness", it is herein referred to a medical condition wherein the subject is medically considered as deaf due to endocochlear defects. In the context of the invention, an endocochlear defect is any kind of defect affecting the cochlea.

According to the invention, the TUB gene is for example the gene which sequence has at least 90% identity with the sequence SEQ ID No.1 represented by the NCBI reference: GENE ID: 7275. Preferably, the TUB gene according to the invention is the gene of sequence SEQ ID No. 1.

According to the invention, the TUB gene comprising the transversion 97 G-> C in exon 7 is the gene of sequence SEQ ID No.2.

The term "sequence identity" refers to the identity between two peptides or between two nucleic acids. Identity between sequences can be determined by comparing a position in each of the sequences which may be aligned for the purposes of comparison. When a position in the compared sequences is occupied by the same base or amino acid, then the sequences are identical at that position. A degree of sequence identity between nucleic acid sequences is a function of the number of identical nucleotides at positions shared by these sequences. A degree of identity between amino acid sequences is a function of the number of identical amino acid sequences that are shared between these sequences. Since two polypeptides may each (i) comprise a sequence (i.e. a portion of a complete polynucleotide sequence) that is similar between two polynucleotides, and (ii) may further comprise a sequence that is divergent between two polynucleotides, sequence identity comparisons between two or more polynucleotides over a "comparison window" refers to the conceptual segment of at least 20 contiguous nucleotide positions wherein a polynucleotide sequence may be compared to a reference nucleotide sequence of at least 20 contiguous nucleotides and wherein the portion of the polynucleotide sequence in the comparison window may comprise additions or deletions (i.e. gaps) of 20 percent or less compared to the reference sequence (which does not comprise additions or deletions) for optimal alignment of the two sequences.

To determine the percent identity of two amino acids sequences or two nucleic acid sequences, the sequences are aligned for optimal comparison. For example, gaps can be introduced in the sequence of a first amino acid sequence or a first nucleic acid sequence for optimal alignment with the second amino acid sequence or second nucleic acid sequence. The amino acid residues or nucleotides at corresponding amino acid positions or nucleotide positions are then compared. When a position in the first sequence is occupied by the same amino acid residue or nucleotide as the corresponding position in the second sequence, the molecules are identical at that position. The percent identity between the two sequences is a function of the number of identical positions shared by the sequences. Hence % identity = number of identical positions/total number of overlapping positions X 100.

In this comparison the sequences can be the same length or can be different in length. Optimal alignment of sequences for determining a comparison window may be conducted by the local homology algorithm of Smith and Waterman (J. Theor. Biol., 91(2): 370-380, 1981), by the homology alignment algorithm of Needleman and Wunsch (J. Mol. Biol, 48(3): 443-453, 1972), by the search for similarity via the method of Pearson and Lipman (Proc. Natl. Acad. Sci. U.S.A., 85(5): 2444-2448, 1988), by computerized implementations of these algorithms (GAP, BESTFIT, FASTA and TFASTA in the Wisconsin Genetics Software Package Release 7.0, Genetic Computer Group, 575, Science Drive, Madison, Wisconsin) or by inspection. The best alignment (i.e. resulting in the highest percentage of identity over the comparison window) generated by the various methods is selected.

The term "sequence identity" thus means that two polynucleotide or polypeptide sequences are identical (i.e. on a nucleotide by nucleotide or an amino acid by amino acid basis) over the window of comparison. The term "percentage of sequence identity" is calculated by comparing two optimally aligned sequences over the window of comparison, determining the number of positions at which the identical nucleic acid base (e.g. A, T, C, G, U, or I) occurs in both sequences to yield the number of matched positions, dividing the number of matched positions by the total number of positions in the window of comparison (i.e. the window size) and multiplying the result by 100 to yield the percentage of sequence identity. The same process can be applied to polypeptide sequences. The percentage of sequence identity of a nucleic acid sequence or an amino acid sequence can also be calculated using BLAST software (Version 2.06 of September 1998) with the default or user defined parameter.

By "transversion" it is herein referred to the substitution, in a polynucleotide molecule, preferably in a DNA molecule, of a purine for a pyrimidine or of a pyrimidine for a purine.

Detecting the transversion 97 G-> C in exon 7 of the TUB gene can be performed either at the protein level or at the nucleic acid level.

Since the transversion 97 G-> C is found in an exon of the TUB gene, it will be obvious to the skilled person that this mutation will appear both in the DNA of the subject and in mRNA products of said mutated gene.

Thus, in the context of the invention, detecting the transversion 97 G-> C in exon 7 of the TUB gene at the nucleic acid level encompass detecting the mutation in the TUB gene at the DNA level, and/or detecting the mutation in products of the gene at the mRNA level.

In an embodiment, detecting the transversion 97 G-> C in exon 7 of the TUB gene at the nucleic acid level consist in detecting the mutation in the TUB gene at the DNA level.

In another embodiment, detecting the transversion 97 G-> C in exon 7 of the TUB gene at the nucleic acid level consist in detecting the mutation in mRNA products of the gene.

According to the invention, mRNA products of the TUB gene is for example the polynucleotide having the sequence SEQ ID No.7 f NCBI reference: NM_003320.4 or the polynucleotide having the sequence SEQ ID No.8 of NCBI reference: NM_177972.2.

In an embodiment, the mRNA product of the TUB gene is the polynucleotide having at least 90% identity with the sequence SEQ ID No.7. In that case, said mRNA product will be considered, in the sense of the invention, as comprising the transversion 97 G-> C in exon 7 if said polynucleotide has the sequence SEQ ID No.9.

In an embodiment, the mRNA product of the TUB gene is the polynucleotide having the sequence SEQ ID No.8. In that case, said mRNA product will be considered, in the sense of the invention, as comprising the transversion 97 G-> C in exon 7 if said polynucleotide has the sequence SEQ ID No. 10.

In order to detect that TUB gene comprises the transversion 97 G->C in exon 7, the person skilled in the art can perform any technique known in the art and suitable for detecting a point mutation in nucleic acids sequences.

Such techniques are well known to the person skilled in the art and include such methods as mass spectrometry, nucleic acid hybridization, primer extension, and sequencing.

Nucleic acid hybridization techniques include for example *in situ* hybridization (performed on a biological sample), southern blot hybridization, or nucleic microarrays. In an embodiment, the step of detecting that the TUB gene comprises the transversion 97 G->C in exon 7 is performed with a nucleic microarray. Many variants of the microarray hybridization technology are available to the person skilled in the art.

According to the invention, a "nucleic microarray" consists of different nucleic acid probes that are attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes can be nucleic acids such as cDNAs ("cDNA microarray") or oligonucleotides ("oligonucleotide microarray"), and the oligonucleotides may be about 25 to about 60 base pairs or less in length.

In a specific embodiment, the nucleic microarray is an oligonucleotide microarray comprising at least one oligonucleotide specific for a gene having the sequence SEQ ID No.2. Preferably, the microarray of the invention comprises oligonucleotides, consisting in 10 to 80 nucleotides, preferably in 20 to 60 nucleotides, specific for a gene having the sequence SEQ ID No.2. More preferably, oligonucleotides are specific to the transversion 97 G-> C in exon 7 of the sequence SEQ ID No.2.

Primer extension techniques include for example isothermal methods and PCR-based techniques. Isothermal techniques include such methods as e.g. nucleic acid sequence-based amplification (NASBA), loop-mediated isothermal amplification (LAMP), helicase-dependent amplification (HDA), rolling circle amplification (RCA), and strand displacement amplification (SDA), exponential amplification reaction (EXPAR), isothermal and chimeric primer-initiated amplification of nucleic acids (ICANs), signal-mediated amplification of RNA technology (SMART) and others (see e.g. Asiello and Baeumner, Lab Chip; 11(8): 1420-1430, 2011). Examples of PCR-based techniques according to the invention include techniques such as, but not limited to, quantitative PCR (Q-PCR), reverse-transcriptase polymerase chain reaction (RT-PCR), quantitative reverse-transcriptase PCR (QRT-PCR), rolling circle amplification (RCA), digital PCR, Restriction Fragment Length Polymorphism Analysis of PCR-Amplified Fragments (PCR-RFLP) and Polymerase chain reaction single-strand conformation polymorphism (PCR-SSCP), all of which can be optionally coupled with sequence analysis.

These techniques are well known and easily available technologies for those skilled in the art and need not be further detailed. In a preferred embodiment, the step of detecting that the TUB gene comprises the transversion 97 G->C in exon 7 is performed by Restriction Fragment Length Polymorphism Analysis of PCR-Amplified Fragments (PCR-RFLP) or Polymerase chain reaction single-strand conformation polymorphism (PCR-SSCP).

Sequencing is an especially well-suited technique in the context of the invention, as is it particularly sensitive.

Thus, in a preferred embodiment, the detection of the transversion 97 G->C in exon 7 of the TUB gene is performed by sequencing.

As used herein, the term "sequencing" is used in a broad sense and refers to any technique known by the skilled person including but not limited to Sanger dideoxy termination sequencing, whole-genome sequencing, sequencing by hybridization, pyrosequencing, capillary electrophoresis, cycle sequencing, single-base extension sequencing, solid- phase sequencing, high-throughput sequencing, massively parallel signature sequencing (MPSS), sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD(R) sequencing, MS-PET sequencing, mass spectrometry, and combinations thereof.

According to the invention, the mutation to be detected is a point mutation, more precisely a transversion 97 G-> C. The detection of a point mutation in a sample requires techniques which are both specific and sensitive.

Advantageously, in the context of the invention, the precision of the detection can be enhanced by the use of massively parallel DNA sequencing. In one embodiment of this approach, the precision is achieved by analysis of a large number (for example, millions or billions) of nucleic acid molecules without any amplification using protocols that relies on previous knowledge of the target sequences. One embodiment uses massively parallel DNA sequencing, such as, but not limited to that performed by the Illumina Genome Analyzer platform (Bentley et al. Nature; 456: 53-59, 2008), the Roche 454 platform (Margulies et al. Nature; 437: 376-380, 2005), the ABI SOLiD platform (McKernan et al., Genome Res; 19: 1527-1541, 2009), the Helicos single molecule sequencing platform (Harris et al. Science; 320: 106-109, 2008), real-time sequencing using single polymerase molecules (Science; 323: 133-138, 2009), Ion Torrent sequencing (WO 2010/008480; Rothberg et al., Nature, 475: 348-352, 2011) and nanopore sequencing (Clarke J et al. Nat Nanotechnol.; 4: 265-270, 2009). In one embodiment, massively parallel sequencing is performed on a random subset of nucleic acid molecules in the biological sample.

In specific embodiments, the method and kit of the invention is adapted to run on ABI PRISM(R) 377 DNA Sequencer, an ABI PRISM(R) 310, 3100, 3100-Avant, 3730, or 3730x1 Genetic Analyzer, an ABI PRISM(R) 3700 DNA Analyzer, or an Applied Biosystems SOLiD(TM) System (all from Applied Biosystems), a Genome Sequencer 20 System (Roche Applied Science), an HiSeq 2500, an HiSeq 2000, a Genome Analyzer llx, a MiSeq Personal Sequencer, a HiScanSQ (all from Illumina), the Genetic Analysis System, including the Single Molecule Sequencer, Analysis Engine and Sample Loader (all from HeliScope), the Ion Proton^{™} Sequencer, or the Ion PGM^{™} Sequencer (both from Ion Torrent). In a preferred embodiment of the invention, the sequences corresponding to the TUB gene are identified in the global sequencing data by comparison with the publicly-deposited TUB sequences, such as the sequence of NCBI reference: GENE ID: 7275.

Alternatively, the presence of transversion 97 G->C in exon 7 of TUB can be detected at the protein level.

The inventors have found that the transversion 97 G-> C in exon 7 of the TUB gene leads to an insertion of a proline instead of an alanine at residue 277 of the Tubby protein homologs.

The skilled man trying to detect this modification may thus decide to detect it at the protein level, by detecting at least one of the mutated Tubby protein homologs "Tubby A277P", that is to say that at least one of the Tubby protein homologs that comprises the substitution A277P.

According to the invention, the Tubby protein homologs comprise proteins which sequence has at least 90% identity with that of the protein "A277P-TUB 561" of sequence SEQ ID No.3 represented by NCBI reference: NP_003311, and/or at least 90% identity with that of the protein "A222P-TUB 506" of sequence SEQ ID No.5 represented by NCBI reference: NP_813977. Thus, in an embodiment, the step of detecting that the TUB gene comprises the transversion 97 G->C in exon 7 comprises the detection that at least one of the Tubby protein homologs comprises the substitution A277P.

When the sequence of the Tubby protein homolog has at least 90% identity with that of the protein "A277P-TUB 561" of sequence SEQ ID No.3, it will be considered, in the sense of the invention, as comprising the substitution A277P if said protein has the sequence SEQ ID No.4.

When the sequence of the Tubby protein homolog has at least 90% identity with that of the protein "A222P-TUB 506" of sequence SEQ ID No.5, it will be considered, in the sense of the invention, as comprising the substitution A277P if said protein has the sequence SEQ ID No.6.

In order to identify that the A277P change in at least one of the Tubby protein homologs, the skilled person may in particular use any technique known in the field suitable for detecting a change in the amino-acid sequence of a protein. Advantageously, the said A277P mutation in the Tubby protein homolog is detected by protein sequencing. Protein sequencing may be classically performed via automated Edman degradation (Biochemistry. 5th edition, Berg JM, Tymoczko JL, Stryer L, New York: W H Freeman; 2002), or for example by mass spectrometry, such as for example particularly electron transfer dissociation mass spectrometry (Syka et al., Proc Natl Acad Sci; 101(26): 9528-9533; 2004).

Alternatively, the change in the amino acid sequence of the Tubby protein homolog may be detected without actually sequencing the said protein, on the basis of e.g., physicochemical properties of the mutated protein. Such properties include e.g., the molecular weight or the isoelectric point of the protein. Variations in these properties caused by the change in the protein sequence can be detected by the usual methods for analyzing the proteins structure and properties. Among other techniques that can be used are included FRET or BRET, methods of microscopy or histochemistry, including methods of confocal microscopy and electron microscopy, methods based on the use of one or more wavelengths excitation and an optical method adapted, as an electrochemical method (the technical voltammetry and AMPEROMETRY), atomic force microscopy, methods of radiofrequency, multipolar resonance spectroscopy, confocal and non-confocal, fluorescence detection, luminescence, chemiluminescence, absorbance, reflectance, transmittance, and birefringence or index of refraction (for example, by Surface plasmon resonance, by Ellipsometry, etc), flow cytometry, radioisotopic or magnetic resonance imaging, analysis by polyacrylamide gel electrophoresis (SDS-PAGE); by spectrophotometry HPLC-Mass, by liquid chromatography / mass spectrophotometry / mass spectrometry (LC -MS/MS). All these techniques are well known to the skilled and do not require to be further detailed herein.

It is advantageous to use immunoassays, wherein the mutated Tubby protein homolog is recognized by antibodies which do not bind the wild-type protein. Immunoassays are well known technologies and include such methods as e.g., immunoprecipitation, immunohistology, including immunochemistry, western blot, dot blot, ELISA, protein chips.

In a preferred embodiment, the detection of the A277P mutation in the Tubby protein homolog involves at least one immunological technique. Preferably, said immunological technique comprises the use of at least one antibody directed against the Tubby protein homolog containing the A277P substitution.

By "antibody directed against an antigen" it is herein referred to any protein molecule, preferentially any immunoglobin molecule, which binds to an antigen with the required selectivity. In other words, the antibody of the invention binds selectively to the desired Tubby protein mutant, but not to the other Tubby protein variants.

By "binds selectively" it is herein referred to the ability of antibodies to preferentially bind to a first antigen rather than to a second antigen. In an embodiment, the antibody directed against the Tubby protein homolog comprising the substitution A277P preferentially binds to the Tubby protein homolog comprising the substitution A277P of the sequence SEQ ID no.4 rather than to the Tubby protein homolog of the sequence SEQ ID no.3.

In another embodiment, the antibody directed against the Tubby protein homolog comprising the substitution A277P preferentially binds to the Tubby protein homolog comprising the substitution A222P of the sequence SEQ ID no.6 rather than to the Tubby protein homolog of the sequence SEQ ID no.5.

The selective character of such a liaison is classically called the binding affinity, commonly expressed by the association constant. The binding affinity can be measured using a variety of techniques known of the person skilled in art including immunoblots methods, immunoprecipitation, radioimmunological tests, immunoenzymatic tests (e.g., ELISA), antibody titration by immunofluorescence and microscopy.

The term "antibody" as used herein is intended to include polyclonal and monoclonal antibodies. An antibody (or "immunoglobulin") consists of a glycoprotein comprising at least two heavy (H) chains and two light (L) chains inter-connected by disulfide bonds. Each heavy chain comprises a heavy chain variable region (or domain) (abbreviated herein as HCVR or VH) and a heavy chain constant region. The heavy chain constant region comprises three domains, CH1, CH2 and CH3. Each light chain comprises a light chain variable region (abbreviated herein as LCVR or VL) and a light chain constant region. The light chain constant region comprises one domain, CL. The VH and VL regions can be further subdivided into regions of hypervariability, termed "complementarity determining regions" (CDR) or "hypervariable regions", which are primarily responsible for binding an epitope of an antigen, and which are interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g. effector cells) and the first component (Clq) of the classical complement system. Antibodies can be of different isotypes (namely IgA, IgD, IgE, IgG or IgM).

Antibody fragments can also be used for detecting Tubby A277P in the present methods, provided they retain the binding specificity of the parent antibody. Antibody fragments according to the invention include Fab, Fab', F(ab')2, scFv, dsFv, ds-scFv, dimers, minibodies, diabodies, and multimers thereof and bispecific antibody fragments.

Polyclonal antibodies are generated from different B cell sources. As such, they recognize different epitopes exhibited by a pathogenic antigen (on the other hand, monoclonal antibodies are derived from a single cell line and recognize the same epitope).

Preferably, monoclonal antibodies are used in the present immunoassays. As used herein, "monoclonal antibody" defines an antibody arising from a homogeneous antibody population. More particularly, the individual antibodies of a population are identical except for a few possible naturally-occurring mutations which can be found in minimal proportions. In other words, a monoclonal antibody consists of a homogeneous antibody arising from the growth of a single cell clone (for example a hybridoma, a eukaryotic host cell transfected with a DNA molecule coding for the homogeneous antibody, a prokaryotic host cell transfected with a DNA molecule coding for the homogeneous antibody, etc.) and is generally characterized by heavy chains of one and only one class and subclass, and light chains of only one type. Monoclonal antibodies are highly specific and are directed against a single antigen. In addition, in contrast with preparations of polyclonal antibodies which typically include various antibodies directed against various determinants, or epitopes, each monoclonal antibody is directed against a single epitope of the antigen.

Techniques to produce antibodies, well as conventional immunological tests mentioned above, are described in particular in Harlow, E. and Lane, D, "Antibodies: A Laboratory Manual», (1988), Cold Spring Harbor Laboratory Press. These techniques are well known of the person skilled in the art and do not require be described in detail here.

The above methods are performed using a biological sample of the patient to be tested. In some cases, the methods according to the invention may further comprise a preliminary step of taking a biological sample from the patient.

By "biological sample", it is herein referred to any sample that is taken from a subject, which includes but is not limited to, for example, epithelium tissue, blood, serum, plasma, sputum, urine, stool, skin, cerebrospinal fluid, saliva, gastric secretions, semen, seminal fluid, tears, spinal tissue or fluid, cerebral fluid, trigeminal ganglion sample, a sacral ganglion sample, adipose tissue, lymphoid tissue, placental tissue, upper reproductive tract tissue, gastrointestinal tract tissue, male genital tissue and fetal central nervous system tissue. In a preferred embodiment, the biological sample is epithelium tissue, preferably oral epithelium tissue. In another preferred embodiment, the biological sample is saliva.

In addition, the methods according to the invention may comprise another preliminary step, between the taking of the sample from the patient and the detection step as defined above, corresponding to the transformation of the biological sample into a nucleic acid sample, preferably a DNA sample, or into a protein sample, which is then ready to use for *in vitro* detection as described above. Preparation or extraction of nucleic acid, in particular of DNA, or proteins from a biological sample is only routine procedure well known to those skilled in the art.

The TUB gene variant which contains a transversion 97 G->C in exon 7 encodes a Tubby protein homolog containing the A277P mutation and is associated with a syndrome characterized by a group of features, said features comprising human obesity, maculopathy and endocochlear deafness.

It has been shown that Tubby protein homolog acts as a transcription regulator which is membrane bound and translocates to the nucleus in response to phosphoinositide hydrolysis. The Tubby protein homolog actually binds to the human Gαq protein. In response to G-protein coupled receptor (GPCR) activation, Gαq recruits phospholipase C-B (PLC-B), resulting in the hydrolysis of PI-containing lipids and the subsequent release of the Tubby protein homolog from the plasma membrane, and accumulation in the nucleus.

The inventors have however discovered that the substitution A277P impairs normal functioning of the Tubby protein homolog. Specifically, the Tubby protein homolog comprising the substitution A277P has a reduced affinity for the human protein Gαq and shows a different cellular localization than the wild type (unmutated) Tubby protein homolog. Indeed, as the wild type Tubby protein homolog tends to accumulate in the nucleus, and has a clear nuclear localization, the Tubby protein homolog comprising the A277P mutation does not.

The loss of function due to the A277P substitution may thus be responsible for the syndrome comprising massive human obesity, maculopathy and endocochlear deafness observed in human subjects that harbor the mutation. As a consequence, active compounds that modulate the affinity of Tubby protein homolog for Gαq and/or the localization of Tubby protein homolog would thus be useful in the prevention or the treatment of the new syndrome. In particular, it is expected that a compound capable of restoring normal Tubby protein homolog functions would be particularly useful for treating or preventing this syndrome. The term "treatment," as used herein, refers to the measures taken for treating a symptom, disorder, or condition. The term "treating" refers to alleviating the symptom, disorder, or condition to which the term "treating" applies.The term "prevention," as used herein, refers to the measures taken in view of preventing a symptom, disorder, or condition. The term "preventing" refers to avoiding the symptom, disorder, or condition to which the term "preventing" applies.

In another aspect, the present invention thus provides a method for identifying a compound which can be used in preventing and/or treating the new syndrome of the invention. The said method comprises the step of identifying a compound capable of restoring at least one of the functions of Tubby protein homolog.

In a preferred embodiment, the invention relates to a method for selecting a compound useful for the prevention and/or the treatment of a syndrome characterized by a group of features, said features comprising diabetic retinopathy, massive human obesity, maculopathy and endocochlear deafness, and early type II diabetes mellitus in a human subject, the said method comprising the steps of:
a) contacting a candidate compound with the human Tubby protein homolog comprising the substitution A277P of sequence SEQ ID No.4 and/or with the human Tubby protein homolog comprising the substitution A222P of sequence SEQ ID No.6;
b) assaying at least one of the functions of the human Tubby protein homolog comprising the substitution A277P of sequence SEQ ID No.4 and/or of the human Tubby protein homolog comprising the substitution A222P of sequence SEQ ID No.6 in presence of the compound of step a);
c) comparing the result obtained from step b) with a reference result; and
d) determining that the candidate compound of step a) is an active compound for the prevention and/or the treatment of a syndrome comprising diabetic retinopathy, massive human obesity, maculopathy and endocochlear r deafness, and early type II diabetes mellitus in a human subject according to the result of the comparison of step c).

The "functions" of the human Tubby protein homolog comprise the binding of the Tubby protein homolog to human Gαq protein and the nuclear localization of said Tubby protein homolog.

In a first embodiment, step b) of the method involves determining the binding of the said human Tubby protein homolog comprising the A277P substitution to human Gαq protein.

By "human Gαq protein", also called human Gq protein or human Gq/11 protein, it is herein referred to the protein encoded by the gene GNAQ (NCBI reference: Gene ID: 2776), of sequence the sequence of NCBI reference NP_002063.2.

Preferably, step b) of the method involves determining the binding affinity of the Tubby protein homolog comprising the substitution A277P of sequence SEQ ID No.4 for the human protein Gαq.

"Binding affinity" generally refers to the strength of the sum total of non-covalent interactions between a single binding site of a molecule and its binding partner.

Unless indicated otherwise, as used herein, "binding affinity" refers to intrinsic binding affinity that reflects a 1: 1 interaction between members of a binding pair (e.g., antibody and antigen). The affinity of a molecule X for its partner Y can generally be represented by the K_{D}. As used herein, the term "K_{D}" refers to the dissociation constant of a particular antibody/antigen interaction.

The binding affinity can be measured using a variety of methods known to those skilled in the art including such methods as e.g., cell imaging, immunoprecipitation assays, radioimmuno assays, ELISAs, alpha screen, chemical cross-linking, two hybrid, affinity chromatography, Far-western blot or surface plasmon resonance (BiaCORE). In particular, cell imaging methods according to the invention include such techniques as e.g. FRAP, FLIP, FRET, BRET, FLIM, PRIM, PRIM, pHluorin, Timer etc (see e.g.Villalobos et al., Annu Rev Biomed Eng., 9: 321-349, 2007). Those techniques are well known to the person skilled in the art and do not need to be further explained herein.

Preferably, the binding is determined by BRET. The technology BRET is a technology known as being representative of the protein dimerization [Angers et al., PNAS, 2000, 97:3684-89].

BRET (Bioluminescence Resonance Energy Transfer) is a non-radiative energy transfer occurring between a bioluminescent donor (Renilla Luciferase (Rluc)) and a fluorescent acceptor, a mutant of GFP (Green Fluorescent Protein) or YFP (Yellow fluorescent protein). In the present case EYFP (Enhanced Yellow Fluorescent Protein) was used. The efficacy of transfer depends on the orientation and the distance between the donor and the acceptor. Then, the energy transfer can occur only if the two molecules are in close proximity (1-10 nm). This property is used to generate protein-protein interaction assays. Indeed, in order to study the interaction between two partners, the first one is genetically fused to the Renilla Luciferase and the second one to the yellow mutant of the GFP. Fusion proteins are generally, but not obligatory, expressed in mammalian cells. In presence of its membrane permeable substrate (coelenterazine), Rluc emits blue light. If the GFP mutant is closer than 10 nm from the Rluc, an energy transfer can occur and an additional yellow signal can be detected. The BRET signal is measured as the ratio between the light emitted by the acceptor and the light emitted by the donor. So the BRET signal will increase as the two fusion proteins are brought into proximity or if a conformational change brings Rluc and GFP mutant closer. BRET is used in routine in the lab and thus needs not be detailed here (see e.g., Hamdan et al., Curr Protoc Neurosci.; Chapter 5:Unit 5.23, 2006).

In another embodiment, step b) of the method involves determining the localization of the said human Tubby protein homolog comprising the A277P substitution. Localization of the said protein can be determined by a variety of techniques, all of which are well known to the person of skills in the art. Localization can be effectively demonstrated with fluorescence microscopy-based techniques, electron microscopy methods, or cellular fractionation procedures. A broad spectrum of fluorescence imaging can be accomplished by using recombinant reporter proteins, (e.g. GFP, CFP, YFP, SNAP-tag®), or fluorescent dyes (e.g. Alexa), or fluorophore-labeled molecules (e.g. antibodies specific for the human Tubby protein homologue). Preferably, the localization of the human Tubby protein homologue A277P of the invention is performed by immunofluorescence with labeled antibodies.

It will be obvious to the person skilled in the art that when a protein is expressed in a cell, it is likely that not all molecules of said protein will actually localize in the same cell compartment. On the other hand, a test compound may also affect the expression of the said protein of the invention. It may thus be convenient, in the context of the invention, to determine the fraction of the Tubby protein homolog comprising the substitution A277P of sequence SEQ ID No.4 and/or of the fraction of the Tubby protein homolog comprising the substitution A222P of sequence SEQ ID No.6, which is localized to the nucleus. The "fraction of nuclear localization" of the Tubby protein homolog A277P of the invention herein refers to the percentage of the amount of said protein localized in the nucleus compartment of the cell compared to the total amount of said protein in all of the cell compartments.

In yet another embodiment, step b) of the method involves determining the binding of the said human Tubby protein homolog comprising the A277P substitution to human Gαq protein and the localization of the said protein.

The results obtained by assaying at least one of the functions of the human Tubby protein homolog comprising the substitution A277P of sequence SEQ ID No.4, and/or of the Tubby protein homolog comprising the substitution A222P of sequence SEQ ID No.6, in presence of the compound are then compared with a reference result.

By "reference result" it is herein referred to a predetermined result, such as for instance a result obtained when the Tubby protein homolog comprising the substitution A277P of sequence SEQ ID No.4 is contacted with a reference compound or a result obtained when the Tubby protein homolog comprising the substitution A277P of sequence SEQ ID No.4 is not contacted with any compound. Another reference result can be for example a result obtained when the Tubby protein homolog comprising the substitution A222P of sequence SEQ ID No.6 is contacted with a reference compound or a result obtained when the Tubby protein homolog comprising the substitution A222P of sequence SEQ ID No.6 is not contacted with any compound.

In a first embodiment, the reference result is a result obtained when the human Tubby protein homologue A277P of the invention is not contacted with any compound. In that particular case, a reference result can be the affinity of the Tubby protein homolog comprising the substitution A277P of sequence SEQ ID No.4 and/or of the Tubby protein homolog comprising the substitution A222P of sequence SEQ ID No.6 for the human protein Gαq when the said protein is not contacted with any compound, or the cellular localization of the Tubby protein homolog comprising the substitution A277P of sequence SEQ ID No.4 and/or of the Tubby protein homolog comprising the substitution A222P of sequence SEQ ID No.6 when the said protein is not contacted with any compound.

Thus, in this embodiment, step c) comprises:
- the comparison of the affinity of the Tubby protein homolog comprising the substitution A277P of sequence SEQ ID No.4 for the human protein Gαq obtained from step b) when the said protein is contacted with said candidate compound with the affinity of the Tubby protein homolog comprising the substitution A277P of sequence SEQ ID No.4 for the human protein Gαq obtained when the said protein is not contacted with any compound, and/or;
- the comparison of the nuclear localization of the Tubby protein homolog comprising the substitution A277P of sequence SEQ ID No.4 when the said protein is contacted with said candidate compound with the nuclear localization of the Tubby protein homolog comprising the substitution A277P of sequence SEQ ID No.4 when the said protein is not contacted with any compound, and/or;
- the comparison of the affinity of the Tubby protein homolog comprising the substitution A222P of sequence SEQ ID No.6 for the human protein Gαq obtained from step b) when the said protein is contacted with said candidate compound with the affinity of the Tubby protein homolog comprising the substitution A222P of sequence SEQ ID No.6 for the human protein Gαq obtained when the said protein is not contacted with any compound, and/or;
- the comparison of the nuclear localization of the Tubby protein homolog comprising the substitution A222P of sequence SEQ ID No.6 when the said protein is contacted with said candidate compound with the nuclear localization of the Tubby protein homolog comprising the substitution A222P of sequence SEQ ID No.6 when the said protein is not contacted with any compound.

In this embodiment, a compound is considered as suitable for the prevention and/or the treatment of a syndrome comprising massive human obesity, maculopathy and endocochlear deafness if:
- the affinity of the Tubby protein homolog comprising the substitution A277P of sequence SEQ ID No.4 for the human protein Gαq obtained from step b) when the said protein is contacted with said candidate compound is superior or equal to the affinity of the Tubby protein homolog comprising the substitution A277P of sequence SEQ ID No.4 for the human protein Gαq obtained when the said protein is not contacted with any compound, or if;
- the nuclear localization of the Tubby protein homolog comprising the substitution A277P of sequence SEQ ID No.4 when the said protein is contacted with said candidate compound is superior or equal to the nuclear localization of the Tubby protein homolog comprising the substitution A277P of sequence SEQ ID No.4 when the said protein is not contacted with any compound, or if;
- the affinity of the Tubby protein homolog comprising the substitution A222P of sequence SEQ ID No.6 for the human protein Gαq obtained from step b) when the said protein is contacted with said candidate compound is superior or equal to the affinity of the Tubby protein homolog comprising the substitution A222P of sequence SEQ ID No.6 for the human protein Gαq obtained when the said protein is not contacted with any compound, or if;
- the nuclear localization of the Tubby protein homolog comprising the substitution A222P of sequence SEQ ID No.6 when the said protein is contacted with said candidate compound is superior or equal to the nuclear localization of the Tubby protein homolog comprising the substitution A222P of sequence SEQ ID No.6 when the said protein is not contacted with any compound, or if;

Alternatively, a reference result may be a result obtained when the protein is a reference human Tubby protein homolog of sequence SEQ ID No.3, and /or a reference human Tubby protein homolog of sequence SEQ ID No.5. In that particular case, a reference result can be the affinity of the Tubby protein homolog of sequence SEQ ID No.3 for the human protein Gαq obtained when not contacted with any compound, or the cellular localization of the Tubby protein homolog of sequence SEQ ID No.3 when not contacted with any compound, or the affinity of the Tubby protein homolog of sequence SEQ ID No.5 for the human protein Gαq obtained when not contacted with any compound, or the cellular localization of the Tubby protein homolog of sequence SEQ ID No.5 when not contacted with any compound.

Thus, in this embodiment, step c) comprises:
- the comparison of the affinity of the Tubby protein homolog comprising the substitution A277P of sequence SEQ ID No.4 for the human protein Gαq obtained from step b) when the said protein is contacted with said candidate compound the with the affinity of the Tubby protein homolog of sequence SEQ ID No.3 when the reference said protein is not contacted with any compound and/or;
- the comparison of the cellular localization of the Tubby protein homolog comprising the substitution A277P of sequence SEQ ID No.4 obtained from step b) when the said protein is contacted with said candidate compound with the cellular localization of the Tubby protein homolog of sequence SEQ ID No.3, and/or;
- the comparison of the affinity of the Tubby protein homolog comprising the substitution A222P of sequence SEQ ID No.6 for the human protein Gαq obtained from step b) when the said protein is contacted with said candidate compound the with the affinity of the Tubby protein homolog of sequence SEQ ID No.5 when the reference said protein is not contacted with any compound and/or;
- the comparison of the cellular localization of the Tubby protein homolog comprising the substitution A222P of sequence SEQ ID No.6 obtained from step b) when the said protein is contacted with said candidate compound with the cellular localization of the Tubby protein homolog of sequence SEQ ID No.5.

In this embodiment, a compound is considered as suitable for the prevention and/or the treatment of a syndrome comprising massive human obesity, maculopathy and endocochlear deafness if:
- the affinity of the Tubby protein homolog comprising the substitution A277P of sequence SEQ ID No.4 for the human protein Gαq obtained from step b) when the said protein is contacted with said candidate compound is inferior of less than 20% or equal to the affinity of the Tubby protein homolog of sequence SEQ ID No.3 when the reference said protein is not contacted with any compound, and/or;
- the nuclear localization of the Tubby protein homolog comprising the substitution A277P of sequence SEQ ID No.4 obtained from step b) when the said protein is contacted with said candidate compound is inferior of less than 20% or equal to the nuclear localization of the Tubby protein homolog of sequence SEQ ID No.3, and/or;
- the affinity of the Tubby protein homolog comprising the substitution A222P of sequence SEQ ID No.6 for the human protein Gαq obtained from step b) when the said protein is contacted with said candidate compound is inferior of less than 20% or equal to the affinity of the Tubby protein homolog of sequence SEQ ID No.5 when the reference said protein is not contacted with any compound, and/or;
- the nuclear localization of the Tubby protein homolog comprising the substitution A222P of sequence SEQ ID No.6 obtained from step b) when the said protein is contacted with said candidate compound is inferior of less than 20% or equal to the nuclear localization of the Tubby protein homolog of sequence SEQ ID No.5.

The practice of the invention employs, unless other otherwise indicated, conventional techniques or protein chemistry, molecular virology, microbiology, recombinant DNA technology, and pharmacology, which are within the skill of the art. Such techniques are explained fully in the literature. (See Ausubel et al., Current Protocols in Molecular Biology, Eds., John Wiley & Sons, Inc. New York, 1995; Remington's Pharmaceutical Sciences, 17th ed., Mack Publishing Co., Easton, Pa., 1985; and Sambrook et al., Molecular cloning: A laboratory manual 2nd edition, Cold Spring Harbor Laboratory Press - Cold Spring Harbor, NY, USA, 1989).

Unless defined otherwise, all technical and scientific terms used herein have the same meaning as is commonly understood by one of the skill in the art to which this invention belongs.

The examples that follow are merely exemplary of the scope of this invention and content of this disclosure. One skilled in the art can devise and construct numerous modifications to the examples listed below without departing from the scope of this invention.

### FIGURE LEGENDS

Figure 1: 5) Bilateral alteration of the central visual field (patient II1). A. Funduscopy, B. OCT (optical coherent tomography), C. electrophysiological examinations and especially multifocal ERG (electroretinography) revealed an occult maculopathy.
Figure 2: Audiogram of I1 (first) and II1 (in grey) patients. The air-conduction pure-tone average reveals a severe hearing loss in all affected patients.
Figure 3: Translocation essay with wild-type (N) or A277P (M) TUB protein with GFP tag in Neuro2A cells.
Figure 4: Mislocalisation of the A277P TUB protein. A to C. Nuclear expression of normal TUB isoform 561 in SH-SY5Y cell. D to I. Nucleolar expression of A277P TUB isoform 561. Scale bar 0.2µm.

### EXAMPLES

### Experimental procedures

### TUB gene screening

We screened 12 genomic fragments corresponding to the 13 exons (Variant 1) and the complete short third intron (located between the exons 3 and 4) of the *TUB* gene in a pool of 159 morbidly obese patients (Male/female ratio 113/46, mean age 45±11.2 years (range 16-74), BMI 50±9.5 kg/m² (range 40-82)) and in 94 control non obese patients (Male/female ratio 53/47 mean age 36±7.5 years (range18-60), BMI 22±2.1 kg/m² (range 20-24)). These fragments were amplified from genomic DNA and the PCR products subjected to single-strand conformation polymorphism (SSCP) analysis. Exon 3 + intron 3 + exon 4 were amplified as a single fragment. Sequences of the primer pairs used for PCR-SSCP analysis are shown in Table 1. Variant SSCP patterns were detected in exons 1, 5, 7 and 11 and in intron 3. The products were directly sequenced to identify nucleotide changes. This allowed us to design PCR-RFLP assays for each shift.

**Table 1: Primers used for PCR-SSCP analysis and sequencing of the amplified fragments of the TUB gene. The amplified fragments included the complete coding sequence of the TUB gene.**

| Exon | Forward Primer | Reverse Primer | Length (bp) |
|---|---|---|---|
| 1 | TTACTATGCAGCCTGAAGTGG | GCTAAAGGCAGGATGTTGAA | 239 |
| 2 | AGGCAGGGATGATGAGGTGA | TCACAGTCTCCTGTGGTTGG | 172 |
| 3n1 | AATGGGCCCAGATATGCTGGG | ATCAGACGGGATCATCCAGGG | 241 |
| 3n2 | AGTGGGTCAGCCAACCTCTGT | AGCCATACACAGCCTGGGCAT | 310 |
| 4 | ACTGTGTTCAGGTGGGAGCT | GCAGGGCTGTAGAGAATGTG | 239 |
| 5 | ATGGACGTCTGAGAATCCAG | TGAGAACCTGGGACTGAGTT | 287 |
| 6 | TGTGTATTTCAGGGGCAGGG | CTGTGGATGCAGACTCACTC | 213 |
| 7 | TTTGGATCCCAGACCACCAAT | AGGTCAGCGGTAGATATGAGA | 369 |
| 8 | TGAGTGGCTCTCATGACTGT | AGGCCCTCTACAGACAGCTT | 255 |
| 9 | GAGGGTGCATGACTCTATAC | GGCATCAAGTCCTGGATTTC | 221 |
| 10 | AGTGGCCAGTGTTGCGTTCT | AAAGAGGGGAGGAAGGGGTA | 157 |
| 11 | ATCCCTTTCTGGGGTGGTCA | ACTCTAGCCAGTGTAGGCAG | 335 |
| 12 | TGTCCTTTTCTCTGTGCCTGT | GGCCTATATACAGAGGATAGG | 261 |

### PCR-based restriction fragment length polymorphism (PCR-RFLP) analysis

For each variant found, a PCR-RFLP assay was designed. All oligonucleotides were synthesized and purified by Sigma-Genosys (Cambridge, UK) and restriction enzymes obtained from New England Biolabs (St Quentin en Yvelines, France). PCR was performed in an MJ Research PTC-100 thermocycler. The PCR primers and PCR conditions followed by the restriction enzyme used were as follows:
**Exon 1**, TUB1 F *5'-TTACTATGCAGCCTGAAGTGG-3'*/ TUB1 R 5'-*GCTAAAGGCAGGATGTTGAA-3'*, 55°C, 2.5 mM MgCl₂. The resulting 239-bp product was cut with *Ear*I (wild-type cuts only).
**Intron 3**, *5'-21M13-AATGGGCCCAGATATGCTGGG-3'*/ TUB3n1 R *5'-M13REV-ATCAGACGGGATCATCCAGGG-3'*, 55°C, 0.5 mM MgCl₂. The resulting 280-bp product was cut with *Nla*III (mutant cuts only). The M13 sequence was attached to the 5'end to facilitate direct sequencing. The primers described above were used for amplification when the mutation introduced or deleted a restriction site for a commercially available restriction enzyme.

For the other exons, primers with a sequence mismatch in the 3' region were designed to create artificial mutation conditional restriction sites. The primer and PCR conditions followed by the restriction enzyme used, are indicated below. Mismatched bases underlined:
**Exon 5**, TUB5F *5'-ACTGTGTTCAGGTGGGAGCT-3'* / TUB5R *5'-CTTCTCCTTCCTAGCGGCCC-3'*, 55°C, 1.5mM MgCl₂ (DMSO 8%). The 210-bp product was cut with *Fau*I (wild-type cuts only);
**Exon 7**, TUB7F 5'-21M13-*TGTGTATTTCAGGGGCAGGG-3'*/ TUB7R *5'-CTG ACG GAC TTC CTG CTA GCA-3'*, 55°C, 1.5mM MgCl₂. The 213-bp product was cut with *Bbv*I (wild-type cuts only);
**Exon 11**, TUB11F 5'- *CAAGGGGCCTCGGAAGAAGA* -*3'*/ TUB11R 5'-*AAAGAGGGGAGGAAGGGGTA-3',* 53°C, 0.5 mM MgCl₂. The 108-bp product was cut with *Sap*I (wild-type cuts only).

To reduce the chances of false-negative results during RFLP experiments, a positive control (with mutation) and a negative control (without mutation) with known genotype, both based on the sequencing result, were digested in the same conditions.

### Statistical analyses

All statistical analyses were done using JMP software (Macintosh version 3.1). Data are expressed as means±standard deviations (SD) [range]. The non-parametric Wilcoxon/Kruskal-Wallis test was used to test for associations between gene variants and phenotypic features in the obese subjects. P values < 0.05 were considered to be statistically significant.

### Cells cultures and experiments

### Cell Lines

The classical human neuroblastoma SH-SY5Y cell line (CT) was used as control. Four additional lentivirally infected SH-SY5Y cell lines were designed and used one overexpressing either:
- the long human Tubby protein isoform containing 561 aminoacid residues (FL), tagged by the FLAG motif, rLV-EF1-TUB561-Flag;
- the short human Tubby protein isoform constituted by 506 amino-acids residues (Fc), tagged by the FLAG motif, rLV-EF1-TUB506-Flag;
- the A277P mutated long human Tubby protein isoform containing 561 aminoacid residues (Flm), tagged by the FLAG motif; rLV-EF1-TUB561-A277P-Flag;
- the short human A222P mutated Tubby protein isoform constituted by 506 amino-acids residues (Fcm); tagged by the FLAG motif, rLV-EF1-TUB506-A222P-Flag.

All cell lines were grown in DMEM medium (Invitrogen), containing 1% of streptomycin/penicillin solution (Invitrogen) and heat inactivated 10% fetal bovine serum (Invitrogen) defined as complete medium. Cultures were maintained in a 75 cm2 flask (VWR) in 10 ml of complete medium in a humidified atmosphere with 5% CO2 at 37°C. Complete medium was changed once a week and cells were passaged weekly. Cells were harvested according to the method described below.

### Purification of nucleoli

Purified nucleoli were prepared as followed from 300 million cells (Pascal Roussel's method). Actively growing cells were washed in cold PBS and lyzed at 4°C in a hypotonic buffer (10 mM Tris-HCl, pH 7.4, 25 mM NaCl, and 3 mM MgCl2). Lysis performed with the Tissue Master 125 homogenizer (OMNI international) was stopped when the nuclei appeared free of cytoplasmic components as assessed by phase microscopy. The nuclei were then collected by centrifugation at 1200 g for 5 min, resuspended in 10 mM Tris-HCl, pH 7.4, 10 mM NaCl, 10 mM MgCl2, 0.25 M sucrose and purified on a 0.88 M sucrose cushion prepared in 10 mM Tris-HCl, pH 7.4, 10 mM NaCl, 1.5 mM MgCl2 at 1200 g for 10 min. The nucleoli were isolated by sonication of nuclei suspended in 10 mM Tris-HCl, pH 7.4, 10 mM NaCl, 1.5 mM MgCl2, 0.34 M sucrose, and 0.25% NP40. The nucleoli are then purified on a 0.88 M sucrose cushion prepared as previously at 2000 g for 20 min. The purified nucleoli are washed by suspension in 10 mM Tris-HCl, pH 7.4, 10 mM NaCl, 1.5 mM MgCl2, 0.34 M sucrose and centrifugation at 2000 g for 5 min. All steps were performed at 4°C and all the solutions contained a cocktail of protease inhibitors (complete, Roche).

### Immunocytochemical analysis of SH-SY5Y cell lines lentivirally infected

SH-SY5Y cell lines infected at MOI 5, either with rLV-EF1-TUB561-Flag, rLV-EF1-TUB561-A277P-Flag rLV-EF1-TUB506-Flag or rLV-EF1-TUB506-A222P-Flag lentiviruses were obtained. Immunocytochemistry on each of these infected SH-SY5Y cell lines was performed as previously described (Klimentzou et al., 2008). Cells were analyzed firstly with an inverted fluorescence microscope (Zeiss, Germany) and digital pictures were obtained at different magnifications. Cells were fixed in 4% paraformaldehyde and blocked in 3% BSA. The cells were stained with mouse monoclonal anti-Flag antibody (Sigma, Saint Louis, Missouri). After incubation with primary antibodies (dilution 1/200) at room temperature for 1h, secondary antibody, Alexa Fluor 488 conjugated goat anti-mouse (Invitrogen, Carlsbad, CA), was used at dilution of 1/200 for 1h. 4', 6'-diamidino-2-phenylindole (DAPI) (Sigma-Aldrich) was used to visualise the nucleus. No staining was detected when the primary antibodies were omitted.

### Cell transfection and microscopy

T293, Neuro 2A and SH-SY5Y cells were maintained in 10% fetal calf serum and DMEM (without phenol red) media, in an atmosphere of 5% CO₂ at 37°C. Transfections of Neuro2A and 293T cells were carried out by mixing wild type or A277P-pGFP plasmid, (Clontech, Palo Alto, CA) with effectene transfection reagent (Qiagen, Valencia, CA) or optifect transfection reagent (Invitrogen, Carlsbad, CA). Cells were kept around 60% confluency on the day of transfection. The transfection procedure strictly followed the manufacturer's instructions. For the test of translocation, cells were typically measured 8h, 10 h, 12 h and 24 h, 48h, 72h and 96 h after transfection. The most striking effects were observed 24 hours after transfection. SH-SY5Y cell lines were also used for transfection experiments with constructs overexpressing each TUB isoform GFP tagged, either normal or mutated i.e. respectively TUB 561-GFP, A277P-TUB 561-GFP, TUB 506-GFP, A222-TUB-506-GFP. Cells were analyzed with an inverted fluorescence microscope (Zeiss, Germany) and digital pictures were obtained at different magnifications.

Time-lapse microscopy was performed using Leica DMI6000 B inverted microscope. An environmental chamber was used to maintain the temperature at 37°C and to provide an atmosphere of humid 5% CO2 in air. Images were acquired, every 10 min, using MetaMorph® Imaging System. ImageJ software was used for image analysis and making movis into an AVI format.

### Crystallography

A construct encoding residues 272 through 561 of the TUB protein was produced in *Escherichia coli* BL21 cells in the expression vector pSMT3 (based on the pET28a vector expression system). This vector encodes a 6-His tag preceding the yeast gene for the sumo-1 protein. The protein was purified by affinity chromatography using nickel affinity resin. The protein was then dialyzed into 10 mM Tris-Cl pH8.1, 200mM NaCl and 2mM DTT, then digested O/N at 4° C with the sumo-specific protease ULP1 to release the fusion partner, and further purified on a mono S column. Finally, a gel filtration step using a superose S75 column equilibrated in 10mM Tris-Cl pH8.1, 250 mM NaCl and 2 mM DTT was used to further purify the protein. When the concentration reached approximately 11 mg/mL, we screened for crystallization conditions by hanging drop, vapor diffusion method. Crystals belonging to space group I23 (*a*=*b*=*c*=149.981) were optimized in 16% PEG 400, 5 mM MgCl₂, 100 mM Sodium Acetate-pH 5.0, with 15mM DTT. Diffraction data to 2.7 Å were collected at 100K on beamline 8-BM at the Advanced Photon Source. Data were indexed and integrated using the Denzo software package, and merging and scaling of data was done using the Scalepack software package (Borek et al., 2003). Phases were determined by molecular replacement with AmoRe (Navaza, 2001) using mouse TUB (PDB ID 1C8Z) as a search model. Refinement was performed with the REFMAC5.1 (Murshudov et al., 1997) program of CCP4i (Potterton et al., 2002, 2003).

### Mass spectrometry analysis

After washes with binding buffer, immunoprecipitation beads were rinsed with 100 µl of NH4HCO3 25 mmol/L. Proteins on beads were digested overnight at 37°C by sequencing grade trypsin (12,5 µg/ml ; Promega Madison, Wi, USA) in 20 µl of NH4HCO3 25 mmol/L. Digests were analysed by a LTQ Velos Orbitrap (Thermo Fisher Scientific, San Jose, CA) coupled to an Easy nano-LC Proxeon system (Thermo Fisher Scientific, San Jose, CA). Chromatographic separation of peptides was performed using the following parameters: column Easy Column Proxeon C18 (10 cm, 75 µm i.d., 120 A), 300nl/min flow, gradient rising from 0 % solvent B (100 % acetonitrile, 0,1% formic acid) to 80% in 75 minutes (solvent A : H2O, 2% acetonitrile and 0,1% formic acid). Peptides were analysed in the orbitrap in full ion scan mode at a resolution of 30000 and a mass range of 300-2000 m/z. Fragments were obtained with a collision-induced dissociation (CID) activation with a collisional energy of 40% and analysed in the LTQ. MS/MS data were acquired in a data dependent mode in which 20 most intense precursor ions were isolated, with a dynamic exclusion of 15 seconds, an isolation width of 1 Dalton, an activation of 0,250 of 10 ms. Data were processed with Proteome Discoverer 1.3 software (Thermo Fisher scientific, San Jose, CA) coupled to an in house Mascot search server (Matrix Science, Boston, MA ; version 2.3.2). The mass tolerance of fragment ions was set to 5 ppm for precursor ions and 0,6 Dalton for fragments. The following modifications were used in variable modifications: oxidation (M), phosphorylations (STY). The maximum number of missed cleavages was limited to 2 for trypsin digestion. MS-MS data were searched against SwissProt, NCBInr databases and human taxonomy. False Discovery Rate (FDR) was calculated using the reversed database approach. The experiments were done on proteins extracts resulting from distinct coimmunoprecipitations of purified nucleoli on one hand and on proteins extracts resulting from coimmunoprecipitations of whole cells of each cell line used.

### Results

### Discovery of the mutation and clinical presentation of the disease

We screened for sequence variations in the *TUB* gene a cohort of 30 probands of families affected by diverse forms of syndromic obesity: 25 probands of Bardet-Biedl syndrome, 2 probands affected by Alström's syndrome, 2 probands affected by Usher's syndrome, and 1 proband affected by an autosomal recessive syndrome, consisting of morbid obesity associated with retinitis pigmentosa. None of the 30 probands affected by these forms of syndromic obesity displayed any nucleotide variation in *tub* gene.

A cohort of 159 morbidly non-syndromic obese patients and 94 control individuals were screened for sequence variations in the *TUB* gene. The sequencing of PCR-amplified fragments encompassing the entire *TUB* coding sequence of all the other patients revealed five nucleotide changes in the remaining subjects; four in exons and one within the second intron (Table 2). The list of the primers used for PCR-SSCP and the sequencing of the amplified fragments of the TUB gene are listed in Table 1.

**Table 2. Summary of TUB gene variations detected by PCR-RFLP analysis.**

| **Exon /intron** | **Nucleic acid change** | **Amino acid change** | **Assay** | **Restriction enzyme** | **Subjects** |
|---|---|---|---|---|---|
| 1 | **146G>A** | R49Q | PCR-RFLP | *Ear*I | *obese and non-obese subjects* |
| 3 | **36**-**C>T** | Intronic | PCR-RFLP | *Nla*III | *obese and non-obese subjects* |
| 5 | **105C>T** | G175G | PCR-RFLP | *Fau*I | *obese and non-obese subjects* |
| 7 | **97G>C** | A277P | PCR-RFLP | *Bbv*I | *massively obese proband* |
| 11 | **39C>T** | S441S | PCR-RFLP | *Sap*I | *obese and non-obese subjects* |

Four of the five variations were found in both obese and non-obese subjects. However, some SNPs located in the tubby gene are weakly but significantly associated with obesity or related phenotype. Two SNPs, 105C>T and 39C>T in exon 5 and 11, showed a higher frequency in common morbid obesity than in normal weight control individuals. In addition, SNPs in exon 5 and 11 were associated with lower leptin levels in the obese groups.

The fifth variation was found in exon 7 (97G→ C), and leads to the amino acid mutation A277P in a massively obese proband. The variation found in exon 7 (97G→ C) leads to the amino acid mutation A277P in the Tubby protein.

Whereas no mutation was discovered in the TUB gene among the 30 probands of families affected by diverse forms of syndromic obesity, inventors discovered that this mutation co-segregates with the syndromic obesity in all six affected members of the proband's family.

The proband (II1), who was 32 years old, had a normal birth weight (4.00 kg). At age 15, his weight was normal (73 kg for 1.80 m; BMI = 22.5 kg/m²). Although puberty occurred normally, his body weight dramatically increased after the age of 17 (100 kg). He weighed 140 kg at age 18, and 220 kg at age 20 (BMI = 60 kg/m²), and displays abnormal eating behavior with hyperphagia (total calorie intake -6000 kcal per day). His leptin concentration was high, but in proportion with his corpulence. Although the basal concentration of testosterone measured at 22 years was low (0.9 ng/ml), luteinizing hormone (LH) and follicle-stimulating hormone (FSH) concentrations were in the normal range. His prolactin concentration was normal. He had low T3 levels, normal free T4 levels, and slightly elevated basal thyroid-stimulating hormone (TSH) levels, suggesting moderate hypothyroidy. Morning and afternoon cortisol levels were normal. In addition to obesity, the proband developed severe diabetes at the age of 20 that was revealed by a ketosis episode. His diabetes was associated with hypertriglyceridemia.

The proband's younger brother (II5), age 25, had a more severe obesity phenotype that developed during infancy. His body weight was over 120 kg at age 14 and 246 kg (BMI = 65.5 kg/m²) at age 17. His food intake behavior was characterized by severe hyperphagia (over 8000 kcal per day), with compulsive manifestations. Like his older brother, he had normal puberty. His basal testosterone concentration was slightly low, but his LH and FSH concentrations were in the normal range.

Their mother (I2) is also heterozygous for the mutation. Her weight was normal at 20 years old (58 kg for 1.73 m), but dramatically increased with age. She reached a maximal weight of 117.5 kg at age 47 (BMI = 39 kg/m²). At this time, her severe obesity was associated with type 2 diabetes. Despite her obesity, actual food intake, as assessed by dietary history, was severely restricted (<1000 kcal/day).

The nucleotide change, which is cosegregating with the newly discovered syndrome, and the resulting alanine to proline substitution are highly susceptible to be useful as diagnostic or predictive biomarker of the syndromic obesity described above. Furthermore, data in this patent application provide evidence that the A277P mutation is a dominant allele, in contrast to the previously characterized knock-out and splice site mutants described in mice, which are recessive.

All family members underwent a complete general and ophthalmic examination. Whereas the father I1 had no diabetes mellitus, all affected members of this family carrying the A277P mutation displayed a type II diabetes mellitus requiring insulinotherapy associated with a pre-proliferative mixed (combining retinal ischemia and oedema) diabetic retinopathy associated with macular cystoid oedema reducing considerably their visual acuity. The fast development of a severe diabetes mellitus with a dramatic diabetic retinopathy has been observed in all the affected patients of this family in stark contrast with what was observed a long time ago for the tubby mouse phenotype. The homozygous mutations of the tubby mouse cause a slowly developing but ultimately a severe obesity. Surprisingly, the human syndrome is characterized by an early-onset massive obesity, unlike the late-onset, moderate obesity of the tubby mice, and by a type 2 diabetes Mellitus (T2DM) susceptible to appear quite early in some patients carrying the A277P mutation. This T2DM is evolving in all affected patients towards a severe form of diabetic retinopathy.

### Neurosensory Defects accompanying the A277P mutation

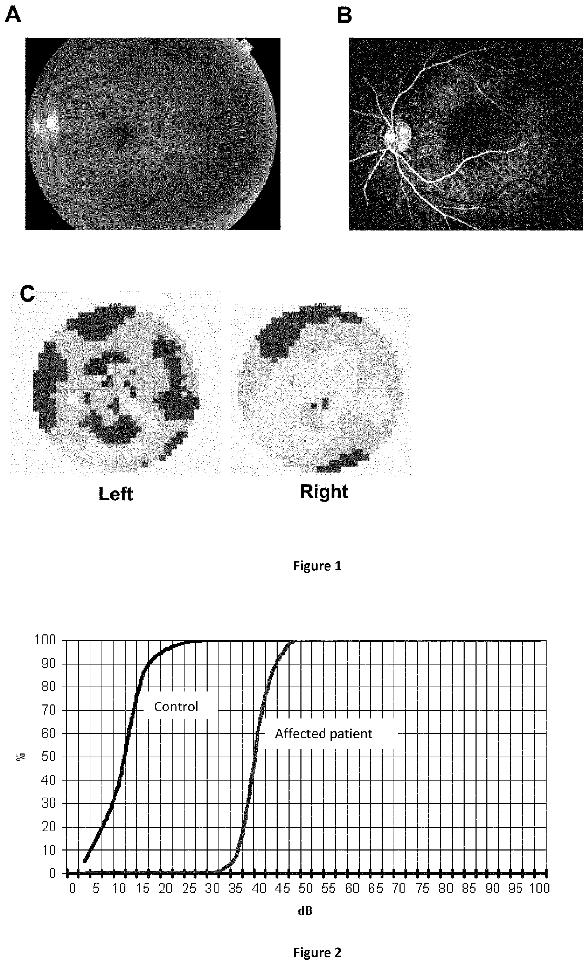

All the affected members of this family presented distinctive clinical and functional macular abnormalities. They all present a bilateral occult maculopathy with visual acuity loss, reduced visual field sensitivity with cecocentral scotomas (on central 10° threshold perimetry) (Figure 1). All affected family members consistently had a strongly disrupted electrooculogram (EOG), reflecting a dysfunction of their RPE. The ERG and EOG were normal in the unaffected father. Most importantly, all affected family members displayed a significant bilateral decrease of their macular retinal thickness, the measurements of which were obtained with the optical coherent tomography device Stratus OCT3 functioning with the latest software version (2.0, Carl Zeiss Meditec, Dublin, CA, USA), and typical alterations of their multifocal ERGs. Results of clinical ophthalmic examinations of the affected family strongly suggest that the initial degenerative maculopathy exclusively affects the macula and especially the foveola, without signs of associated pigmentary retinopathy. Only the oldest affected patient I2 presented a noticeably decreased visual acuity and the most severe foveolar scotomatas.

The patients' hearing was tested by clinical examination, audio tests including pure tone audiometry and evoked response audiometry. The air-conduction pure-tone average (ACPTA) threshold at conversational frequencies (0.5, 1, and 2 KHz) was calculated for each ear and used to define the severity of hearing loss (Figure 2). The mother's (I2) ACPTA was symmetrical, with a 38 dB bilateral hearing loss. Tympanograms and evoked response audiometry of all patients were normal, suggesting that the deafness was of endocochlear origin. No correlation was found between the severity of obesity (BMI) and the severity of hearing loss in this family.

### Molecular basis of pathogenicity:

### MATPLA does not play any role in the neurosensory phenotype

It should be noted that the tubby mouse neurosensory phenotype is modified by the moth1 (mtap1a) gene (Boggon et al., 1999; Ikeda et al., 1999, 2002). All exons of the MAP1a (Table 3) human gene (GI:95147554) were sequenced in all members of the family carrying the A277P mutation and in 100 unrelated control individuals. No specific nucleotide variation was found in the affected patients as compared to the unaffected father I1 and 100 control patients screened. In contrast with tubby mouse neurosensory phenotype, the results of our study suggest that, for A277P human mutant patients, MATP1A does not play any role in the neurosensory phenotype.

### The A277P mutation substantially reduces the ability of the TUB protein to translocate from the cell membrane into the nucleus

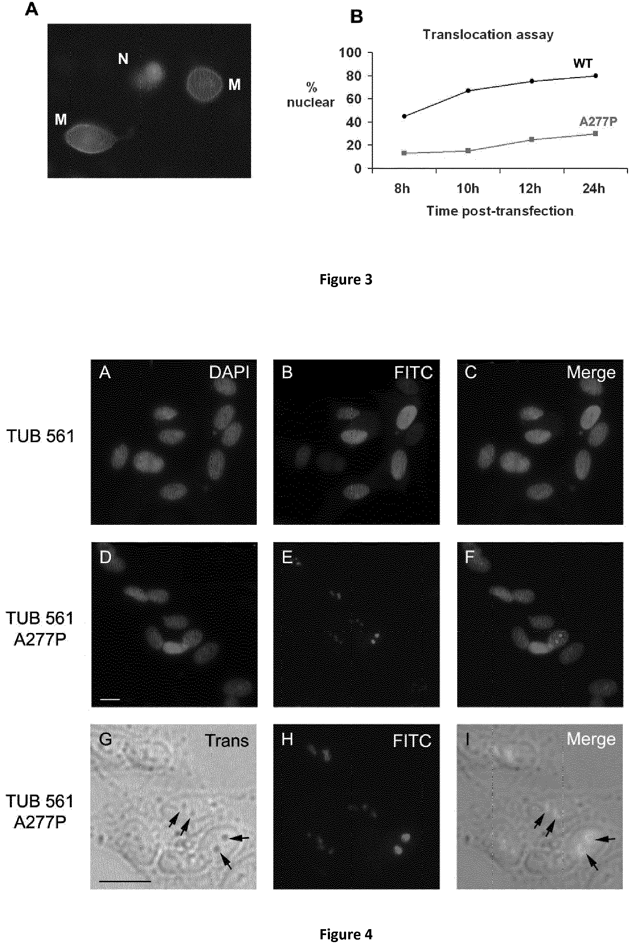

Little is known about the biochemical functions of the TUB protein; however, its subcellular localization is known to be controlled by Gαq signaling (Santagata 2001). To assess whether this behavior differs in the wild-type and A277P mutant, TUB 561 and mutated TUB561 were tagged at the N-terminus with GFP and transfected into PC12 and Neuro2A cultured cells. Translocation from the plasma membrane into the nucleus (Figure 3) was assayed by GFP fluorescence microscopy over a 96 hour-time course. The GFP-A277P protein shows a marked reduction in translocation efficiency compared to wild-type GFP-TUB. Similar results were obtained in HEK 293T and PC12 cell lines. Furthermore, whereas the wild-type protein completely moved to the cell nucleus within 72 to 96 hours, a substantial fraction of the cells transfected with the mutant protein continued to exhibit plasma membrane-associated and cytoplasmic fluorescence. These results suggest that the A277P mutation substantially reduces the ability of the TUB protein to translocate from the cell membrane into the nucleus.

### Mislocalization of the A277P long TUB isoform and of the A222P short TUB isoform.

Detailed and repeated transfections of the plasmid vector expressing the mutated fluorescently labelled long TUB isoform in three different cell lines suggested that accumulation of the fluorescence of the A277P-TUB isoform into the cell nuclei and then within their nucleoli was occurring more slowly and was in the average less intense than that of the TUB 561 isoform. Cultured neuroblastoma SH-SY5Y cell lines infected with lentiviruses bearing either the long normal TUB isoform coupled to a FLAG tag (FL); the long mutated TUB isoform coupled to a FLAG tag (FLm) the short TUB isoform coupled to a Flag tag (Fc) or the short mutated TUB isoform coupled also to a Flag Tag (Fcm) were used for immunocytofluorescent experiments. Whereas the FL cells produced an immunocytochemical signal characterized by a labelling of the whole nucleus associated with a stronger immunostaining of the nucleolus, in each cell observed (Figures 4A, 4B, 4C), the FLm cells displayed an immunocytochemical labelling restricted exclusively to the nucleolus (Figures 4D, 4E, 4F, 4G, 4H, 4I). Additionally and strikingly, whereas the Fc cells produced an immunocytochemical labelling distribution characterized by a nuclear labelling apparently restricted exclusively to the nucleolus, a very weak immunostaining of the cytoplasmic compartment and a detectable immunostaining of the neuritic compartment in each cell observed; the Fcm cells displayed an immunocytochemical labelling in the whole nucleus compartment with a more intense staining of the nucleoli, a weaker but significant labelling of the cytoplasmic compartment and a significant staining of the neuritic compartment, which is more intense at the neuritic extremities. These data suggest that the long and short TUB isoforms may play common and/or complementary roles in the nuclear compartments but fulfil different functions in the cytoplasmic and neuritic compartments. Furthermore, these data support a strong impact of the A277P mutation on Tubby function which results in the syndrome described above.

### Crystal structure of the C-terminal domain from the human tubby protein

Previously we determined the structure of the C-terminal domain of mouse Tub (residues 243 - 506), which corresponds to residues 298-561 of human TUB 561 isoform (Boggon et al., 1999). Since the A277P mutation lies outside this region, we prepared a larger recombinant protein in order to characterize the structural effects of the mutation. Crystals belonging to space group I23 were obtained by vapor diffusion, and diffract to a maximal resolution of 2.7 Å. The wild-type structure was solved by molecular replacement using mouse Tub (PDB ID 1C8Z) as a search model. While electron density is not observed for several N-terminal residues, 9 additional residues can be visualized; they form reciprocal contacts with a symmetry-related molecule. This interface could represent a biologically relevant TUB homodimer. The new residues stretch away from the tubby domain beta barrel and bind near 3 loops of an oppositely oriented TUB molecule. Residues S291, T293, A294 and E296 form 6 hydrogen bonds with backbone and side chain atoms from residues G436, Y494, L496, S506 and N509 of its mate. TUB dimerization would provide a mechanism to explain the dominant inheritance of this new *TUB* mutant phenotype. Thus, it is reasonable to think that an A277P mutation could perturb one molecule of the homodimer and act essentially in a dominant negative way to poison the function of the other. The crystal structure of TUB provides evidence for potential dimerization, which could underlie the dominant mode of transmission.

### Affinity of the short TUB isoform for Gα_{q} is dramatically decreased in the A277P mutant while the affinity of the long TUB isoform for Gα_{q} remains normal.

The crystal structure reported here shows that the site of mutation is distant from the TUB phosphatidylinositol (Ptdlns) binding site, responsible for plasma membrane binding. This suggests that the Alanine 277 Proline mutation might interfere with membrane dissociation by perhaps altering the interaction between TUB and Gα_{q} rather than with the Ptdlns binding site. Coprecipitation experiments reveal reproducibly and unambiguously that affinity of the short TUB isoform for Gα_{q} is dramatically decreased in the A277P mutant.

## Claims

1. A method for diagnosing in a subject a syndrome **characterized by** a group of features, said features comprising diabetic retinopathy, massive human obesity, maculopathy, endocochlear deafness, and early type II diabetes mellitus, said method comprising a step of detecting the transversion 97 G->C in exon 7 in the TUB gene, in a biological sample of the said subject.

2. A method for assessing the risk for a human subject of developing a syndrome comprising diabetic retinopathy, massive human obesity, maculopathy, endocochlear deafness, and early type II diabetes mellitus, said method comprising a step of detecting the transversion 97 G->C in exon 7 of the TUB gene, in a biological sample from the subject.

3. The method of any one of claims 1 or 2, wherein the step of detecting that the TUB gene comprises the transversion 97 G->C in exon 7 is performed at the nucleic acid level or at the protein level.

4. The method of any one of claims 1 to 3, wherein the step of detecting that the TUB gene comprises the transversion 97 G->C in exon 7 is performed with a nucleic microarray.

5. The method of any one of claims 1 to 3, wherein the step of detecting that the TUB gene comprises the transversion 97 G->C in exon 7 is performed by Restriction Fragment Length Polymorphism Analysis of PCR-Amplified Fragments (PCR-RFLP) or Polymerase chain reaction single-strand conformation polymorphism (PCR-SSCP).

6. The method of any one of claims 1 to 3, wherein the step of detecting that the TUB gene comprises the transversion 97 G->C in exon 7 is performed by sequencing.

7. The method of anyone of claims 1 to 6, wherein the step of detecting that the TUB gene comprises the transversion 97 G->C in exon 7 comprises the detection that the Tubby protein homolog comprises the substitution A277P.

8. The method of claim 7, wherein the step of detecting that the Tubby protein homolog comprises the substitution A277P is performed by protein sequencing.

9. The method of anyone of claims 1 to 8, wherein the said biological sample is a sample of epithelium tissue, preferably oral epithelium tissue.

10. The method of anyone of claims 1 to 9, wherein the said biological sample is saliva.

11. A method for selecting an active compound for the prevention and/or the treatment of a syndrome comprising diabetic retinopathy, massive human obesity, maculopathy, endocochlear deafness, and early type II diabetes mellitus in a human subject, comprising the steps of:
a) contacting a candidate compound with the human Tubby protein homolog comprising the substitution A277P of sequence SEQ ID No.4 and/or with the human Tubby protein homolog comprising the substitution A222P of sequence SEQ ID No.6;
b) assaying at least one of the functions of the human Tubby protein homolog comprising the substitution A277P of sequence SEQ ID No.4 and/or of the human Tubby protein homolog comprising the substitution A222P of sequence SEQ ID No. 6 in presence of the compound of step a);
c) comparing the result obtained from step b) with a reference result; and
d) determining that the candidate compound of step a) is an active compound for the prevention and/or the treatment of a syndrome comprising diabetic retinopathy, massive human obesity, maculopathy, endocochlear deafness, and early type II diabetes mellitus in a human subject according to the result of the comparison of step c)

12. The method of claim 11, wherein step b) comprises determining the binding affinity of the said human Tubby protein homolog comprising the A277P substitution of sequence SEQ ID No.4 and/or of the human Tubby protein homolog comprising the substitution A222P of sequence SEQ ID No.6 to human Gαq protein and/or the localization of the said Tubby protein homologue.

13. The method of any one of claims 11 or 12, wherein step b) comprises determining the binding affinity of the Tubby protein homolog comprising the substitution A277P of sequence SEQ ID No.4 and/or of the human Tubby protein homolog comprising the substitution A222P of sequence SEQ ID No.6 for the human Gαq protein.

14. The method of claim 13, wherein the said binding affinity is determined by BRET.

15. The method of claim 12 wherein determining the localization of the said human Tubby protein homolog comprising the A277P substitution of sequence SEQ ID No.4 and/or of the human Tubby protein homolog comprising the substitution A222P of sequence SEQ ID No.6 is performed by immunofluorescence.

## Patentansprüche

1. Verfahren zur Diagnose eines Syndroms bei einem Subjekt, **gekennzeichnet durch** eine Gruppe von Merkmalen, wobei die Merkmale diabetische Retinopathie, massive menschliche Fettleibigkeit, Makulopathie, endocochleäre Taubheit und frühen Diabetes mellitus Typ II umfassen, wobei das Verfahren einen Schritt des Nachweisens der Transversion 97 G->C in Exon 7 im TUB-Gen in einer biologischen Probe des Subjekts umfasst.

2. Verfahren zur Beurteilung des Risikos, dass ein menschliches Subjekt ein Syndrom entwickelt, umfassend diabetische Retinopathie, massive menschliche Fettleibigkeit, Makulopathie, endocochleäre Taubheit und frühen Diabetes mellitus Typ II, wobei das Verfahren einen Schritt des Nachweisens der Transversion 97 G->C in Exon 7 des TUB-Gens in einer biologischen Probe des Subjekts umfasst.

3. Verfahren nach einem der Ansprüche 1 oder 2, wobei der Schritt des Nachweisens, dass das TUB-Gen die Transversion 97 G->C in Exon 7 umfasst, auf der Nukleinsäureebene oder auf der Proteinebene durchgeführt wird.

4. Verfahren nach einem der Ansprüche 1 bis 3, wobei der Schritt des Nachweisens, dass das TUB-Gen die Transversion 97 G->C in Exon 7 umfasst, mit einem Nuklein-Microarray durchgeführt wird.

5. Verfahren nach einem der Ansprüche 1 bis 3, wobei der Schritt des Nachweisens, dass das TUB-Gen die Transversion 97 G->C in Exon 7 umfasst, durch Restriktionsfragmentlängenpolymorphismus-Analysis von PCR-amplifizierten Fragmenten (PCR-RFLP) oder Polymerasekettenreaktion-Einzelstrang-Konformationspolymorphismus (PCR-SSCP) durchgeführt wird.

6. Verfahren nach einem der Ansprüche 1 bis 3, wobei der Schritt des Nachweisens, dass das TUB-Gen die Transversion 97 G->C in Exon 7 umfasst, durch Sequenzierung durchgeführt wird.

7. Verfahren nach einem der Ansprüche 1 bis 6, wobei der Schritt des Nachweisens, dass das TUB-Gen die Transversion 97 G->C in Exon 7 umfasst, den Nachweis umfasst, dass das Tubby-Protein-Homolog die A277P-Substitution umfasst.

8. Verfahren nach Anspruch 7, wobei der Schritt des Nachweisens, dass das Tubby-Protein-Homolog die A277P-Substitution umfasst, durch Proteinsequenzierung durchgeführt wird.

9. Verfahren nach einem der Ansprüche 1 bis 8, wobei die biologische Probe eine Probe von Epithelgewebe, vorzugsweise Mundepithelgewebe, ist.

10. Verfahren nach einem der Ansprüche 1 bis 9, wobei die biologische Probe Speichel ist.

11. Verfahren zur Auswahl eines Wirkstoffs zur Vorbeugung und/oder Behandlung eines Syndroms, umfassend diabetische Retinopathie, massive menschliche Fettleibigkeit, Makulopathie, endocochleäre Taubheit und frühen Diabetes mellitus Typ II bei einem menschlichen Subjekt, umfassend die folgenden Schritte:
a) In-Kontakt-Bringen einer Kandidatenverbindung mit dem menschlichen Tubby-Protein-Homolog, umfassend die A277P-Substitution der Sequenz SEQ ID Nr. 4, und/oder mit dem menschlichen Tubby-Protein-Homolog, umfassend die A222P-Substitution der Sequenz SEQ ID Nr. 6;
b) Untersuchen von mindestens einer der Funktionen des menschlichen Tubby-Protein-Homologs, umfassend die A277P-Substitution der Sequenz SEQ ID Nr. 4, und/oder des menschlichen Tubby-Protein-Homologs, umfassend die A222P-Substitution der Sequenz SEQ ID Nr. 6, in Anwesenheit der Verbindung von Schritt a);
c) Vergleichen des Ergebnisses, das in Schritt b) erhaltenen wurde, mit einem Referenzergebnis; und
d) Bestimmen, dass die Kandidatenverbindung von Schritt a) ein Wirkstoff zur Vorbeugung und/oder Behandlung eines Syndroms ist, umfassend diabetische Retinopathie, massive menschliche Fettleibigkeit, Makulopathie, endocochleäre Taubheit und frühen Diabetes mellitus Typ II bei einem menschlichen Subjekt, gemäß dem Ergebnis des Vergleichs von Schritt c).

12. Verfahren nach Anspruch 11, wobei Schritt b) das Bestimmen der Bindungsaffinität des menschlichen Tubby-Protein-Homologs, umfassend die A277P-Substitution der Sequenz SEQ ID Nr. 4, und/oder des menschlichen Tubby-Protein-Homologs, umfassend die A222P-Substitution der Sequenz SEQ ID Nr. 6 des menschlichen Gαq-Proteins, und/oder die Lokalisierung des Tubby-Protein-Homologs umfasst.

13. Verfahren nach einem der Ansprüche 11 oder 12, wobei Schritt b) das Bestimmen der Bindungsaffinität des Tubby-Protein-Homologs, umfassend die A277P-Substitution der Sequenz SEQ ID Nr. 4, und/oder des menschlichen Tubby-Protein-Homologs, umfassend die A222P-Substitution der Sequenz SEQ ID Nr. 6 für das menschliche Gαq-Protein umfasst.

14. Verfahren nach Anspruch 13, wobei die Bindungsaffinität durch BRET bestimmt wird.

15. Verfahren nach Anspruch 12, wobei das Bestimmen der Lokalisierung des menschlichen Tubby-Protein-Homologs, umfassend die A277P-Substitution der Sequenz SEQ ID Nr. 4, und/oder des menschlichen Tubby-Protein-Homologs, umfassend die A222P-Substitution der Sequenz SEQ ID Nr. 6, durch Immunfluoreszenz durchgeführt wird.

## Revendications

1. Méthode de diagnostic chez un sujet d'un syndrome **caractérisé par** un groupe de caractéristiques, lesdites caractéristiques comprenant une rétinopathie diabétique, une obésité massive humaine, une maculopathie, une surdité endocochléaire, et un diabète de type II précoce, ladite méthode comprenant une étape de détection de la transversion 97 G->C dans l'exon 7 dans le gène TUB, dans un échantillon biologique dudit sujet.

2. Méthode d'estimation du risque pour un sujet humain de développer un syndrome comprenant une rétinopathie diabétique, une obésité massive humaine, une maculopathie, une surdité endocochléaire, et un diabète de type II précoce, ladite méthode comprenant une étape de détection de la transversion 97 G->C dans l'exon 7 dans le gène TUB, dans un échantillon biologique provenant du sujet.

3. Méthode selon l'une quelconque des revendications 1 ou 2, dans laquelle l'étape de détection que le gène TUB comprend la transversion 97 G->C dans l'exon 7 est effectuée au niveau de l'acide nucléique ou au niveau des protéines.

4. Méthode selon l'une quelconque des revendications 1 à 3, dans laquelle l'étape de détection que le gène TUB comprend la transversion 97 G->C dans l'exon 7 est effectuée avec une micropuce nucléique.

5. Méthode selon l'une quelconque des revendications 1 à 3, dans laquelle l'étape de détection que le gène TUB comprend la transversion 97 G->C dans l'exon 7 est effectuée par l'analyse de polymorphisme de longueur de fragment de restriction de fragments amplifiés par PCR (PCR-RFLP) ou réaction en chaîne de la polymérase-polymorphisme de conformation simple brin (PCR-SSCP).

6. Méthode selon l'une quelconque des revendications 1 à 3, dans laquelle l'étape de détection que le gène TUB comprend la transversion 97 G->C dans l'exon 7 est effectuée par séquençage.

7. Méthode selon l'une quelconque des revendications 1 à 6, dans laquelle l'étape de détection que le gène TUB comprend la transversion 97 G->C dans l'exon 7 comprend la détection que l'homologue de la protéine Tubby comprend la substitution A277P.

8. Méthode selon la revendication 7, dans laquelle l'étape de détection que l'homologue de la protéine Tubby comprend la substitution A277P est effectuée par séquençage de la protéine.

9. Méthode selon l'une quelconque des revendications 1 à 8, dans laquelle ledit échantillon biologique est un échantillon de tissu épithélial, de préférence de tissu épithélial buccal.

10. Méthode selon l'une quelconque des revendications 1 à 9, dans laquelle ledit échantillon biologique est la salive.

11. Méthode de sélection d'un composé actif pour la prévention et/ou le traitement d'un syndrome comprenant une rétinopathie diabétique, une obésité massive humaine, une maculopathie, une surdité endocochléaire, et un diabète de type II précoce chez un sujet humain, comprenant les étapes suivantes :
a) la mise en contact d'un composé candidat avec l'homologue de la protéine Tubby humaine comprenant la substitution A277P de séquence SEQ ID NO : 4 et/ou avec l'homologue de la protéine Tubby humaine comprenant la substitution A222P de séquence SEQ ID NO : 6 ;
b) l'analyse d'au moins l'une des fonctions de l'homologue de la protéine Tubby humaine comprenant la substitution A277P de séquence SEQ ID NO : 4 et/ou de l'homologue de la protéine Tubby humaine comprenant la substitution A222P de séquence SEQ ID NO : 6 en présence du composé de l'étape a) ;
c) la comparaison du résultat obtenu à partir de l'étape b) avec un résultat de référence ; et
d) la détermination que le composé candidat de l'étape a) est un composé actif pour la prévention et/ou le traitement d'un syndrome comprenant une rétinopathie diabétique, une obésité massive humaine, une maculopathie, une surdité endocochléaire, et un diabète de type II précoce chez un sujet humain selon le résultat de la comparaison de l'étape c).

12. Méthode selon la revendication 11, dans laquelle l'étape b) comprend la détermination de l'affinité de liaison dudit homologue de la protéine Tubby humaine comprenant la substitution A277P de séquence SEQ ID NO : 4 et/ou de l'homologue de la protéine Tubby humaine comprenant la substitution A222P de séquence SEQ ID NO : 6 à la protéine Gαq humaine et/ou de la localisation dudit homologue de la protéine Tubby.

13. Méthode selon l'une quelconque des revendications 11 ou 12, dans laquelle l'étape b) comprend la détermination de l'affinité de liaison de l'homologue de la protéine Tubby comprenant la substitution A277P de séquence SEQ ID NO : 4 et/ou de l'homologue de la protéine Tubby humaine comprenant la substitution A222P de séquence SEQ ID NO : 6 pour la protéine Gαq humaine.

14. Méthode selon la revendication 13, dans laquelle ladite affinité de liaison est déterminée par BRET.

15. Méthode selon la revendication 12, dans laquelle la détermination de la localisation dudit homologue de la protéine Tubby humaine comprenant la substitution A277P de séquence SEQ ID NO : 4 et/ou de l'homologue de la protéine Tubby humaine comprenant la substitution A222P de séquence SEQ ID NO : 6 est effectuée par immunofluorescence.
